# EUROPEAN PATENT SPECIFICATION

(11) **EP 3 638 670 B1**
(45) Date of publication and mention of the grant of the patent: **21.07.2021**
(21) Application number: 18728902.0
(22) Date of filing: 07.06.2018
(51) Int. Cl.: C07D 471/04, A61K 31/437, A61P 35/00

(54) **SUBSTITUTED PYRROLOPYRIDINE-DERIVATIVES AS MAP4K1 MODULATORS FOR THE TREATMENT OF CANCER DISEASES**
SUBSTITUIERTE PYRROLOPYRIDINDERIVATE ALS MAP4K1-MODULATOREN ZUR BEHANDLUNG VON KREBSERKRANKUNGEN
DÉRIVÉS DE PYRROLOPYRIDINE SUBSTITUÉS UTILISÉS EN TANT QUE MODULATEURS DE MAP4K1 POUR LE TRAITEMENT DE MALADIES CANCÉREUSES

(30) Priority: 13.06.2017 EP 17175846
(43) Date of publication of application: 22.04.2020
(73) Proprietor: Bayer Pharma Aktiengesellschaft, 13353 Berlin (DE); Bayer Aktiengesellschaft, 51373 Leverkusen (DE); Deutsches Krebsforschungszentrum Heidelberg, 69120 Heidelberg (DE)
(72) Inventor: SCHMEES, Norbert, 13469 Berlin (DE); BUCHMANN, Bernd, 16540 Hohen Neuendorf (DE); FRIBERG, Anders Roland, 13189 Berlin (DE); BRIEM, Hans, 10719 Berlin (DE); HUSEMANN, Manfred, 16540 Hohen Neuendorf (DE); BÖMER, Ulf, 16548 Glienicke (DE); LEDER, Gabriele, 14163 Berlin (DE); CARRETERO, Rafael, 69121 Heidelberg (DE); STÖCKIGT, Detlef, 14471 Potsdam (DE); OFFRINGA, Rienk, 69115 Heidelberg (DE)
(74) Representative: BIP Patents
(86) International application number: PCT/EP2018/065043
(87) International publication number: WO 2018/228923

(56) References cited:
- EP-A1- 1 921 078
- WO-A2-2006/004884

## Description

The present invention relates to protein-inhibitory substituted pyrrolopyridine derivatives, to pharmaceutical compositions and combinations comprising the compounds according to the invention, and to the prophylactic and therapeutic use of the inventive compounds, respectively to the use of said compounds for manufacturing pharmaceutical compositions for the treatment or prophylaxis of diseases, in particular for neoplastic disorders, repectively cancer or conditions with dysregulated immune responses or other disorders associated with aberrant MAP4K1 signaling, as a sole agent or in combination with other active ingredients.

The present invention further relates to the use, respectively to the use of said compounds for manufacturing pharmaceutical compositions for the treatment or prophylaxis of protein inhibitors in benign hyperplasias, atherosclerotic disorders, sepsis, autoimmune disorders, vascular disorders, viral infections, in neurodegenerative disorders, in inflammatory disorders, in atherosclerotic disorders and in male fertility control.

### Background

Although cancer cell commonly can be recognize by the adaptive immune system, the response generated is evidently not capable of eliminating the tumor. A major reason for this is the presence of immunosuppressive mechanisms in the tumor microenvironment. In this respect, inhibitors of T-cell immune checkpoint such as CTLA-4, PD-1 or PD-L1 were recently shown to result in a remarkable clinical efficacy in subsets of cancer patients. Besides cell surface receptors that act as negative immune regulators, several mediators of intracellular signaling have been identified that also represent potential immunoevasive mechanisms utilized by the tumor.

One of these is MAP4K1, also known as hematopoietic progenitor kinase 1 (HPK1). MAP4K1 (GenelD11184) is a serine/threonine kinase and member of the Germinal Center Kinase family. In the adult organism MAP4K1 expression is restricted to hematopoietic cell types. The MAP4K1 protein consist of a N-terminal kinase domain, followed by a proline-rich domain that can interact with adaptor molecules through SH2 and SH3 domains, and a C-terminal citron homology domain of which the exact function remains to be identified. Through its proline-rich domain, MAP4K1 is capable of binding to a diversity of adaptors in hematopoietic cells, including those involved in T-cell receptor (TCR), B-cell receptor (BCR) and cytokine signaling (Hu et al., Genes Dev. 1996 Sep 15;10(18):2251-64, 2.; Ling et al.,. J Biol Chem. 2001 Jun 1;276(22), Sauer et al., J Biol Chem. 2001 Nov 30;276(48):45207-16., Tsuji et al., J Exp Med. 2001 Aug 20;194(4):529-39, Boomer et al., J Cell Biochem. 2005 May 1;95(1):34-44).

The function of MAP4K1 has been studied in greatest detail in the context of TCR signaling. Upon TCR stimulation, MAP4K1 is phosphorylated on tyrosine 381 (Y-381; Y-379 in mouse) (Di Bartolo et al., J Exp Med. 2007 Mar 19;204(3):681-91). Consequently, MAP4K1 is recruited to the TCR-signaling complex where it induces dissociation of this complex through its serine/threonine kinase function. In particular MAP4K1 phosphorylates the SLP-76 adaptor protein at Serine-376, resulting in downregulation of AP-1 and Erk2 pathways. As, such, MAPK1 acts as a negative feedback on TCR-signaling (Liou et al., Immunity. 2000 Apr;12(4):399-408; Lasserre et al., J Cell Biol. 2011 Nov 28;195(5):839-53.). Alternatively, MAP4K1 can be triggered to suppress T cell function by prostaglandin E2 (PGE2), and possibly also by transforming growth factor beta (TGF-beta), factors that are commonly found in the tumor microenvironment. Notably, MAP4K1 activation by these mediators involves protein kinase A (PKA)-dependent phosphorylation of Serine 171 (S-171; also in mouse) (Alzabin et al., Cancer Immunol Immunother. 2010 Mar;59(3):419-29; Sawasdikosol et al., J Biol Chem. 2007 Nov 30;282(48):34693-9.).

Further important insights into the function of MAP4K1 in the regulation of T cell immunity stem from *in vivo* and *in vitro* experiments respectively with MAP4K1 deficient mice produced by two laboratories and with immune cells isolated from these mice (Shui et al., Nat Immunol. 2007 Jan;8(1):84-91; Alzabin et al., Cancer Immunol Immunother. 2010 Mar;59(3):419-29). MAP4K1-deficient mice show an apparent normal phenotype, are fertile and exhibit normal lymphocyte development. These animals are prone to develop T-cell dependent autoimmune reactivity as indicated by development of a more severe disease score in the EAE (experimental autoimmune encephalomyelitis) model of multiple sclerosis (Shui et al., Nat Immunol. 2007 Jan;8(1):84-91). In case of the second strain, a dysregulation of immune function was observed when, at the age of approximately 6 months, MAP4K1-deficient mice develop a spontaneous autoimmune phenotype (Alzabin et al., Cancer Immunol Immunother. 2010 Mar;59(3):419-29). *In vitro* studies showed that MAP4K1-/- T-cells display hyper-responsiveness upon TCR-stimulation. These cells proliferate and secrete pro-inflammatory cytokines like IL-2 or IFNg to a significantly greater extent than their wild-type counterparts (Shui et al., Nat Immunol. 2007 Jan;8(1):84-91). Furthermore, MAP4K1-/- T-cells are resistant to PGE2-mediated suppression of T cell proliferation, suppression of IL-2 production and induction of apoptosis (Alzabin et al., Cancer Immunol Immunother. 2010 Mar;59(3):419-29). In the context of tumor immunology, *in vivo* experiments revealed that MAP4K1-/- mice are much more resistant to tumorigenesis by PGE2-producing Lewis lung carcinoma than wild type mice, which correlated with increased T-lymphocyte infiltration in the tumor areas. The crucial role of T-cells in tumor rejection was supported by experiments in which MAP4K1-/- T-cells adoptively transferred into T-cell-deficient mice were able to eradicate tumors more efficiently than wild-type T-cells (Alzabin et al., Cancer Immunol Immunother. 2010 Mar;59(3):419-29). The important role of the kinase enzymatic activity was demonstrated by studies were only wild type MAP4K1, but not the MAP4K1 kinase-dead mutant, could mediate serine-phosphorylation of the TCR-signaling complex component SLP-76 and subsequent binding of SLP-76 to the negative regulator of TCR-signaling 14-3-3-t (Shui et al., Nat Immunol. 2007 Jan;8(1):84-91). MAP4K1 also regulates the stimulation and activation of dendritic cells. MAP4K1 deficient Bone marrow derived cells (BMDC) express after maturation and stimulation higher level of costimulatory molecules and produce more proinflammatory cytokines. Also elimination of tumors was observed to be more efficient by MAP4K1 -/- BMDC compared to their wildtype counterparts (Alzabin et al., J Immunol. 2009 May 15;182(10):6187-94).

### Prior art

In WO 2016/205942 HPK1, respectively inhibitors and methods of their use in cancer treatment are described. Especially, the application concerns thieno-pyridinones that can be used in anti-cancer therapy. These compounds differ from the instant compounds in their chemical structure.

In WO 2016/195776 inhibitors and methods for leukemia, cancer and diabetes treatment dependent on inhibition the interaction of menin with of MLL1, MLL2 and MLL-fusion oncoproteins are described. These compounds differ from the instant compounds in their chemical structure.

In WO 2006/014325 C-MET modulators and their use in cancer treatment are described. These compounds differ from the instant compounds in their chemical structur.

In WO 2005/058891 Rho kinase inhibitors and their use in cardiovascular and cancer treatment are described. These compounds differ from the instant compounds in their chemical structure.

In WO 2015/089479 several inhibitors are described that show inhibition of several kinases (e.g., BTK, HCK, TAK1 and HPK1). These compounds differ from the instant compounds in their chemical structure. Documents EP 1 921 078 and WO 2006/004884 reveal structurally similar kinase inhibitors.

It would therefore be desirable to provide novel compounds having prophylactic and therapeutic properties.

Accordingly, it is an object of the present invention to provide compounds and pharmaceutical compositions comprising these compounds used for prophylactic and therapeutic applications for hyperproliferative disorders, in particular for cancer, respectively tumour disorders, and conditions with dysregulated immune responses, as a sole agent or in combination with other active ingredients.

A further object of the present invention is to provide compounds and pharmaceutical compositions comprising these compounds for manufacturing pharmaceutical compositions for the treatment or prophylaxis of protein inhibitors in benign hyperplasias, atherosclerotic disorders, sepsis, autoimmune disorders, vascular disorders, viral infections, in neurodegenerative disorders, in inflammatory disorders, in atherosclerotic disorders and in male fertility control.

Surprisingly, the compounds according to the invention inhibit the MAP4K1 protein and inhibit the growth of cancer cells. Accordingly, they provide novel structures for the therapy of human and animal disorders, in particular of cancers.

The present invention relates to compounds of formula (I) 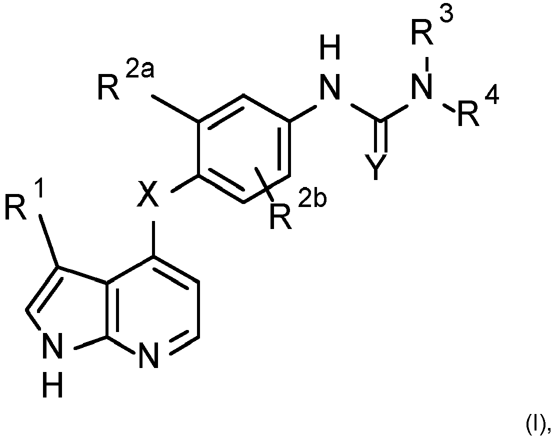 in which
- X: represents a nitrogen, a sulphur or an oxygene atom,
- Y: represents a sulphur or an oxygene atom,
- R¹: represents hydrogen, halogen, cyano, C₁-C₆-alkyl or halo-C₁-C₆-alkyl,
- R^{2a}: represents C₁-C₆-alkoxy, halo-C₁-C₆-alkyl or halo-C₁-C₆-alkoxy
- R^{2b}: represents hydrogen, halogen, cyano or C₁-C₃-alkyl,
- R³: represents hydrogen or C₁-C₆-alkyl,
- R⁴: represents hydrogen or represents C₁-C₆-alkyl-, which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy-, -S(=O)₂NH₂, -C(=O)-R⁹, -C(=O)-NH-R^{x}, -NH-C(=O)-R⁹, -NH-S(=O)₂-R⁹, -S(=O)₂-R⁹, -S(=O)(=NR^{y})-R^{x}, -S(=O)-R^{x}, C₃-C₁₀-cycloalkyl- which itself may optionally be mono- or polysubstituted by dentical or different substituents from the group consisting of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸,-C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
monocyclic heteroaryl- having 5 or 6 ring atoms which itself may optionally be mono- or polysubstituted by identical or different substituents from the group consisting of halogen, amino, hydroxy, cyano, nitro, carboxy, C₁-C₆-alkyl-, C₁-C₆-alkoxy-, C₁-C₆- alkoxy-C₁-C₆-alkyl-, hydroxy-C₁-C₆-alkyl-, C₁-C₆-alkylamino-, amino-C₁-C₆-alkyl-, C₁-C₆-alkylamino-C₁-C₆-alkyl, halo-C₁-C₆-alkyl-, halo-C₁-C₆-alkoxy-, C₃-C₁₀-cycloalkyl and a monocyclic heterocyclyl radical having 3 to 8 ring atoms,
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
phenyl- which itself may optionally be mono- or polysubstituted by identical or different substituents from the group consisting of halogen, amino, hydroxy, cyano, nitro, carboxy, C₁-C₆-alkyl-, C₁-C₆-alkoxy-, C₁-C₆-alkoxy-C₁-C₆-alkyl-, C₁-C₆-alkyl-amino-, amino-C₁-C₆-alkyl-, C₁-C₆-alkylaminocarbonyl-, C₁-C₆-alkyl-aminosulphonyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
spirocycloalkyl-, heterospirocycloalkyl-, bicycloalkyl-, heterobicycloalkyl-, bridged cycloalkyl or a bridged heterocycloalkyl, naphthyl or bicyclic heteroaryl, or partially saturated bicyclic aryl- or heteroaryl,
each of which mentioned *supra* may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
- R⁴: represents C₃-C₁₀-cycloalkyl- which may optionally be mono- or polysubstituted by dentical or different substituents from the group consisting of of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷,-S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents monocyclic heteroaryl- having 5 or 6 ring atoms which may optionally be mono- or polysubstituted by identical or different substituents from the group consisting of of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents monocyclic heterocyclyl- having 3 to 8 ring atoms which may optionally be mono- or polysubstituted by identical or different substituents from the group consisting of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶,-NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents phenyl- which may optionally be mono- or polysubstituted by identical or different substituents from the group consisting of of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-,phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents a spirocycloalkyl radical, a heterospirocycloalkyl radical, a bicycloalkyl, a heterobicycloalkyl radical, a bridged cycloalkyl radical or a bridged heterocycloalkyl radical, a naphthyl radical or a bicyclic heteroaryl radical, or a partially saturated bicyclic aryl- or heteroaryl radical, where the radicals mentioned may optionally be mono- or polysubstituted by identical or different substituents from the group consisting of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
- R³ and R⁴: together with the nitrogen atom form a 4 to 10 membered heterocycloalkyl ring, which may optionally be mono- or polysubstituted by identical or different substituents from the group consisting of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷ ,-S(=O)₂-NR⁵R⁶, -S(=O)-R⁸,-S(=O)(=NR⁵)-R⁶,-S(=O)₂-R⁸, -NH-S(=O)₂-R⁸, or a monocyclic heterocyclyl radical having 3 to 8 ring atoms,
- R⁵ and R⁶: independently of one another represent hydrogen, C₁-C₃-alkyl-, cyclopropyl- or di-C₁-C₃-alkylamino-C₁-C₃-alkyl-,
- R⁷: represents hydroxy, C₁-C₆-alkyl-, C₁-C₆-alkoxy-, halo-C₁-C₆-alkyl-, hydroxy-C₁-C₃-alkyl-, C₁-C₃-alkoxy-C₁-C₃-alkyl-, C₃-C₈-cycloalkyl-, phenyl-, monocyclic heterocyclyl- having 3 to 8 ring atoms or monocyclic heteroaryl- having 5 or 6 ring atoms where phenyl-, heteroaryl- and heterocyclyl- may optionally be mono- or disubstituted by halogen, C₁-C₃-alkoxy- or C₁-C₃-alkyl-,
- R⁸: represents C₁-C₆-alkyl-,
- R⁹: represents C₁-C₆-alkyl-, -NH₂, -NH-C₁-C₆-alkyl, -N(C₁-C₆-alkyl)₂, or C₁-C₆-alkoxy-C₁-C₆-alkyl-,
or
represents monocyclic heterocyclyl- having 3 to 8 ring atoms which itself may optionally be mono- or polysubstituted by identical or different substituents from the group consisting of of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-, phenyl-, halophenyl-, phenyl-C₁-C₆-alkyl-, phenoxy-, pyridinyl-, -C(=O)-NR⁵R⁶,-NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
- R^{x}: represents C₁-C₆-alkyl- or C₁-C₆-alkoxy-C₁-C₆-alkyl-,
- R^{y}: represents hydrogen, halo-C₁-C₆-alkyl, C₁-C₆-alkyl, C₁-C₆-alkyl substituted with C₃-C₆-cycloalkyl,
and their polymorphs, enantiomers, diastereomers, racemates, tautomers, solvates, physiologically acceptable salts and solvates of these salts.

The compounds of formula (I) are particularly suitable for a large number of prophylactic and therapeutic applications, in particular for hyperproliferative disorders, for tumour disorders and as proteine inhibitors and further for viral infections, for neurodegenerative disorders, for inflammatory disorders, for atherosclerotic disorders and for male fertility control.

Further, it covers their use in combination with other anti cancer medications such as immunotherapeutics, targeted anti cancer agents, radiation or chemotherapy.

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon atom. Commonly, it is possible for the number of optional substituents, when present, to be 1, 2 or 3.

The term "comprising" when used in the specification includes "consisting of".
If within the present text any item is referred to as "as mentioned herein", it means that it may be mentioned anywhere in the present text.
The terms as mentioned in the present text have the following meanings:
The term "halogen" means a fluorine, chlorine, bromine or iodine, particularly a fluorine, chlorine or bromine atom.

The term "C₁-C₆-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("C₁-C₄-alkyl"), *e.g.* a methyl, ethyl, propyl, isopropyl, butyl, *sec*-butyl isobutyl, or *tert*-butyl group, more particularly 1, 2 or 3 carbon atoms ("C₁-C₃-alkyl"), *e.g.* a methyl, ethyl, n-propyl or isopropyl group.

The term "hydroxy-C₁-C₆-alkyl" means a linear or branched, saturated, monovalent hydrocarbon group in which the term "C₁-C₆-alkyl" is defined *supra,* and in which 1 or 2 hydrogen atoms are replaced with a hydroxy group, e.g. a hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl, 1-hydroxy-2-methyl-propyl group.

The term "C₁-C₆-alkoxy" means a linear or branched, saturated, monovalent group of formula (C₁-C₆-alkyl)-O-, which means methoxy, ethoxy, n-propoxy, isopropoxy, n-butoxy, sec-butoxy, isobutoxy or *tert*-butoxy.

The term "C₁-C₆-alkoxy-C₁-C₆-alkyl-" means a linear or branched, saturated, monovalent group of formula (C₁-C₆-alkyl)-O-(C₁-C₆-alkyl)-, in which the term "C₁-C₆-alkyl" is defined *supra,* and in which 1 to 3 hydrogen atoms of the C₁-C₆-alkyl group are replace by C₁-C₆-alkoxy.

The term "C₁-C₆-alkylamino-" means an amino radical having one or two alkyl substituents (selected independently of one another) having generally 1 to 6 (C₁-C₆-alkylamino) and preferably 1 to 3 (C₁-C₃-alkylamino) carbon atoms. (C₁-C₃)-Alkylamino represents, for example, a monoalkylamino radical having 1 to 3 carbon atoms or a dialkylamino radical having 1 to 3 carbon atoms each per alkyl substituent.
The following may be mentioned by way of example:
methylamino, ethylamino, *n*-propylamino, isopropylamino, *tert*-butylamino, *n*-pentylamino, *n*-hexylamino, *N,N*-dimethylamino, *N,N*-diethylamino, *N*-ethyl-*N*-methylamino, *N*-methyl-N-*n-*propylamino, *N*-isopropyl-*N*-n-propylamino, *N-tert*-butyl-*N*-methylamino, *N*-ethyl-*N-n*-pentylamino and *N-n*-hexyl-*N*-methylamino.

The term "amino-C₁-C₆-alkyl-" means a linear or branched, saturated, monovalent hydrocarbon group in which the term "C₁-C₆-alkyl" is defined *supra,* and in which 1 or 2 hydrogen atoms are replaced with an amino group, *e.g.* a aminomethyl, 1-aminoethyl, 2-aminoethyl, 1,2-diaminoethyl, 3-aminopropyl, 2-aminopropyl, 1-aminopropyl, 1-aminopropan-2-yl, 2-aminopropan-2-yl, 2,3-diaminopropyl, 1,3-diaminopropan-2-yl, 3-amino-2-methyl-propyl, 2-amino-2-methyl-propyl, 1-amino-2-methyl-propyl group.

The term "C₁-C₆-alkylamino-C₁-C₆-alkyl means that the alkylaminoalkyl group is attached via the alkyl moiety to the remainder of the molecule, *e.g. N,N*-dimethylaminoethyl-, *N,N-*dimethylaminomethyl-, *N,N*-diethylaminoethyl-, *N,N*-dimethylaminopropyl-, *N-*methylaminoethyl-, *N*-methylaminomethyl-.

The term "halo-C₁-C₆-alkyl-" means an alkyl radical having at least one halogen substituent. The term "C₁-C₆-alkyl-" is as defined *supra.*
A halo-C₁-C₆-alkyl radical is an alkyl radical having 1-6 carbon atoms and at least one halogen substituent. If a plurality of halogen substituents is present, these may also be different from one another. Preference is given to fluoro-C₁-C₆-alkyl, fluoro-C₁-C₄-alkyl, fluoro-C₁-C₃-alkyl, chloro-C₁-C₆-alkyl, chloro-C₁-C₄-alkyl, chloro-C₁-C₃-alkyl, bromo-C₁-C₆-alkyl, bromo-C₁-C₄-alkyl and bromo-C₁-C₃-alkyl radicals.
In this regard preferred are difluoromethyl, trifluoromethyl, 2,2,2-trifluoroethyl, pentafluoroethyl, 4,4,5,5,5-pentafluoropentyl or 3,3,4,4,5,5,5-heptafluoropentyl.
Preference is given to perfluorinated alkyl radicals which are named as "perfluoro-C₁-Cₓ-alkyl-" wherein x is the maximum number of carbon atoms such as trifluoromethyl or 2,2,2-trifluoroethyl.

The term "haloalkoxy" means an alkoxy radical having at least one halogen substituent.
A halo-C₁-C₆-alkoxy radical is an alkoxy radical having 1-6 carbon atoms and at least one halogen substituent. If a plurality of halogen substituents is present, these may also be different from one another. Preference is given to fluoro-C₁-C₆-alkoxy, fluoro-C₁-C₄-alkoxy, fluoro-C₁-C₃-alkoxy, chloro-C₁-C₆-alkoxy, chloro-C₁-C₄-alkoxy, chloro-C₁-C₃-alkoxy, bromo-C₁-C₆-alkoxy, bromo-C₁-C₄-alkoxy and bromo-C₁-C₃-alkoxy radicals.
Preference is given to perfluorinated alkyl radicals which are named as "perfluoro-C₁-Cₓ-alkoxy-" wherein x is the maximum number of carbon atoms such as trifluoromethoxy and 2,2,2-trifluoroethoxy radicals.

The term "halophenyl" means a phenyl radical which is mono- or polysubstituted by identical or different substituents from the group consisting of fluorine, chlorine and bromine.

The term "C₃-C₁₀-cycloalkyl" means a saturated, monovalent, monocyclic hydrocarbon ring which contains 3, 4, 5, 6, 7, 8, 9 or 10 carbon atoms ("C₃-C₁₀-cycloalkyl"). Said C₃-C₁₀-cycloalkyl group is a monocyclic hydrocarbon ring, e.g. a cyclopropyl, cyclobutyl, cyclopentyl, cyclohexyl, cycloheptyl, cyclooctyl, cyclononyl or cyclodecanyl. Preferred are 3 to 8 (C₃-C₈-cycloalkyl) and particularly preferably 3 to 7 (C₃-C₇-cycloalkyl) carbon atoms.

The term "C₁-C₆-alkylcarbonylamino" means the group alkyl-C(=O)-NH- having generally 1 to 6 (C₁-C₆-alkylcarbonylamino), preferably 1 to 4 and particularly preferably 1 to 3 carbon atoms in the alkyl moiety.

The term "phenyl-C₁-C₆-alkyl-" is understood to mean a group composed of an optionally substituted phenyl radical and a C₁-C₆-alkyl group, and bonded to the rest of the molecule via the C₁-C₆-alkyl group. Here, the C₁-C₆-alkyl is as defined *supra.*
Examples which may be mentioned include benzyl, phenethyl, phenylpropyl, phenylpentyl, with benzyl being preferred.

The term "monocyclic heterocyclyl-" means a non-aromatic monocyclic ring system having one, two or three heteroatoms which may be identical or different. The heteroatoms may be nitrogen atoms, oxygen atoms or sulphur atoms. The sulphur atom may be substituted with oxygene to form a -S(=O)- or -S(=O)₂- group in the ring.
A monocyclic heterocyclyl ring according to the present invention may have 3 to 8, preferably 4 to 7, particularly preferably 5 or 6 ring atoms.

By way of example and with preference, the following may be mentioned for monocyclic heterocyclyl radicals having 3 ring atoms:
aziridinyl-.

By way of example and with preference, the following may be mentioned for monocyclic heterocyclyl radicals having 4 ring atoms:
azetidinyl-, oxetanyl- and 1,1-dioxidothietanyl-.

By way of example and with preference, the following may be mentioned for monocyclic heterocyclyl radicals having 5 ring atoms:
pyrrolidinyl-, imidazolidinyl-, pyrazolidinyl-, pyrrolinyl-, dioxolanyl-, 1,1-dioxidotetrahydro-thiophen-yl-, oxopyrrolidinyl- and tetrahydrofuranyl-.

By way of example and with preference, the following may be mentioned for monocyclic heterocyclyl radicals having 6 ring atoms:
piperidinyl-, piperazinyl-, morpholinyl-, dioxanyl-, tetrahydropyranyl-, 1,1-dioxidotetrahydro-2H-thiopyranyl-, oxopiperidinyl- and thiomorpholinyl-.

By way of example and with preference, the following may be mentioned for monocyclic heterocyclyl radicals having 7 ring atoms:
azepanyl-, oxepanyl-, 1,3-diazepanyl-, 1,4-diazepanyl-.

By way of example and with preference, the following may be mentioned for monocyclic heterocyclyl radicals having 8 ring atoms:
oxocanyl-, azocanyl-.

From among the monocyclic heterocyclyl radicals, preference is given to 4- to 7-membered saturated heterocyclyl radicals having up to two heteroatoms from the group consisting of O, N and S.
Particular preference is given to morpholinyl-, piperidinyl- and pyrrolidinyl-.

The term "4- to 10-membered heterocycloalkyl" means a monocyclic, saturated heterocycle with 4, 5, 6, 7, 8, 9 or 10 ring atoms in total, which contains one or two identical or different ring heteroatoms from the series N and O, it being possible for said heterocycloalkyl group to be attached to the rest of the molecule via any one of the carbon atoms or, if present, a nitrogen atom. The term "heterocycloalkyl" is as defined *supra.*
Said heterocycloalkyl group, without being limited thereto, can be a 4-membered ring, such as azetidinyl or oxetanyl, for example; or a 5-membered ring, such as tetrahydrofuranyl, 1,3-dioxolanyl, pyrrolidinyl, imidazolidinyl, pyrazolidinyl, 1,2-oxazolidinyl or 1,3-oxazolidinyl, for example; or a 6-membered ring, such as tetrahydropyranyl, piperidinyl, morpholinyl, piperazinyl, 1,3-dioxanyl, 1,4-dioxanyl or 1,2-oxazinanyl, for example.
Particularly, "4- to 6-membered heterocycloalkyl" means a 4- to 6-membered heterocycloalkyl as defined *supra* containing one ring oxygen atom and optionally one further ring heteroatom from the series: N, O. More particularly, "5- or 6-membered heterocycloalkyl" means a monocyclic, saturated heterocycle with 5 or 6 ring atoms in total, containing one ring oxygen atom.

The term "monocyclic heteroaryl" means a monovalent, aromatic ring having 5 or 6 ring atoms (a "5- or 6-membered heteroaryl" group), which contains at least one ring heteroatom and optionally one or two further ring heteroatoms from the series: N, O and/or S, and which is bound via a ring carbon atom or optionally via a ring nitrogen atom (if allowed by valency). Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl.
In general, and unless otherwise mentioned, the heteroaryl or heteroarylene groups include all possible isomeric forms thereof, *e.g.:* tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.
Particularly, the heteroaryl group is a isothiazolyl, pyrazolyl, pyridinyl, pyridazinyl or pyrimidinyl group.

The terms "spirocycloalkyl" and "heterospirocycloalkyl" mean C₅-C₁₂-spirocycloalkyl or C₅-C₁₂-heterospirocycloalkyl where one, two, three or four carbon atoms are replaced by heteroatoms as defined above in any combination is understood to mean a fusion of two saturated ring systems which share one common atom.
Examples are spiro[2.2]pentyl, spiro[2.3]hexyl, azaspiro[2.3]hexyl, spiro[3.3]heptyl, azaspiro[3.3]heptyl, oxaazaspiro[3.3]heptyl, thiaazaspiro[3.3]heptyl, oxaspiro[3.3]heptyl, oxazaspiro[3.5]nonyl, oxazaspiro[3.4]octyl, oxazaspiro[5.5]undecyl, diazaspiro[3.3]heptyl, thiazaspiro[3.3]heptyl, thiazaspiro[3.4]octyl, azaspiro[5.5]decyl, and the further homologous spiro[3.4], spiro[4.4], spiro[5.5], spiro[6.6], spiro[2.4], spiro[2.5], spiro[2.6], spiro[3.5], spiro[3.6], spiro[4.5], spiro[4.6] and spiro[5.6] systems including the variants modified by heteroatoms as per the definition. Preference is given to C₆-C₁₀-heterospirocycloalkyl-, by way of example and with particular preference 2-azaspiro[3.3]heptyl-, 2,2-dioxido-2-thia-6-azaspiro[3.3]heptyl. 1,1-dioxido-1-thia-6-azaspiro[3.3]heptyl, 1-thia-6-azaspiro[3.3]heptyl-, 2-thia-6-azaspiro[3.3]heptyl-, 2-oxa-6-azaspiro[3.3]heptyl-,2,6-diazaspiro[3.3]heptyl-, 2-oxa-6-azaspiro[3.4]octyl-, 2-oxa-6-azaspiro[3.5]nonyl-,2-oxa-7-azaspiro[3.5]nonyl-, 8-azaspiro[4.5]decyl-, 2,8-diazaspiro[4.5]decyl- or 3-oxa-1,8-diazaspiro[4.5]decyl-.

The terms "bicycloalkyl" and "heterobicycloalkyl" mean C₆-C₁₂-bicycloalkyl or C₆-C₁₂-heterobicycloalkyl where one, two, three or four carbon atoms are replaced by heteroatoms as defined above in any combination is understood to mean a fusion of two saturated ring systems which share two directly adjacent atoms.
Examples are radicals derived from bicyclo[2.2.0]hexyl-, bicyclo[3.3.0]octyl-, bicyclo[4.4.0]decyl-, bicyclo[5.4.0]undecyl-, bicyclo[3.2.0]heptyl-, bicyclo[4.2.0]octyl-, bicyclo[5.2.0]nonyl-, bicyclo[6.2.0]decyl-, bicyclo[4.3.0]nonyl-, bicyclo[5.3.0]decyl-, bicyclo[6.3.0]undecyl- and bicyclo[5.4.0]undecyl-, including the variants modified by heteroatoms, for example azabicyclo[3.3.0]octyl-, azabicyclo[4.3.0]nonyl-, diazabicyclo[4.3.0]nonyl-, oxazabicyclo[4.3.0]nonyl-, thiazabicyclo[4.3.0]nonyl- or azabicyclo[4.4.0]decyl-, and the further possible combinations as per the definition. Preference is given to C₆-C₁₀-heterobicycloalkyl-, by way of example and with particular preference perhydrocyclopenta[c]pyrrolyl-, perhydrofuro[3,2-c]pyridinyl-, perhydropyrrolo[1,2-a]pyrazinyl-, perhydropyrrolo[3,4-c]pyrrolyl-.
Preferred examples of C₆-C₁₂-bicycloalkyl- are perhydronaphthalenyl- (decalinyl-), perhydrobenzoannulenyl-, perhydroazulenyl-, perhydroindanyl-, perhydropentalenyl-.

The terms "bridged cycloalkyl" and "bridged heterocycloalkyl" mean a bridged C₆-C₁₂ ring system such as bridged C₆-C₁₂-cycloalkyl- or bridged C₆-C₁₂-heterocycloalkyl- is understood to mean a fusion of at least two saturated rings which share two atoms that are not directly adjacent to one another. This may give rise either to a bridged carbocycle (bridged cycloalkyl-) or to a bridged heterocycle (bridged heterocycloalkyl-) where one, two, three or four carbon atoms are replaced by heteroatoms as defined above in any combination.
Examples are bicyclo[2.2.1]heptyl-, azabicyclo[2.2.1]heptyl-, oxazabicyclo[2.2.1]heptyl-, thiazabicyclo[2.2.1]heptyl-, diazabicyclo[2.2.1]heptyl-, bicyclo[2.2.2]octyl-, azabicyclo[2.2.2]octyl-, diazabicyclo[2.2.2]octyl-, oxazabicyclo[2.2.2]octyl-, thiazabicyclo[2.2.2]octyl-, bicyclo[3.2.1]octyl-, azabicyclo[3.2.1]octyl-, diazabicyclo[3.2.1]octyl-, oxazabicyclo[3.2.1]octyl-, thiazabicyclo[3.2.1]octyl-, bicyclo[3.3.1]nonyl-, azabicyclo[3.3.1]nonyl-, diazabicyclo[3.3.1]nonyl- oxazabicyclo[3.3.1]nonyl-, thiazabicyclo[3.3.1]nonyl-, bicyclo[4.2.1]nonyl-, azabicyclo[4.2.1]nonyl-, diazabicyclo[4.2.1]nonyl-, oxazabicyclo[4.2.1]nonyl-, thiazabicyclo[4.2.1]nonyl-, bicyclo[3.3.2]decyl-, azabicyclo[3.3.2]decyl-, diazabicyclo[3.3.2]decyl-, oxazabicyclo[3.3.2]decyl-, thiazabicyclo[3.3.2]decyl- or azabicyclo[4.2.2]decyl- and the further possible combinations according to the definition. Preference is given to bridged C₆-C₁₀-heterocycloalkyl-, by way of example and with particular preference
2-azabicyclo[2.2.1]heptyl-, 2,5-diazabicyclo[2.2.1]heptyl-, 2-oxa-5-azabicyclo[2.2.1]heptyl-, 8-azabicyclo[3.2.1]octyl-, 8-oxa-3-azabicyclo[3.2.1]octyl-, 3,9-diazabicyclo[4.2.1]nonyl-.

Where the plural form of the word compounds, salts, polymorphs, hydrates, solvates and the like, is used herein, this is taken to mean also a single compound, salt, polymorph, isomer, hydrate, solvate or the like.
By "stable compound' or "stable structure" is meant a compound that is sufficiently robust to survive isolation to a useful degree of purity from a reaction mixture, and formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.

Preferred compounds are those which produce the more desirable biological activity.
Separated, pure or partially purified isomers and stereoisomers or racemic or diastereomeric mixtures of the compounds of the present invention are also included within the scope of the present invention. The purification and the separation of such materials can be accomplished by standard techniques known in the art.

Preferred isomers are those which produce the more desirable biological activity. These separated, pure or partially purified isomers or racemic mixtures of the compounds of this invention are also included within the scope of the present invention. The purification and the separation of such materials can be accomplished by standard techniques known in the art. The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.,* HPLC columns using a chiral phase), with or without conventional derivatisation, optimally chosen to maximise the separation of the enantiomers. Suitable HPLC columns using a chiral phase are commercially available, such as those manufactured by Daicel, *e.g.,* Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.
In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* (R)- or (S)-isomers, in any ratio. Isolation of a single stereoisomer, *e.g.* a single enantiomer or a single diastereomer, of a compound of the present invention is achieved by any suitable state of the art method, such as chromatography, especially chiral chromatography, for example.

Further, the compounds of the present invention can exist as N-oxides, which are defined in that at least one nitrogen of the compounds of the present invention is oxidised. The present invention includes all such possible N-oxides.

The present invention also covers useful forms of the compounds of the present invention, such as metabolites, hydrates, solvates, prodrugs, salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, e.g. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N*-methylpiperidine, *N*-methyl-glucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(*n*-butyl)ammonium, *N*-benzyl-*N,N,N-*trimethylammonium, choline or benzalkonium.

Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.
The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown. Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.
This applies analogously to cases in which synthesis intermediates or example compounds or salts thereof have been obtained, by the preparation and/or purification processes described, as solvates, such as hydrates, with (if defined) unknown stoichiometric composition. Furthermore, the present invention includes all possible crystalline forms, or polymorphs, of the compounds of the present invention, either as single polymorph, or as a mixture of more than one polymorph, in any ratio.

Moreover, the present disclosure also includes prodrugs of the compounds according to the invention. The term "prodrugs" here designates compounds which themselves can be biologically active or inactive, but are converted (for example metabolically or hydrolytically) into compounds according to the invention during their residence time in the body.
The invention further includes all possible crystallized and polymorphic forms of the inventive compounds, whereby the polymorphs are existing either as a single polymorph form or are existing as a mixture of several polymorphs in all concentrations.
The invention further includes all possible cyclodextrin clathrates, i.e alpha-, beta-, or gamma-cyclodextrins, hydroxypropyl-beta-cyclodextrins, methylbetacyclodextrins.

Of selected interest are those compounds of formula (I), in which
- X: represents a nitrogen, a sulphur or an oxygene atom,
- Y: represents a sulphur or an oxygene atom,
- R¹: represents hydrogen, halogen, cyano, C₁-C₃-alkyl or halo-C₁-C₆-alkyl,
- R^{2a}: represents C₁-C₃-alkoxy, halo-C₁-C₃-alkyl or halo-C₁-C₃-alkoxy
- R^{2b}: represents hydrogen, halogen, cyano or C₁-C₃-alkyl,
- R³: represents hydrogen,
- R⁴: represents hydrogen or represents C₁-C₆-alkyl-,
which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy-,
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms, or
- R⁴: represents monocyclic heteroaryl- having 5 or 6 ring atoms which may optionally be mono- or polysubstituted by identical or different substituents from the group consisting of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-,
and their polymorphs, enantiomers, diastereomers, racemates, tautomers, solvates, physiologically acceptable salts and solvates of these salts.

Of selected interest are those compounds of formula (I), in which
- X: represents a nitrogen, a sulphur or an oxygene atom,
- Y: represents a sulphur or an oxygene atom,
- R¹: represents halogen or perfluoro-C₁-C₃-alkyl,
- R^{2a}: represents C₁-C₃-alkoxy, perfluoro-C₁-C₃-alkyl or perfluoro-C₁-C₃-alkoxy
- R^{2b}: represents hydrogen or halogen,
- R³: represents hydrogen or C₁-C₆-alkyl,
- R⁴: represents hydrogen or represents C₁-C₆-alkyl-, which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
- R⁴: represents monocyclic heteroaryl- having 6 ring atoms which may optionally be mono- or polysubstituted by identical or different substituents from the group consisting of of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-Ci₀-cycloalkyl-,
and their polymorphs, enantiomers, diastereomers, racemates, tautomers, solvates, physiologically acceptable salts and solvates of these salts.

Of selected interest are those compounds of formula (I), in which
- X: represents an oxygene atom,
- Y: represents an oxygene atom,
- R¹: represents chlorine or trifluoromethyl,
- R^{2a}: represents methoxy, trifluoromethyl or trifluoromethoxy,
- R^{2b}: represents hydrogen or fluorine,
- R³: represents hydrogen,
- R⁴: represents hydrogen or represents C₂- or C₃-alkyl-, which is substituted by 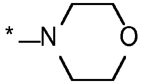
- R⁴: represents a pyrimidinyl or pyridazinyl which may optionally be mono- or polysubstituted by C₁-C₆-alkyl-,
and their polymorphs, enantiomers, diastereomers, racemates, tautomers, solvates, physiologically acceptable salts and solvates of these salts.

Compounds of most interest are those as follows:
- 1-[2-(morpholin-4-yl)ethyl]-3-[3-(trifluoromethyl)-4-{[3-(trifluoromethyl)-1H-pyrrolo[2,3-b]pyridin-4-yl]oxy}phenyl]urea
- 1-[3-(morpholin-4-yl)propyl]-3-[3-(trifluoromethyl)-4-{[3-(trifluoromethyl)-1H-pyrrolo[2,3-b]pyridin-4-yl]oxy}phenyl]urea
- 1-[3-(trifluoromethyl)-4-{[3-(trifluoromethyl)-1H-pyrrolo[2,3-b]pyridin-4-yl]oxy}phenyl]urea
- 1-[3-fluoro-5-(trifluoromethyl)-4-{[3-(trifluoromethyl)-1H-pyrrolo[2,3-b]pyridin-4-yl]oxy}phenyl]-3-[3-(morpholin-4-yl)propyl]urea
- 1-[3-fluoro-5-(trifluoromethyl)-4-{[3-(trifluoromethyl)-1H-pyrrolo[2,3-b]pyridin-4-yl]oxy}phenyl]-3-[2-(morpholin-4-yl)ethyl]urea
- 1-{4-[(3-chloro-1 H-pyrrolo[2,3-b]pyridin-4-yl)oxy]-3-(trifluoromethyl)phenyl}-3-[3-(morpholin-4-yl)propyl]urea
- 1-{4-[(3-chloro-1 H-pyrrolo[2,3-b]pyridin-4-yl)oxy]-3-(trifluoromethyl)phenyl}-3-[2-(morpholin-4-yl)ethyl]urea
- 1-{4-[(3-chloro-1 H-pyrrolo[2,3-b]pyridin-4-yl)oxy]-3-(trifluoromethyl)phenyl}urea
- 1-{4-[(3-chloro-1H-pyrrolo[2,3-b]pyridin-4-yl)oxy]-3-(trifluoromethyl)phenyl}-3-pyridazin-3-ylurea
- 1-{4-[(3-chloro-1H-pyrrolo[2,3-b]pyridin-4-yl)oxy]-3-(trifluoromethyl)phenyl}-3-(2-methylpyrimidin-5-yl)urea
- 1-(3-methoxy-4-{[3-(trifluoromethyl)-1H-pyrrolo[2,3-b]pyridin-4-yl]oxy}phenyl)-3-[3-(morpholin-4-yl)propyl]urea
- 1-[3-(morpholin-4-yl)propyl]-3-[3-(trifluoromethoxy)-4-{[3-(trifluoromethyl)-1H-pyrrolo[2,3-b]pyridin-4-yl]oxy}phenyl]urea
- 1-[2-(morpholin-4-yl)ethyl]-3-[3-(trifluoromethoxy)-4-{[3-(trifluoromethyl)-1 H-pyrrolo[2,3-b]pyridin-4-yl]oxy}phenyl]urea
and their polymorphs, enantiomers, diastereomers, racemates, tautomers, solvates, physiologically acceptable salts and solvates of these salts.

In accordance with a further embodiment, the present invention covers compounds of general formula (I), in which X represents an oxygene atom.

In accordance with a further embodiment, the present invention covers compounds of general formula (I), in which Y represents an oxygene atom.

In accordance with a further embodiment, the present invention covers compounds of general formula (I), in which X and Y represent an oxygene atom.

In accordance with a further embodiment, the present invention covers compounds of general formula (I), in which R¹ represents hydrogen, halogen, cyano, C₁-C₃-alkyl or halo-C₁-C₃-alkyl.

In accordance with a further embodiment, the present invention covers compounds of general formula (I), in which R¹ represents halogen or halo-C₁-C₆-alkyl.

In accordance with a further embodiment, the present invention covers compounds of general formula (I), in which R¹ represents halogen or perfluoro-C₁-C₃-alkyl.

In accordance with a further embodiment, the present invention covers compounds of general formula (I), in which R¹ represents chlorine or trifluoromethyl.

In accordance with a further embodiment, the present invention covers compounds of general formula (I), in which R^{2a} represents C₁-C₃-alkoxy, halo-C₁-C₃-alkyl or halo-C₁-C₃-alkoxy.

In accordance with a further embodiment, the present invention covers compounds of general formula (I), in which R^{2a} represents C₁-C₃-alkoxy, perfluoro-C₁-C₃-alkyl or perfluoro-C₁-C₃-alkoxy.

In accordance with a further embodiment, the present invention covers compounds of general formula (I), in which R^{2a} represents methoxy, trifluoromethyl or trifluoromethoxy.

In accordance with a further embodiment, the present invention covers compounds of general formula (I), in which R^{2a} represents methoxy, trifluoromethyl or trifluoromethoxy and R^{2b} represents hydrogen or fluorine.

In accordance with a further embodiment, the present invention covers compounds of general formula (I), in which R^{2b} represents hydrogen or halogen.

In accordance with a further embodiment, the present invention covers compounds of general formula (I), in which R^{2b} represents hydrogen or fluorine.

In accordance with a further embodiment, the present invention covers compounds of general formula (I), in which R^{2b} represents hydrogen or fluorine and R³ represents hydrogen.

In accordance with a further embodiment, the present invention covers compounds of general formula (I), in which R³ represents hydrogen.

In accordance with a further embodiment, the present invention covers compounds of general formula (I), in which X and Y represent an oxygene atom, R¹ represents trifluoromethyl and R³ represents hydrogen.

In accordance with a further embodiment, the present invention covers compounds of general formula (I), in which R⁴ represents 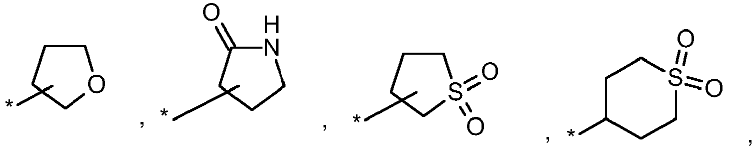 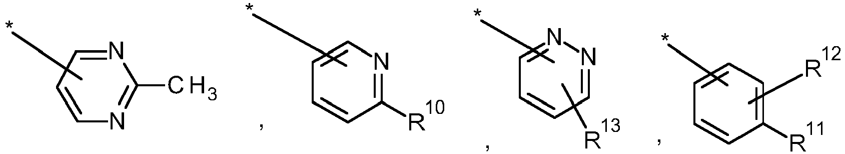 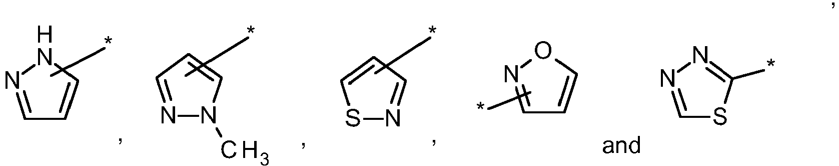 where "*" denotes the point of attachment to the remainder of the molecule. In accordance with a further embodiment, the present invention covers compounds of general formula (I), in which R⁴ represents 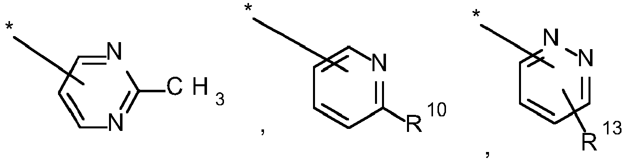 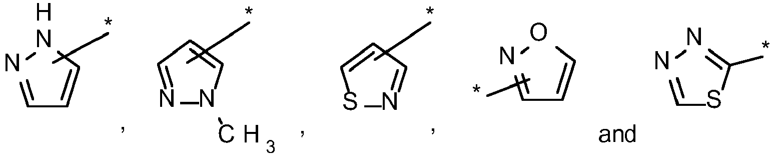 where "*" denotes the point of attachment to the remainder of the molecule. In accordance with a further embodiment, the present invention covers compounds of general formula (I), in which R⁴ represents C₁-C₆-alkyl-, which may optionally be mono- or disubstituted by identical or different substituents from the group consisting of 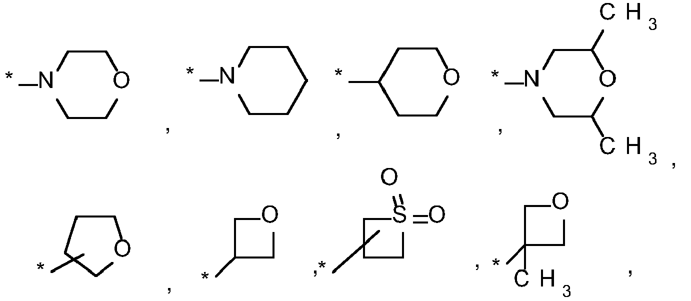 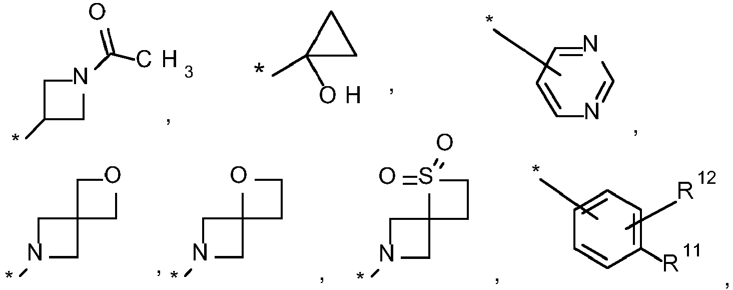 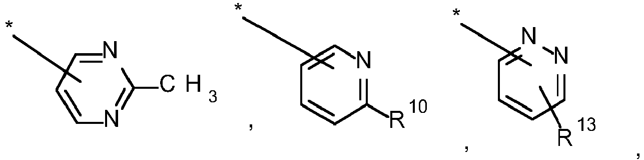 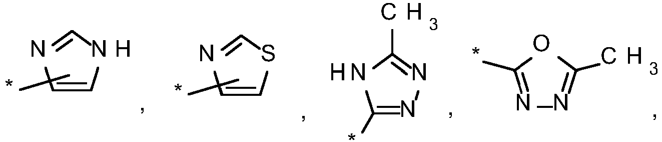 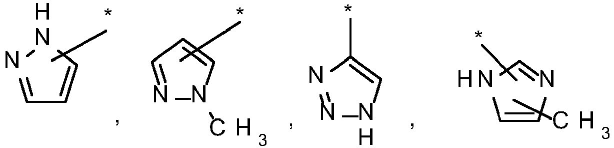 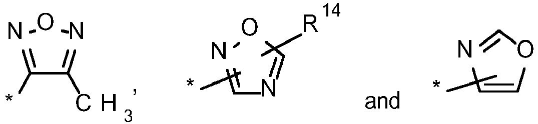 where "*" denotes the point of attachment to the remainder of the molecule. In accordance with a further embodiment, the present invention covers compounds of general formula (I), in which R⁴ represents C₁-C₆-alkyl-, which may optionally be mono- or disubstituted by identical or different substituents from the group consisting of 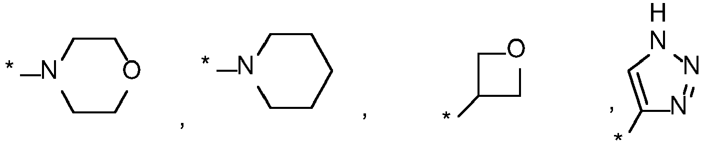 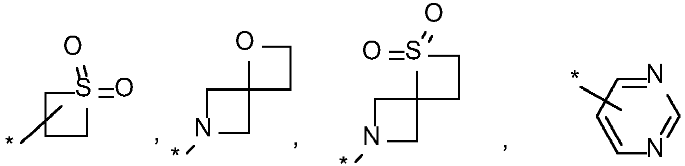 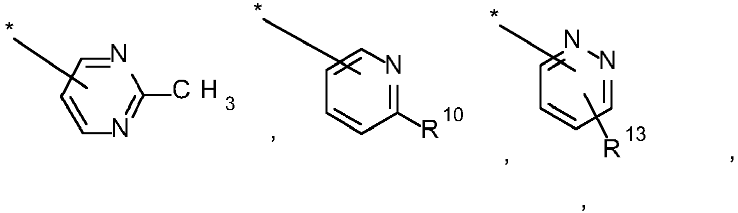 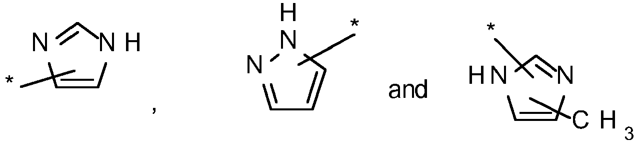 where "*" denotes the point of attachment to the remainder of the molecule.

In accordance with a further embodiment, the present invention covers compounds of general formula (I), in which R⁴ represents hydrogen, C₁-C₆-alkyl-, which may optionally be mono- or disubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy-, -S(=O)₂NH₂, -C(=O)-R⁹, -C(=O)-NH-R^{x}, -NH-C(=O)-R⁹, -NH-S(=O)₂-R⁹, -S(=O)₂-R⁹, in which R⁹ represents methyl, -NH₂ or -CH₂-CH₂-O-CH₃ or 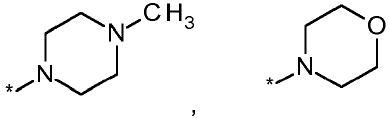 where "*" denotes the point of attachment to the remainder of the molecule. and R^{x} represents methyl or -CH₂-CH₂-O-CH₃.

In accordance with a further embodiment, the present invention covers compounds of general formula (I), in which R⁴ represents C₁-C₆-alkyl-, which may optionally be substituted by 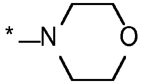 or
R⁴ represents a pyrimidinyl or pyridazinyl which may optionally be mono- or polysubstituted by C₁-C₆-alkyl-,
where "*" denotes the point of attachment to the remainder of the molecule.

In accordance with a further embodiment, the present invention covers compounds of general formula (I), in which R⁴ represents C₂- or C₃-alkyl-, which is substituted by 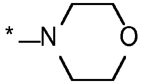 where "*" denotes the point of attachment to the remainder of the molecule.

The compounds of general formula (I) of the present invention can be converted to any salt, preferably pharmaceutically acceptable salts, as described herein, by any method which is known to the person skilled in the art. Similarly, any salt of a compound of general formula (I) of the present invention can be converted into the free compound, by any method which is known to the person skilled in the art.

Compounds of general formula (I) of the present invention demonstrate a valuable pharmacological spectrum of action, which could not have been predicted. Compounds of the present invention have surprisingly been found to effectively inhibit MAP4K1 and it is possible therefore that said compounds be used for the treatment or prophylaxis of diseases, preferably cancer or conditions with dysregulated immune responses or other disorders associated with aberrant MAP4K1 signaling, in humans and animals.
Disorders and conditions particularly suitable for treatment with an MAP4K1 inhibitor of the present invention are liquid and solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancers include, but are not limited to, triple negative breast cancer, invasive ductal carcinoma, invasive lobular carcinoma, ductal carcinoma *in situ,* and lobular carcinoma *in situ.*
Examples of cancers of the respiratory tract include, but are not limited to, small-cell and non-small-cell lung carcinoma, as well as bronchial adenoma and pleuropulmonary blastoma. Examples of brain cancers include, but are not limited to, brain stem and hypophtalmic glioma, cerebellar and cerebral astrocytoma, glioblastoma, medulloblastoma, ependymoma, as well as neuroectodermal and pineal tumour.
Tumours of the male reproductive organs include, but are not limited to, prostate and testicular cancer.
Tumours of the female reproductive organs include, but are not limited to, endometrial, cervical, ovarian, vaginal, and vulvar cancer, as well as sarcoma of the uterus.
Examples of ovarian cancer include, but are not limited to serous tumour, endometrioid tumour, mucinous cystadenocarcinoma, granulosa cell tumour, Sertoli-Leydig cell tumour and arrhenoblastoma.
Examples of cervical cancer include, but are not limited to squamous cell carcinoma, adenocarcinoma, adenosquamous carcinoma, small cell carcinoma, neuroendocrine tumour, glassy cell carcinoma and villoglandular adenocarcinoma.
Tumours of the digestive tract include, but are not limited to, anal, colon, colorectal, esophageal, gallbladder, gastric, pancreatic, rectal, small-intestine, and salivary gland cancers. Examples of esophageal cancer include, but are not limited to esophageal cell carcinomas and adenocarcinomas, as well as squamous cell carcinomas, leiomyosarcoma, malignant melanoma, rhabdomyosarcoma and lymphoma.
Examples of gastric cancer include, but are not limited to intestinal type and diffuse type gastric adenocarcinoma.
Examples of pancreatic cancer include, but are not limited to ductal adenocarcinoma, adenosquamous carcinomas and pancreatic endocrine tumours.
Tumours of the urinary tract include, but are not limited to, bladder, penile, kidney, renal pelvis, ureter, urethral and human papillary renal cancers.
Examples of kidney cancer include, but are not limited to renal cell carcinoma, urothelial cell carcinoma, juxtaglomerular cell tumour (reninoma), angiomyolipoma, renal oncocytoma, Bellini duct carcinoma, clear-cell sarcoma of the kidney, mesoblastic nephroma and Wilms' tumour. Examples of bladder cancer include, but are not limited to transitional cell carcinoma, squamous cell carcinoma, adenocarcinoma, sarcoma and small cell carcinoma.
Eye cancers include, but are not limited to, intraocular melanoma and retinoblastoma. Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.
Skin cancers include, but are not limited to, squamous cell carcinoma, Kaposi's sarcoma, malignant melanoma, Merkel cell skin cancer, and non-melanoma skin cancer. Head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.
Lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.
Sarcomas include, but are not limited to, sarcoma of the soft tissue, osteosarcoma, malignant fibrous histiocytoma, lymphosarcoma, and rhabdomyosarcoma.
Leukemias include, but are not limited to, acute myeloid leukemia, acute lymphoblastic leukemia, chronic lymphocytic leukemia, chronic myelogenous leukemia, and hairy cell leukemia.

The term "treating" or "treatment" as stated throughout this document is used conventionally, for example the management or care of a subject for the purpose of combating, alleviating, reducing, relieving, improving the condition of a disease or disorder, such as a carcinoma. The compounds of the present invention can be used in particular in therapy and prevention, *i.e.* prophylaxis, of tumour growth and metastases, especially in solid tumours of all indications and stages with or without pre-treatment of the tumour growth.
Generally, the use of chemotherapeutic agents and/or anti-cancer agents in combination with a compound or pharmaceutical composition of the present invention will serve to:
1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of general formula (I) of the present invention can also be used in combination with radiotherapy and/or surgical intervention.

In a further embodiment of the present invention, the compounds of general formula (I) of the present invention may be used to sensitize a cell to radiation, *i.e.* treatment of a cell with a compound of the present invention prior to radiation treatment of the cell renders the cell more susceptible to DNA damage and cell death than the cell would be in the absence of any treatment with a compound of the present invention. In one aspect, the cell is treated with at least one compound of general formula (I) of the present invention.
Thus, the present invention also provides a method of killing a cell, wherein a cell is administered one or more compounds of the present invention in combination with conventional radiation therapy.

The present invention also provides a method of rendering a cell more susceptible to cell death, wherein the cell is treated with one or more compounds of general formula (I) of the present invention prior to the treatment of the cell to cause or induce cell death. In one aspect, after the cell is treated with one or more compounds of general formula (I) of the present invention, the cell is treated with at least one compound, or at least one method, or a combination thereof, in order to cause DNA damage for the purpose of inhibiting the function of the normal cell or killing the cell.

In other embodiments of the present invention, a cell is killed by treating the cell with at least one DNA damaging agent, *i.e.* after treating a cell with one or more compounds of general formula (I) of the present invention to sensitize the cell to cell death, the cell is treated with at least one DNA damaging agent to kill the cell. DNA damaging agents useful in the present invention include, but are not limited to, chemotherapeutic agents (e.g. cis platin), ionizing radiation (X-rays, ultraviolet radiation), carcinogenic agents, and mutagenic agents.
In other embodiments, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In one aspect of the invention, a compound of general formula (I) of the present invention is administered to a cell prior to the radiation or other induction of DNA damage in the cell. In another aspect of the invention, a compound of general formula (I) of the present invention is administered to a cell concomitantly with the radiation or other induction of DNA damage in the cell. In yet another aspect of the invention, a compound of general formula (I) of the present invention is administered to a cell immediately after radiation or other induction of DNA damage in the cell has begun.

In another aspect, the cell is in vitro. In another embodiment, the cell is in vivo.

The compounds of the present invention can be administered as the sole pharmaceutical agent or in combination with one or more other pharmaceutically active ingredients where the combination causes no unacceptable adverse effects. The present invention also covers such pharmaceutical combinations. For example, the compounds of the present invention can be combined with: ¹³¹I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (¹²¹I), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, roniciclib , samarium (¹⁵³Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (^{99m}Tc) nofetumomab merpentan, ^{99m}Tc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

The compounds of the invention can further be combined with other reagents targeting the immune system, such as immune checkpoint inhibitors, e.g. aPD-1/-L1 axis antagonists. PD-1, along with its ligands PD-L1 and PD-L2, function as negative regulators of T cell activation. MAP4K1 suppresses immune cell function. PD-L1 is overexpressed in many cancers and overexpression of PD-1 often occurs concomitantly in tumor infiltrating T cells. Thus results in attenuation of T cell activation and evasion of immune surveillance, which contributes to impaired antitumor immune responses. (Keir M E et al. (2008) Annu. Rev. Immunol. 26:677).

In addition, the inventive compounds can also be used as a therapeutic in a variety of other disorders wherein MAP4K1 is involved such as, cardiovascular and lung diseases. Accordingly, the compounds according to the invention are suitable for the treatment and/or prophylaxis in particular of cardiovascular, inflammatory and fibrotic disorders and of renal disorders, in particular of acute and chronic renal insufficiency, and also of acute and chronic renal failure.

Accordingly, the compounds according to the invention can be used in medicaments for the treatment and/or prophylaxis of cardiovascular, inflammatory and fibrotic disorders, renal disorders, in particular of acute and chronic renal insufficiency, and also of acute and chronic renal failure.

For the purpose of the present invention the term renal insufficiency comprises both acute and chronic manifestations of renal insufficiency, and also underlying or related renal disorders such as diabetic and non-diabetic nephropathies, hypertensive nephropathies, ischaemic renal disorders, renal hypoperfusion, intradialytic hypotension, obstructive uropathy, renal stenoses, glomerulopathies, glomerulonephritis (such as, for example, primary glomerulonephritides; minimal change glomerulonephritis (lipoidnephrosis); membranous glomerulonephritis; focal segmental glomerulosclerosis (FSGS); membrane-proliferative glomerulonephritis; crescentic glomerulonephritis; mesangioproliferative glomerulonephritis (IgA nephritis, Berger's disease); post-infectious glomerulonephritis; secondary glomerulonephritides: diabetes mellitus, lupus erythematosus, amyloidosis, Goodpasture syndrome, Wegener granulomatosis, Henoch-Schönlein purpura, microscopic polyangiitis, acute glomerulonephritis, pyelonephritis (for example as a result of: urolithiasis, benign prostate hyperplasia, diabetes, malformations, abuse of analgesics, Crohn's disease), glomerulosclerosis, arteriolonecrose of the kidney, tubulointerstitial diseases, nephropathic disorders such as primary and congenital or aquired renal disorder, Alport syndrome, nephritis, immunological kidney disorders such as kidney transplant rejection and immunocomplex-induced renal disorders, nephropathy induced by toxic substances, nephropathy induced by contrast agents, diabetic and non-diabetic nephropathy, renal cysts, nephrosclerosis, hypertensive nephrosclerosis and nephrotic syndrome which can be characterized diagnostically, for example by abnormally reduced creatinine and/or water excretion, abnormally elevated blood concentrations of urea, nitrogen, potassium and/or creatinine, altered activity of renal enzymes, for example glutamyl synthetase, altered urine osmolarity or urine volume, elevated microalbuminuria, macroalbuminuria, lesions on glomerulae and arterioles, tubular dilatation, hyperphosphataemia and/or the need for dialysis. The present invention also comprises the use of the compounds according to the invention for the treatment and/or prophylaxis of sequelae of renal insufficiency, for example pulmonary oedema, heart failure, uremia, anemia, electrolyte disturbances (for example hypercalemia, hyponatremia) and disturbances in bone and carbohydrate metabolism.

The present invention also comprises the use of the compounds according to the invention for the treatment and/or prevention of sequelae of renal insufficiency, for example pulmonary oedema, heart failure, uraemia, anaemia, electrolyte disturbances (for example hyperkalaemia, hyponatraemia) and disturbances in bone and carbohydrate metabolism.

The compounds according to the invention are further suitable for the treatment and/or prevention of polycystic kidney disease (PCKD) and of the syndrome of inappropriate ADH secretion (SIADH).

Furthermore, the compounds according to the invention are also suitable for the treatment and/or prophylaxis of metabolic syndrome, hypertension, resistant hypertension, acute and chronic heart failure, coronary heart disease, stable and unstable angina pectoris, peripheral and cardiac vascular disorders, arrhythmias, atrial and ventricular arrhythmias and impaired conduction, for example atrioventricular blocks degrees I-III (AB block I-III), supraventricular tachyarrhythmia, atrial fibrillation, atrial flutter, ventricular fibrillation, ventricular flutter, ventricular tachyarrhythmia, Torsade de pointes tachycardia, atrial and ventricular extrasystoles, AV-junctional extrasystoles, sick sinus syndrome, syncopes, AV-nodal re-entry tachycardia, Wolff-Parkinson-White syndrome, of acute coronary syndrome (ACS), autoimmune cardiac disorders (pericarditis, endocarditis, valvolitis, aortitis, cardiomyopathies), shock such as cardiogenic shock, septic shock and anaphylactic shock, aneurysms, boxer cardiomyopathy (premature ventricular contraction (PVC)), for treatment and/or prophylaxis of thromboembolic disorders and ischaemias such as myocardial ischaemia, myocardial infarction, stroke, cardiac hypertrophy, transient and ischaemic attacks, preeclampsia, inflammatory cardiovascular disorders, spasms of the coronary arteries and peripheral arteries, oedema formation, for example pulmonary oedema, cerebral oedema, renal oedema or oedema caused by heart failure, peripheral circulatory disturbances, reperfusion damage, arterial and venous thromboses, myocardial insufficiency, endothelial dysfunction, to prevent restenoses, for example after thrombolysis therapies, percutaneous transluminal angioplasties (PTA), transluminal coronary angioplasties (PTCA), heart transplants and bypass operations, and also micro- and macrovascular damage (vasculitis), increased levels of fibrinogen and of low-density lipoprotein (LDL) and increased concentrations of plasminogen activator inhibitor 1 (PAI-1), and also for treatment and/or prophylaxis of erectile dysfunction and female sexual dysfunction.

In addition, the compounds according to the invention are also suitable for treatment and/or prophylaxis of asthmatic disorders, pulmonary arterial hypertension (PAH) and other forms of pulmonary hypertension (PH) including left-heart disease, HIV, sickle cell anaemia, thromboembolisms (CTEPH), sarcoidosis, COPD or pulmonary fibrosis-associated pulmonary hypertension, chronic-obstructive pulmonary disease (COPD), acute respiratory distress syndrome (ARDS), acute lung injury (ALI), alpha-1-antitrypsin deficiency (AATD), pulmonary fibrosis, pulmonary emphysema (for example pulmonary emphysema induced by cigarette smoke) and cystic fibrosis (CF).

The compounds described in the present invention are also active compounds for control of central nervous system disorders characterized by disturbances of the NO/cGMP system. They are suitable in particular for improving perception, concentration, learning or memory after cognitive impairments like those occurring in particular in association with situations/diseases/syndromes such as mild cognitive impairment, age-associated learning and memory impairments, age-associated memory losses, vascular dementia, craniocerebral trauma, stroke, dementia occurring after strokes (post stroke dementia), post-traumatic craniocerebral trauma, general concentration impairments, concentration impairments in children with learning and memory problems, Alzheimer's disease, Lewy body dementia, dementia with degeneration of the frontal lobes including Pick's syndrome, Parkinson's disease, progressive dementia with corticobasal degeneration, amyolateral sclerosis (ALS), Huntington's disease, demyelinization, multiple sclerosis, thalamic degeneration, Creutzfeld-Jacob dementia, HIV dementia, schizophrenia with dementia or Korsakoff's psychosis. They are also suitable for treatment and/or prophylaxis of central nervous system disorders such as states of anxiety, tension and depression, CNS-related sexual dysfunctions and sleep disturbances, and for controlling pathological disturbances of the intake of food, stimulants and addictive substances.

The compounds according to the invention are furthermore also suitable for controlling cerebral blood flow and thus represent effective agents for controlling migraines. They are also suitable for the prophylaxis and control of sequelae of cerebral infarction (cerebral apoplexy) such as stroke, cerebral ischaemia and craniocerebral trauma. The compounds according to the invention can likewise be used for controlling states of pain and tinnitus.
In addition, the compounds according to the invention have anti-inflammatory action and can therefore be used as anti-inflammatory agents for treatment and/or prophylaxis of sepsis (SIRS), multiple organ failure (MODS, MOF), inflammatory disorders of the kidney, chronic intestinal inflammations (IBD, Crohn's disease, UC), pancreatitis, peritonitis, rheumatoid disorders, inflammatory skin disorders and inflammatory eye disorders.

Furthermore, the compounds according to the invention can also be used for treatment and/or prophylaxis of autoimmune diseases.

The compounds according to the invention are also suitable for treatment and/or prophylaxis of fibrotic disorders of the internal organs, for example the lung, the heart, the kidney, the bone marrow and in particular the liver, and also dermatological fibroses and fibrotic eye disorders. In the context of the present invention, the term fibrotic disorders includes in particular the following terms: hepatic fibrosis, cirrhosis of the liver, pulmonary fibrosis, endomyocardial fibrosis, nephropathy, glomerulonephritis, interstitial renal fibrosis, fibrotic damage resulting from diabetes, bone marrow fibrosis and similar fibrotic disorders, scleroderma, morphea, keloids, hypertrophic scarring (also following surgical procedures), naevi, diabetic retinopathy, proliferative vitroretinopathy and disorders of the connective tissue (for example sarcoidosis).

The compounds according to the invention are also suitable for controlling postoperative scarring, for example as a result of glaucoma operations.

The compounds according to the invention can also be used cosmetically for ageing and keratinized skin.

Moreover, the compounds according to the invention are suitable for treatment and/or prophylaxis of hepatitis, neoplasms, osteoporosis, glaucoma and gastroparesis.

The present invention further provides the use of the compounds according to the invention for treatment and/or prophylaxis of disorders, especially the disorders mentioned above.

The present invention further provides the use of the compounds according to the invention for the treatment and/or prophylaxis of chronic renal disorders, acute and chronic renal insufficiency, diabetic, inflammatory or hypertensive nephropaties, fibrotic disorders, cardiac insufficiency, angina pectoris, hypertension, pulmonary hypertension, ischemias, vascular disorders, thromboembolic disorders, arteriosclerosis, sickle cell anemia, erectile dysfunction, benign prostate hyperplasia, dysuria associated with benign prostate hyperplasia, Huntington, dementia, Alzheimer and Creutzfeld-Jakob.

The present invention further provides a method for treatment and/or prophylaxis of disorders, in particular the disorders mentioned above, using an effective amount of at least one of the compounds according to the invention.

The present invention further provides a method for the treatment and/or prophylaxis of chronic renal disorders, acute and chronic renal insufficiency, diabetic, inflammatory or hypertensive nephropathies, fibrotic disorders, cardiac insufficiency, angina pectoris, hypertension, pulmonary hypertension, ischemias, vascular disorders, thromboembolic disorders, arteriosclerosis, sickle cell anemia, erectile dysfunction, benign prostate hyperplasia, dysuria associated with benign prostate hyperplasia, Huntington, dementia, Alzheimer and Creutzfeld-Jakob.

In another embodiment, the inventive compounds can also be used to treat or to prevent uterine fibroids (uterine leiomyoma or uterine myoma) in women.
Uterine fibroids are benign tumors of the myometrium, the smooth muscle layer of the uterus. Uterine fibroids grow slowly during a women's life, and their growth is dependent on the female sexual hormones estradiol and progesterone [Kawaguchi K et al. Immunohistochemical analysis of oestrogen receptors, progesterone receptors and Ki-67 in leiomyoma and myometrium during the menstrual cycle and pregnancy Virchows Arch A Pathol Anat Histopathol. 1991;419(4):309-15.], therefore the highest prevalence of uterine fibroids with approx. 70% and >80% in white and afro-american women, respectively, is found from 35 years of age onwards to menopause, when they shrink due to reduced hormone levels [Baird DD et al. High cumulative incidence of uterine leiomyoma in black and white women: Ultrasound evidence Am J Obstet Gynecol. 2003 Jan;188(1):100-7.]. Approx 30% and 45% of white and afro-american women, respectively, do show clinically relevant symptoms due to their fibroids, which are heavy menstrual bleeding and pain, which is related to the menstrual cycle [David M et al. Myoma-associated pain frequency and intensity: a retrospective evaluation of 1548 myoma patients. Eur J Obstet Gynecol Reprod Biol. 2016 Apr;199:137-40]. Heavy menstrual bleeding in this respect is defined by a blood loss of more than 80 mL in a menstrual bleeding period [Fraser IS et al. The FIGO Recommendations on Terminologies and Definitions for Normal and Abnormal Uterine Bleeding, Semin Reprod Med 2011; 29(5): 383-390]. Submucosal position of the uterine fibroids, e.g. those located directly below the endometrium, seems to have an even more severe effect on uterine bleeding, which may result in anemia in affected women [Yang JH et al. Impact of submucous myoma on the severity of anemia. Fertil Steril. 2011 Apr;95(5):1769-72]. Furthermore, uterine fibroids, due to their symptoms, do severly affect the quality of life of affected women [Downes E et al. The burden of uterine fibroids in five European countries. Eur J Obstet Gynecol Reprod Biol. 2010 Sep;152(1):96-102].

Compounds of the present invention can be utilized to inhibit, block, reduce or decrease MAP4K1 activation by exogenous and/or endogenous ligands for the reduction of tumour growth and the modulation of dysregulated immune responses e.g. to block immunosuppression and increase immune cell activation and infiltration in the context of cancer and cancer immunotherapy; This method comprises administering to a mammal in need thereof, including a human, an amount of a compound of this invention, or a pharmaceutically acceptable salt, isomer, polymorph, metabolite, hydrate, solvate or ester thereof; which is effective to treat the disorder.

The present invention also provides methods of treating a variety of other disorders wherein MAP4K1 is involved such as, but not limited to, disorders with dysregulated immune responses, inflammation, vaccination for infection & cancer, viral infections, obesity and diet-induced obesity, adiposity, metabolic disorders, hepatic steatosis and uterine fibroids.
These disorders have been well characterized in humans, but also exist with a similar etiology in other mammals, and can be treated by administering pharmaceutical compositions of the present invention.

The term "treating" or "treatment" as used in the present text is used conventionally, e.g., the management or care of a subject for the purpose of combating, alleviating, reducing, relieving, improving the condition of a disease or disorder, such as liquid and solid tumours.
In accordance with a further aspect, the present invention covers compounds of general formula (I), as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular cancer or conditions with dysregulated immune responses or other disorders associated with aberrant MAP4K1 signaling.

The pharmaceutical activity of the compounds according to the invention can be explained by their activity as MAP4K1 inhibitors.

In accordance with a further aspect, the present invention covers the use of compounds of general formula (I), as described *supra,* or stereoisomers, tautomers, N-oxides, hydrates, solvates, and salts thereof, particularly pharmaceutically acceptable salts thereof, or mixtures of same, for the treatment or prophylaxis of diseases, in particular cancer or conditions with dysregulated immune responses or other disorders associated with aberrant MAP4K1 signaling, particularly liquid and solid tumours.

In accordance with a further aspect, the present invention covers the compounds of general formula (I), as described *supra,* or stereoisomers, tautomers, N-oxides, hydrates, solvates, and salts thereof, particularly pharmaceutically acceptable salts thereof, or mixtures of same, for the use of treatment or prophylaxis of diseases, in particular cancer or conditions with dysregulated immune responses or other disorders associated with aberrant MAP4K1 signaling, particularly liquid and solid tumours.

In accordance with a further aspect, the present invention covers the use of compounds of general formula (I), as described *supra,* or stereoisomers, tautomers, N-oxides, hydrates, solvates, and salts thereof, particularly pharmaceutically acceptable salts thereof, or mixtures of same, in a method of treatment or prophylaxis of diseases, in particular cancer or conditions with dysregulated immune responses or other disorders associated with aberrant MAP4K1 signaling, particularly liquid and solid tumours.

In accordance with a further aspect, the present invention covers use of a compound of general formula (I), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular cancer or conditions with dysregulated immune responses or other disorders associated with aberrant MAP4K1 signaling, particularly liquid and solid tumours.

In accordance with a further aspect, the present invention covers a method of treatment or prophylaxis of diseases, in particular cancer or conditions with dysregulated immune responses or other disorders associated with aberrant MAP4K1 signaling, particularly liquid and solid tumours, using an effective amount of a compound of general formula (I), as described *supra,* or stereoisomers, tautomers, N-oxides, hydrates, solvates, and salts thereof, particularly pharmaceutically acceptable salts thereof, or mixtures of same.

In accordance with a further aspect, the present invention covers pharmaceutical compositions, in particular a medicament, comprising a compound of general formula (I), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore covers pharmaceutical compositions, in particular medicaments, which comprise at least one compound according to the invention, conventionally together with one or more pharmaceutically suitable excipients, and to their use for the above mentioned purposes.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.
For these administration routes, it is possible for the compounds according to the invention to be administered in suitable administration forms.
For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),
- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention.
In accordance with another aspect, the present invention covers pharmaceutical combinations, in particular medicaments, comprising at least one compound of general formula (I) of the present invention and at least one or more further active ingredients, in particular for the treatment and/or prophylaxis of cancer or conditions with dysregulated immune responses or other disorders associated with aberrant MAP4K1 signalinggeneric name disorders, particularly liquid and solid tumours.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

Based upon standard laboratory techniques known to evaluate compounds useful for the treatment of cancer or conditions with dysregulated immune responses or other disorders associated with aberrant MAP4K1 signaling, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### Experimental section

NMR peak forms are stated as they appear in the spectra, possible higher order effects have not been considered. The multiplicities are stated according to the signal form which appears in the spectrum, NMR-spectroscopic effects of a higher order were not taken into consideration. Multiplicity of the NMR signals: s = singlet, d = doublet, t = triplet, q = quartet, quin = quintet, br = broad signal, m = multiplet. NMR signals: shift in [ppm]. Combinations of multiplicity could be e.g. dd = doublet from doublet.

Chemical names were generated using the ACD/Name software from ACD/Labs. In some cases generally accepted names of commercially available reagents were used in place of ACD/Name generated names. Table 1 lists the abbreviations used in this paragraph and in the Examples section as far as they are not explained within the text body. Other abbreviations have their meanings customary *per se* to the skilled person.

**Table 1: Abbreviations**

| | |
|---|---|
| AUC | Area Under Curve |
| DCM | dichloro methane |
| DMSO | dimethyl sulphoxide |
| EAE | experimental autoimmune encephalomyelitis |
| EDTA | Ethylenediaminetetraacetic acid |
| Expl. | Example |
| FCS | fetal calf serum |
| HMDS | Hexamethyldisilazane |
| LPS | lipopolysaccharide |
| mL | milliliter |
| µL | microliter |
| min. | minute(s) |
| RT | room temperature |
| sat. | saturated |
| SDS | Sodium dodecyl sulfate |
| TNFa | tumour necrosis factor alpha |
| uM | micromolar |

The various aspects of the invention described in this application are illustrated by the following examples which are not meant to limit the invention in any way.

The example testing experiments described herein serve to illustrate the present invention and the invention is not limited to the examples given.

### Experimental section - general part

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.
In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### General synthesis of compounds of general formula (I) of the present invention

The following paragraphs outline a variety of synthetic approaches suitable to prepare compounds of the general formula (I), and intermediates useful for their synthesis.
In addition to the routes described below, also other routes may be used to synthesise the target compounds, in accordance with common general knowledge of a person skilled in the art of organic synthesis. The order of transformations exemplified in the following schemes is therefore not intended to be limiting, and suitable synthesis steps from various schemes can be combined to form additional synthesis sequences. In addition, interconversion of any of the substituents, in particular R¹, R², R³ and R⁴, which are as defined in formula (I) *supra,* can be achieved before and/or after the exemplified transformations. These modifications can be, for example, the introduction of protective groups, cleavage of protective groups, reduction or oxidation of functional groups, halogenation, metallation, metal catalysed coupling reactions, exemplified by but not limited to e.g. Buchwald, Suzuki, Sonogashira and Ullmann coupling, ester saponifications, amide coupling reactions, and/or substitution or other reactions known to a person skilled in the art. These transformations include those which introduce a functionality allowing for further interconversion of substituents. Appropriate protective groups and their introduction and cleavage are well-known to a person skilled in the art (see for example T.W. Greene and P.G.M. Wuts in Protective Groups in Organic Synthesis, 4th edition, Wiley 2006). Further, it is possible that two or more successive steps may be performed without work-up being performed between said steps, *e.g.* a "one-pot" reaction, as it is well-known to a person skilled in the art.

Compounds of general formula (I) can be assembled according to Scheme 1, by reaction of amine derivatives of formula (II), in which R¹, R^{2a}, R^{2b}, and X are as defined for the compounds of general formula (I), and a second amine derivative (III), in which R³, and R⁴ are as defined for the compounds of general formula (I), by means of urea formation well known to the person skilled in the art. Said urea formation can be performed by reaction of compounds of the formula (II) with the intermediacy of a formed and possibly isolated isocyanate or isothiocyanate (IVa) using a suitable reagent such as 1,1'-carbonylbis-1H-imidazole, di- or triphosgene for Y=O, or as thiophosgene or 1,1'-thiocarbonylbis-1H-imidazole for Y=S. Compounds of general formula (I) can also be assembled by conversion of amine derivatives of formula (II) to an intermediately formed and possibly isolated carbamate or thiocarbamate (IVb) using a suitable reagent such as phenyl chloroformate or O-phenyl chlorothionoformate in which Z is H, NO₂, or perfluoro in an appropriate solvent such as tetrahydrofuran, dichloromethane, or ethylacetate in the presence of an appropriate base such as pyridine, sodium hydrogencarbonate, or triethylamine. This intermediate (IVb) is then reacted with the second amine derivative (III) in an appropriate solvent such as pyridine, or dimethylformamide. In a similar way the compounds of general formula (I) can be assembled using the amine (III) as starting material. With the former described reaction the amine (III) can react to the intermediately formed isocyanate or isothiocyanate (Va for R³ = H), if it is not commercially available, in which R⁴ and Y are as defined for the compounds of general formula (I) or the carbamate or thiocarbamate (Vb) using a suitable reagent such as phenyl chloroformate or O-phenyl chlorothionoformate in which Z is H, NO₂, or perfluoro and R³, R⁴ and Y are as defined for the compounds of general formula (I) with the second amine (II).

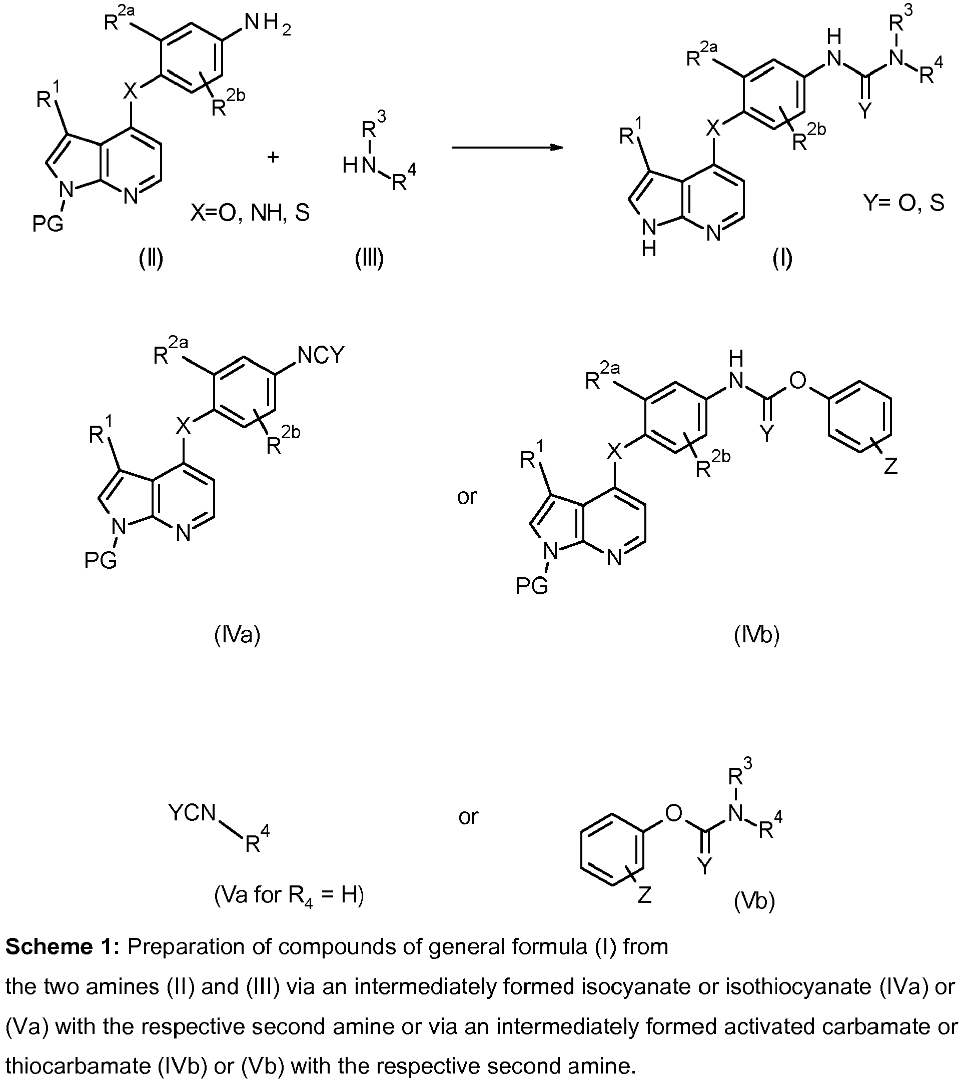

Depending on the choice of protecting group PG in formula (II), (IVa), and (IVb), which is preferentially trimethylsilylethyloxymethyl (SEM), but can be any other protecting group well known to the person skilled in the art, the deprotection can be performed using trifluoroacetic acid in the case of trimethylsilylethyloxymethyl, in an inert solvent such as dichloromethane, within a temperature range from 0°C to the boiling point of the used solvent. The deprotection in the case of trimethylsilylethyloxymethyl can be also performed using tetra-butylammonium fluoride in the presence of ethylenediamine in an inert solvent such as tetrahydrofuran within a temperature range from 0°C to the boiling point of the used solvent.

Preferred herein is the performance of said urea formation using the amine (II) and the intermediately formed and maybe isolated carbamate (IVb) and the subsequent reaction with the respective second amine (III) in DMF or using the amine (II) in a reaction with the respective cyanate or isothiocyanate (Va) in pyridine as a solvent, within a temperature range from 0°C to 100°C.

The amine intermediates of formula (II) are known to the person skilled in the art and can, if not commercially available, be prepared according to Schemes 2 and 3 shown below. The second amine derivatives of formula (III) are either commercially available in some structural variety, or they can be prepared using synthetic methods described in many textbooks such as March's Advanced Organic Chemistry: Reactions, Mechanisms, and Structure, 7th Edition.

As illustrated in Scheme 2, the amine derivatives of formula (II) are known, commercially available, or can be prepared from the commercially available heterocyle of the formula (VI), in which R^{1a} represents a hydrogen or a trifluoromethyl group. Said heterocycle of the formula (VI) can be reacted with nitric acid, in the case of leaving group (LG) = NO₂, or in a two step sequence for LG = Cl using MCPBA to form a 7-N-oxide, which then reacts in a subsequent step with methanesulfonyl chloride to give intermediate heterocycles of formula (VII).

Compounds of the formula (VII) are converted to chlorides of formula (VIII) using a two step sequence, which begins with production of a 7-N-oxide which then reacts in a subsequent step with trichloroacetyl chloride in the presence of HMDS. Said heterocycles of formula (VIII) can be transformed to the protected intermediates of the formula (IX) using an apprioate reagent such as trimetylsilylethoxymethyl chloride, triisopropylsilyl chloride or trityl chloride or other reagents known to a person skilled in the art. Preferably, trimetylsilylethoxymethyl chloride is used, in the presence of a base such as sodium hydride, triethyl amine, or ethyl diisopropyl amine in an inert solvent such as THF, DMSO or DMF.

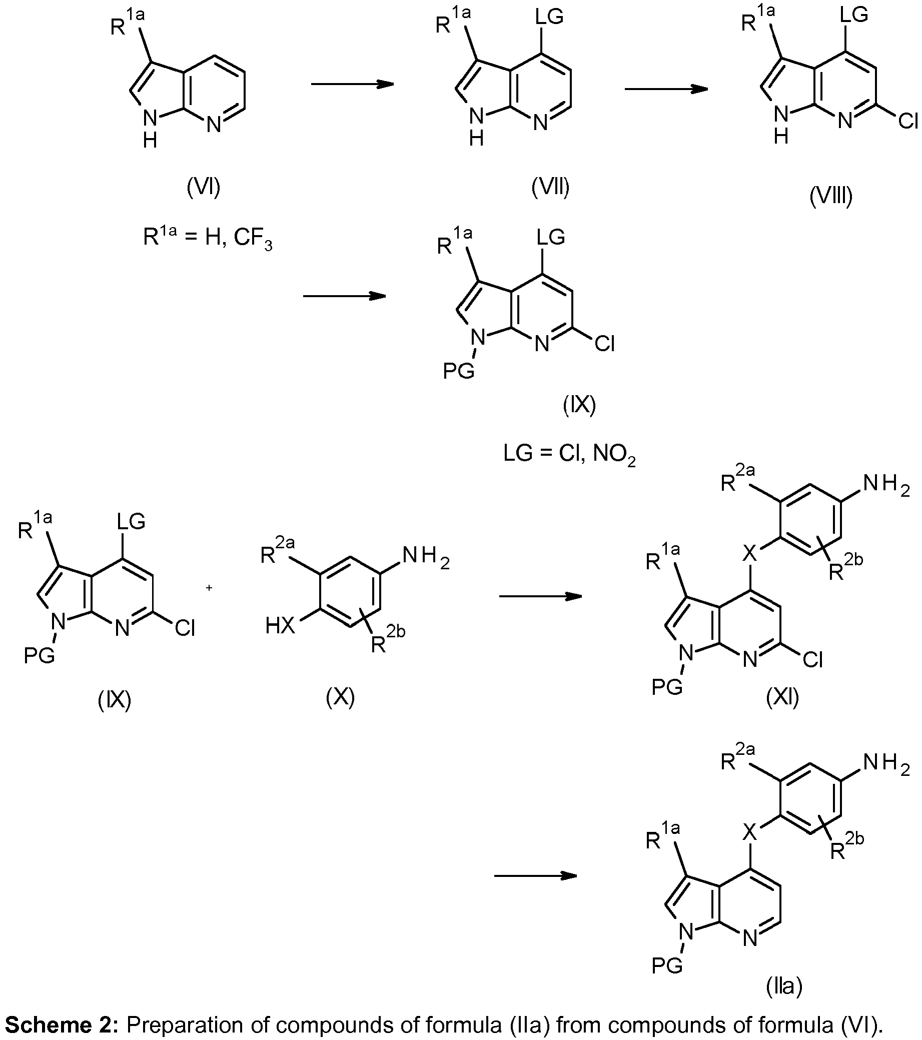

Protected heterocycles of the formula (IX) are then reacted with a compound of the formula (X) in the presence of sodium hydride or an alkali carbonate, such as sodium carbonate, potassium carbonate, or cesium carbonate, in a suitable solvent such as DMSO or DMF, as well known to the person skilled in the art, to give compounds of formula (XI). For compounds of formula (X) with X=NH it may be necessary to protect the other amino function in an intermediate fashion.

Dechlorination is preferentially performed using a hydrogen atmosphere and palladium on carbon as catalyst in an inert solvent such as ethanol, ethyl acetate or dichloromethane at 20-50°C as described in Org. Process Res. Dev. 2010, page 168-173, to give a subset of the amines of the formula (II) named (IIa) in which R^{1a} are as defined for compounds of gerenal formula (VI).

All other amines of the subset (IIb) from the general formula (II) can be assembled from the amine (IIa) with R^{1a}=H according to Scheme 3.

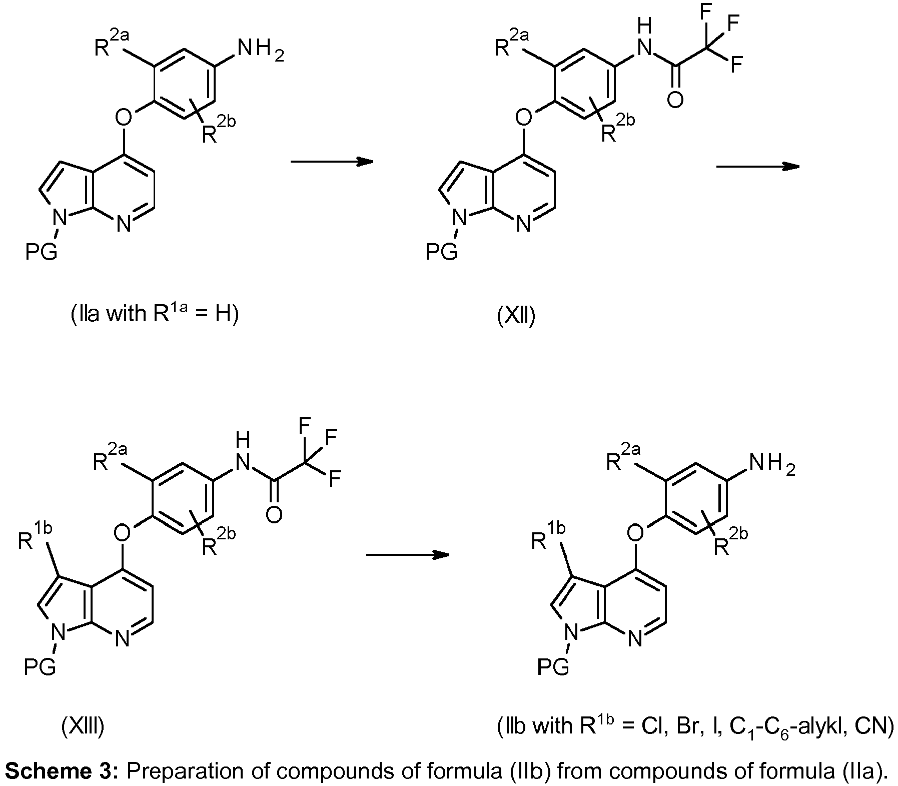

Protection of the free amine in (IIa) with R^{1a}=H using trifluoroacetic anhydride in an inert solvent such as dichloromethane in the presence of an tertiary amine such as triethyl amine or ethyl diisopropyl amine, yields the amides of the general formula (XII) in which R^{2a} and R^{2b} are as defined for the compounds of general formula (I). Said amides of formula (XII) can be converted into halogen substituted compounds of the general formula (XIII) with R^{1b} = Cl, Br, or I using the corresponding N-halo-succinimide in an inert solvent such as dichloro methane or tetrachloro methane. In the case of compounds of the general formula (XIII) with R^{1b} = CN, and C₁-C₆-alkyl, a subsequent reaction of a said halogen compound of the general formula (XII) with R^{1b} = Br or I is used as starting material. For formation of the corresponding nitrile, a reaction using cuprous cyanide in an inert solvent such as dimethyl formamide or dimethyl acetamide at elevated temperatures, for example between 90 - 120°C is required. Furthermore, a palladium-catalyzed method known to the person skilled in the art, for example, with zinc cyanide or potassium ferrocyanide as described in Chem. Soc. Rev., 2011, 40, 5049-5067 can be utilized. For the preparation of compounds of the general formula (XIII) with R^{1b} = C₁-C₆-alkyl a Suzuki reaction known to the person skilled in the art is used as described, for example in Metal-Catalyzed Cross-Coupling Reactions, Second Edition (Editors: Armin de Meijere, François Diederich, Wiley-VCH) or in Catal. Lett. (2016) page 820-840. After saponification of compounds of the general formula (XII) using lithium, sodium or potassium hydroxide, the compounds of the general formula (IIb) are obtained. Taken together, formulae (IIa) and (IIb) constitute formula (II).

A complementary approach to compounds of formula (II) is described in Scheme 4.

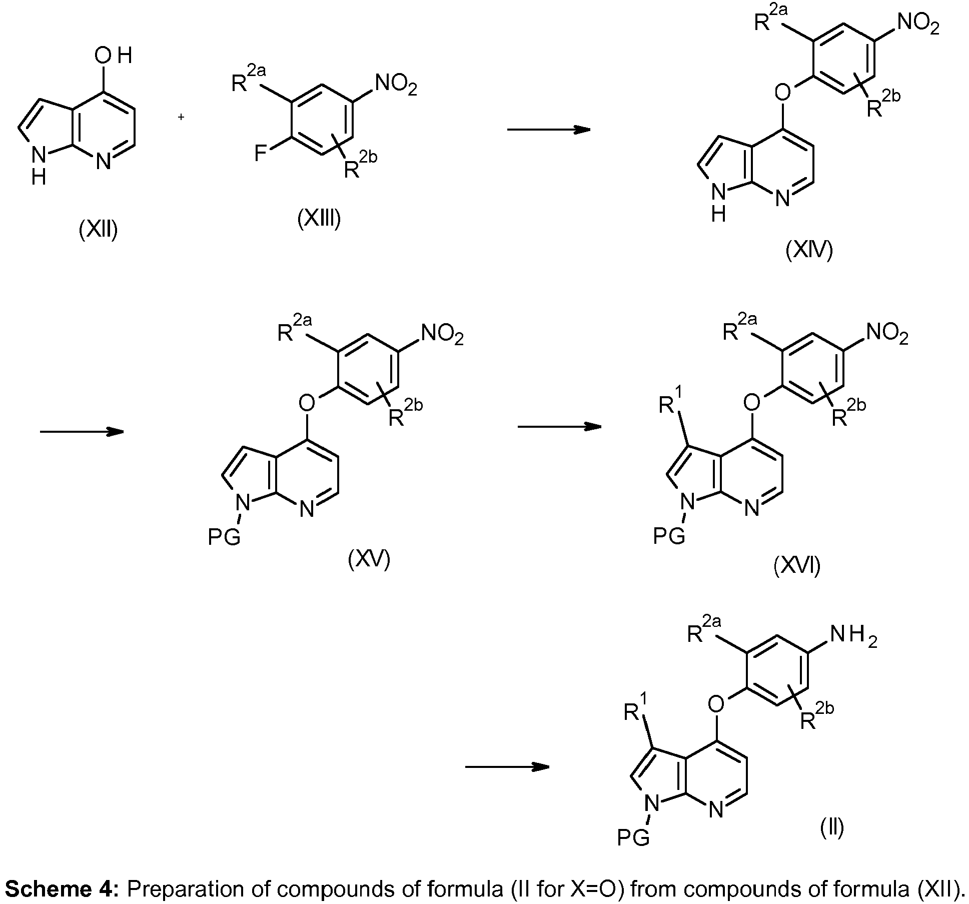

The respective fluoro-nitrobenzene derivatives of formula (XIII) are reacted with 4-hydroxy-7-azaindole (XII) (e.g. US2007/238726), in a suitable solvent system, for example dimethylsulfoxide, in the presence of a base, for example potassium carbonate or cesium carbonate, in a temperature range from room temperature to the boiling point of the respective solvent. Preferable the reaction is carried out at room temperature to furnish intermediates of general formula (XIV).

Intemediates of general formula (XIV), are then protected with an appropriate protecting group, for example using an appropriate reagent such as trimetylsilylethoxymethyl chloride, triisopropylsilyl chloride or trityl chloride or other reagents known to a person skilled in the art. Preferably, trimetylsilylethoxymethyl chloride is used, in the presence of a base such as sodium hydride, triethylamine, or ethyldiisopropylamine in an inert solvent such as tetrahydrofuran, dimethylsulfoxide or dimethylformamide to afford compounds of formula (XV).

Compounds of formula (XV) can be converted into halogen substituted compounds of the general formula (XVI) with R¹ = Cl, Br, or I using the corresponding N-halo-succinimide in an inert solvent such as dichloromethane, dimethylformamide, or tetrachloromethane, in a temperature range from room temperature to the boiling point of the respective solvent. Preferable the reaction is carried out at room temperature to furnish intermediates of general formula (XVI).

Compounds of general formula (XVI), where R¹ = I, can be reacted with trifluoromethylation reagents, for example diphenyl(trifluoromethyl)sulfonium trifluoromethanesulfonate as described in Angew. Chem. Int. Ed. 2011, 50, pg. 1896-1900, or any other reagent known to one skilled in the art, to afford compounds of general formula (XVI) where R¹ = CF₃. Preferably, diphenyl(trifluoromethyl)sulfonium trifluoromethanesulfonate is used, in the presence of additives, such as copper(0), in a solvent such as dimethylformamide, in a temperature range from room temperature to the boiling point of the respective solvent. Ideally, the reaction is carried out at 60°C to furnish intermediates of general formula (XVI) where R¹ = CF₃.

In the case of compounds of the general formula (XVI) with R¹ = CN, and C₁-C₆-alkyl, a subsequent reaction of a said halogen compounds of the general formula (XVI) with R¹ = Br or I is required. For formation of the corresponding nitrile, a reaction using cuprous cyanide in an inert solvent such as dimethyl formamide or dimethyl acetamide at elevated temperatures, for example between 90 - 120°C is required. Furthermore, a palladium-catalyzed method known to the person skilled in the art, for example, with zinc cyanide or potassium ferrocyanide as described in Chem. Soc. Rev., 2011, 40, 5049-5067 can be utilized. For the preparation of compounds of the general formula (XVI) with R¹ = C₁-C₆-alkyl a Suzuki reaction known to the person skilled in the art is used as described, for example in Metal-Catalyzed Cross-Coupling Reactions, Second Edition (Editors: Armin de Meijere, François Diederich, Wiley-VCH) or in Catal. Lett. (2016) page 820-840.

Reduction of the nitro functionalitiy contained within compounds of formula (XVI) affords amines of formula (II). Preferentially, the reaction is performed with the addition of a reducing agent, for example Iron(0), with additives such as ammonium chloride, in an appropriate mixture of solvents, for example a mixture of water, tetrahydrofuran, and methanol, in a temperature range from room temperature to the boiling point of the respective solvent. Ideally the reaction is performed at 80 °C to furnish amine intermediates of general formula (II). The reaction can be carried out using alternative reducing agents to those skilled in the art, for example tin(II)chloride, in an appropriate solvent, such as methanol, in a temperature range from room temperature to the boiling point of the respective solvent. Ideally the reaction is performed at room temperature to furnish amine intermediates of general formula (II).

Method 1: (prep. HPLC) System: Labomatic, Pump: HD-5000, Fraction Collector: LABOCOL Vario-4000, UV-Detector: Knauer UVD 2.1S; Column: Chromatorex RP C18 10µm 125x30 mm; Solvent: A = water + 0.1% Vol. ammonia (99%), B = Acetonitril; Flow: 150 mL/min; temperature: room temperature

### Intermediate 1

### 4-{[6-chloro-3-(trifluoromethyl)-1-{[2-(trimethylsilyl)ethoxy]methyl}-1 H-pyrrolo[2,3-b]pyridin-4-yl]oxy}-3-(trifluoromethyl)aniline

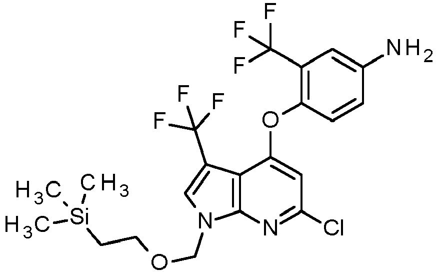

A solution of 6-chloro-4-nitro-3-(trifluoromethyl)-1-{[2-(trimethylsilyl)ethoxy]methyl}-1H-pyrrolo[2,3-b]pyridine (500 mg, 1.26 mmol, see Synthesis 2007, page 251-258, Org. Process Res. Dev. 2010, page 168-173), 4-amino-2-(trifluoromethyl)phenol (246 mg, 1.39 mmol) and potassium carbonate (524 mg, 3.79 mmol) in DMSO (5.0 mL) was stirred at 120°C for 3 hours. After cooling to room temperature the reaction mixture was diluted with ethyl actate (200 mL). This organic phase was washed two times with water (30 mL) and once with brine (20 mL), then dried over sodium sulfate and after filtration evaporated to dryness. The resulting residue was purified via a Biotage chromatography system (28g snap KP-NH column, hexane / 0 - 70% ethyl acetate) to obtain 550 mg (93 % purity, 77 % yield) of the desired title compound. ¹H-NMR (400 MHz, DMSO-d6) δ [ppm]: -0.11 - -0.07 (m, 9H), 0.79 - 0.88 (m, 2H), 3.54 - 3.61 (m, 2H), 5.61 (s, 2H), 5.73 (s, 2H), 6.30 - 6.32 (m, 1H), 6.90 (dd, 1H), 6.99 (d, 1H), 7.17 (d, 1H), 8.35 (s, 1H).

### Intermediate 2

### 3-(trifluoromethyl)-4-{[3-(trifluoromethyl)-1-{[2-(trimethylsilyl)ethoxy]methyl}-1H-pyrrolo[2,3-b]pyridin-4-yl]oxy}aniline

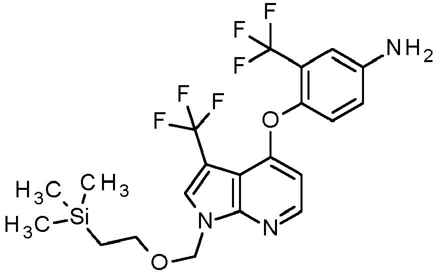

To a solution of 4-{[6-chloro-3-(trifluoromethyl)-1-{[2-(trimethylsilyl)ethoxy]methyl}-1H-pyrrolo[2,3-b]pyridin-4-yl]oxy}-3-(trifluoromethyl)aniline (545 mg, 1.04 mmol, intermediate 1), and triethylamine (170 µL, 1.2 mmol) in ethanol (36 mL) was given 10% Pd on carbon (54.5 mg). This mixture was stirred in an hydrogen atmosphere for 7 hours at room temperature. Then the mixture was filtered through Celite and the Celite was washed with ethyl acetate. The organic phase was evaporated to dryness and the resulting residue was purified via a Biotage chromatography system (28g snap KP-NH column, hexane / 10 - 70% ethyl acetate) to obtain 495 mg (83 % purity, 80 % yield) of the desired title compound.
¹H-NMR (400 MHz, DMSO-d6) δ [ppm]: -0.11 - -0.08 (m, 9H), 0.79 - 0.86 (m, 2H), 3.54 - 3.60 (m, 2H), 5.65 (s, 2H), 5.67 (s, 2H), 6.37 (d, 1H), 6.88 (dd, 1H), 6.97 (d, 1H), 7.08 (d, 1H), 8.23 (d, 1H), 8.28 (s, 1H).

### Intermediate 3

### phenyl [3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate

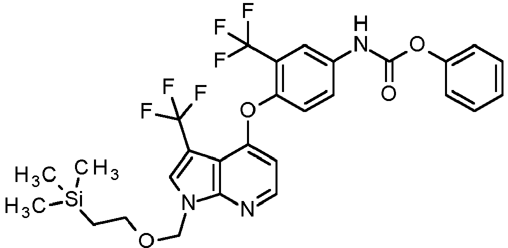

To a solution of 3-(trifluoromethyl)-4-{[3-(trifluoromethyl)-1-{[2-(trimethylsilyl)ethoxy]methyl}-1H-pyrrolo[2,3-b]pyridin-4-yl]oxy}aniline (490 mg, 997 µmol), intermediate 2) in pyridine (460 µL) and THF (6.9 mL) was slowly added at 0°C phenyl carbonochloridate (140 µL, 1.1 mmol). After stirring this mixture 5 minutes at 0°C and then 30 minutes at room temperature ethyl acetate (150 mL) was added. This organic phase was washed with 1N hydrochloric acid (30 mL), water, concentrated aqueous sodium hydrogencarbonate, brine, dried over sodium sulfate, filtered and evaporated to dryness. The resulting residue was purified via a Biotage chromatography system (10g snap KP-Sil column, hexane / 10 - 60% ethyl acetate) to obtain 479 mg (89 % purity, 69 % yield) of the desired title compound.
¹H-NMR (400 MHz, DMSO-d6) δ [ppm]: -0.10 - -0.07 (m, 9H), 0.78 - 0.89 (m, 2H), 3.55 - 3.62 (m, 2H), 5.69 (s, 2H), 6.51 (d, 1H), 6.71 - 6.79 (m, 1H), 7.24 - 7.31 (m, 2H), 7.42 - 7.49 (m, 3H), 7.83 (dd, 1H), 8.08 (d, 1H), 8.29 (d, 1H), 8.35 (s, 1H), 10.71 (br s, 1H).

### Intermediate 4

### 1-[2-(morpholin-4-yl)ethyl]-3-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea

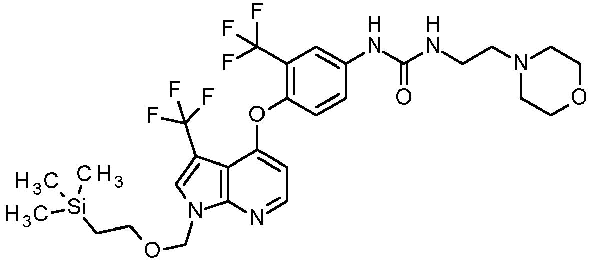

To a solution of phenyl [3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate (150 mg, 245 µmol, intermediate 3) in DMF (1.2 mL) was added 2-(morpholin-4-yl)ethanamine (42 µL, 320 µmol) and this mixture was stirred at 60°C for 2 hours. After cooling to room temperature ethyl acetate (80 mL) was added and the organic phase was extracted two times with water, once with brine, dried over sodium sulfate, filtered and evaporated to dryness. The resulting residue was purified via a Biotage chromatography system (11g snap KP-NH column, hexane / 50 - 100% ethyl acetate, then ethyl acetate / 0 - 30% methanol) to obtain 144 mg (100 % purity, 91 % yield) of the desired title compound.
¹H-NMR (400 MHz, DMSO-d6) δ [ppm]: -0.11 - -0.07 (m, 9H), 0.80 - 0.87 (m, 2H), 2.36 - 2.44 (m, 6H), 3.23 (q, 2H), 3.55 - 3.63 (m, 6H), 5.68 (s, 2H), 6.23 (t, 1H), 6.45 (d, 1H), 7.31 (d, 1H), 7.62 (dd, 1H), 8.08 (d, 1H), 8.26 (d, 1H), 8.33 (s, 1H), 9.13 (s, 1H).

### Intermediate 5

### 1-[3-(morpholin-4-yl)propyl]-3-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea

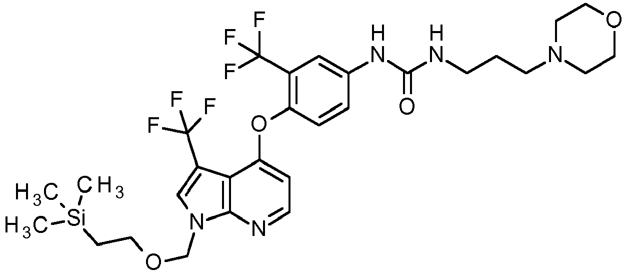

In analogy to intermediate 4), phenyl [3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate (150 mg, 245 µmol), intermediate 3) and 3-(morpholin-4-yl)propan-1-amine (47 µL, 320 µmol) were reacted in DMF (1.2 mL) and we obtained after purification using a Biotage chromatography system 136 mg (91 % purity, 76 % yield) of the desired title compound.
¹H-NMR (400 MHz, DMSO-d6) δ [ppm]: -0.11 - -0.07 (m, 9H), 0.80 - 0.87 (m, 2H), 1.61 (quin, 2H), 2.27 - 2.38 (m, 6H), 3.13 (q, 2H), 3.55 - 3.61 (m, 6H), 5.68 (s, 2H), 6.33 (t, 1H), 6.45 (d, 1H), 7.31 (d, 1H), 7.63 (dd, 1H), 8.07 (d, 1H), 8.27 (d, 1H), 8.33 (s, 1H), 8.94 (s, 1H).

### Intermediate 6

### 1-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea

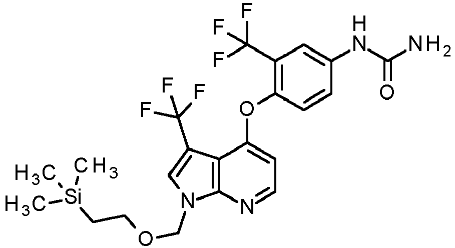

In analogy to intermediate 4), phenyl [3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate (150 mg, 245 µmol), intermediate 3) and ammonia (6.0 µL, 7.0 M, 42 µmol) were reacted in DMF (250 µL) and we obtained after purification using a Biotage chromatography system 11.2 mg (50 % yield) of the desired title compound.
¹H-NMR (400 MHz, DMSO-d6) δ [ppm]: -0.14 - -0.03 (m, 9H), 0.79 - 0.89 (m, 2H), 3.55 - 3.61 (m, 2H), 5.68 (s, 2H), 6.07 (s, 2H), 6.45 (d, 1H), 7.31 (d, 1H), 7.66 (dd, 1H), 8.07 (d, 1H), 8.26 (d, 1H), 8.33 (s, 1H), 9.05 (s, 1H).

### Intermediate 7

### 4-{[6-chloro-3-(trifluoromethyl)-1-{[2-(trimethylsilyl)ethoxy]methyl}-1H-pyrrolo[2,3-b]pyridin-4-yl]oxy}-3-fluoro-5-(trifluoromethyl)aniline

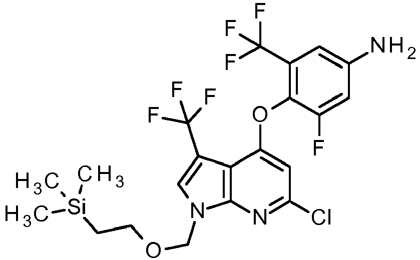

In analogy to intermediate 1), we obtained from 6-chloro-4-nitro-3-(trifluoromethyl)-1-{[2-(trimethylsilyl)ethoxy]methyl}-1H-pyrrolo[2,3-b]pyridine (400 mg, 1.01 mmol), 4-amino-2-fluoro-6-(trifluoromethyl)phenol (217 mg, 1.11 mmol) and potassium carbonate (419 mg, 3.03 mmol) in DMSO (4.0 mL) after purification using a Biotage chromatography system 374 mg (81 % purity, 55 % yield) of the desired title compound.
¹H-NMR (400 MHz, DMSO-d6) δ [ppm]: -0.14 - -0.04 (m, 9H), 0.79 - 0.87 (m, 2H), 3.54 - 3.62 (m, 2H), 5.62 (s, 2H), 6.07 (s, 2H), 6.48 (d, 1H), 6.78 - 6.85 (m, 2H), 8.38 (s, 1H).

### Intermediate 8

### 3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}aniline

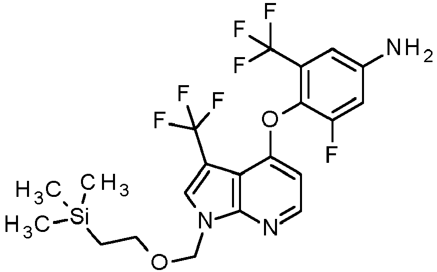

In analogy to intermediate 2), we obtained from 4-{[6-chloro-3-(trifluoromethyl)-1-{[2-(trimethylsilyl)ethoxy]methyl}-1H-pyrrolo[2,3-b]pyridin-4-yl]oxy}-3-fluoro-5-(trifluoromethyl)aniline (342 mg, 629 µmol, intermediate 7) in an hydrogenation reaction using palladium on carbon (34.2 mg, 10 % purity, 32.1 µmol) and triethylamine (110 µL, 750 µmol) in ethanol (22 mL) after purification using a Biotage chromatography system 191 mg (71 % purity, 43 % yield) of the desired title compound.
¹H-NMR (400 MHz, DMSO-d6) δ [ppm]: -0.12 - -0.04 (m, 9H), 0.78 - 0.85 (m, 2H), 3.55 - 3.61 (m, 2H), 5.67 (s, 2H), 6.00 (s, 2H), 6.45 (dd, 1H), 6.76 - 6.85 (m, 2H), 8.26 (d, 1H), 8.31 (s, 1H).

### Intermediate 9

### phenyl [3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate

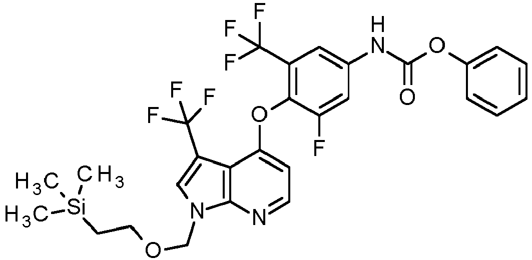

In analogy to intermediate 3), we obtained from 3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}aniline (188 mg, 369 µmol, intermediate 8) and phenyl carbonochloridate (51 µL, 410 µmol) in a mixture of pyridine (170 µL, 2.2 mmol) and THF (2.6 mL) after purification using a Biotage chromatography system 219 mg (90 % purity, 85 % yield) of the desired title compound.
¹H-NMR (400 MHz, DMSO-d6) δ [ppm]: -0.13 - -0.05 (m, 9H), 0.78 - 0.86 (m, 2H), 3.55 - 3.61 (m, 2H), 5.69 (s, 2H), 6.56 (dd, 1H), 6.72 - 6.76 (m, 1H), 7.26 - 7.33 (m, 2H), 7.43 - 7.50 (m, 2H), 7.84 - 7.93 (m, 2H), 8.28 (d, 1H), 8.37 (d, 1H), 10.93 (br s, 1H).

### Intermediate 10

### 1-[3-fluoro-5-(trifluoromethyl)-4-{[3-(trifluoromethyl)-1-{[2-(trimethylsilyl)ethoxy]methyl}-1 H-pyrrolo[2,3-b]pyridin-4-yl]oxy}phenyl]-3-[3-(morpholin-4-yl)propyl]urea

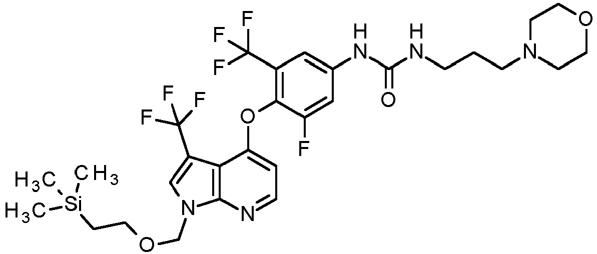

In analogy to intermediate 4), we obtained from phenyl [3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate (108 mg, 172 µmol), intermediate 9) and 3-(morpholin-4-yl)propan-1-amine (25 µL, 170 µmol) in DMF (850 µL) after purification using a Biotage chromatography system 97.4 mg (92 % purity, 76 % yield) of the desired title compound.
¹H-NMR (400 MHz, DMSO-d6) δ [ppm]: -0.12 - -0.05 (m, 9H), 0.78 - 0.85 (m, 2H), 1.61 (quin, 2H), 2.27 - 2.38 (m, 6H), 3.14 (q, 2H), 3.55 - 3.61 (m, 6H), 5.68 (s, 2H), 6.47 (t, 1H), 6.51 (dd, 1H), 7.75 (s, 1H), 7.85 (dd, 1H), 8.27 (d, 1H), 8.35 (s, 1H), 9.16 (s, 1H).

### Intermediate 11

### 1-[3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]-3-[2-(morpholin-4-yl)ethyl]urea

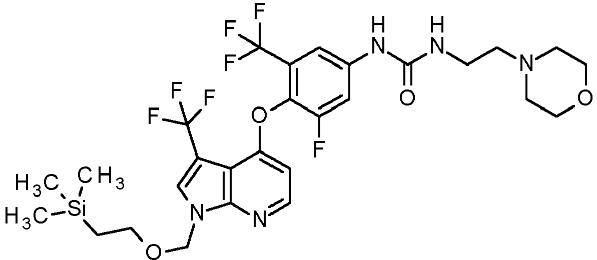

In analogy to intermediate 4), we obtained from phenyl [3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]carbamate (108 mg, 172 µmol), intermediate 9) and 2-(morpholin-4-yl)ethanamine (23 µL, 170 µmol) in DMF (850 µL) after purification using a Biotage chromatography system 89.3 mg (92 % purity, 72 % yield) of the desired title compound. ¹H-NMR (400 MHz, DMSO-d6) δ [ppm]: -0.12 - -0.05 (m, 9H), 0.79 - 0.85 (m, 2H), 2.37 - 2.44 (m, 6H), 3.24 (q, 2H), 3.55 - 3.63 (m, 6H), 5.68 (s, 2H), 6.36 (t, 1H), 6.51 (dd, 1H), 7.74 (s, 1H), 7.83 (dd, 1H), 8.27 (d, 1H), 8.35 (s, 1H), 9.35 (s, 1H).

### Intermediate 12

### 4-[4-nitro-2-(trifluoromethyl)phenoxy]-1H-pyrrolo[2,3-b]pyridine

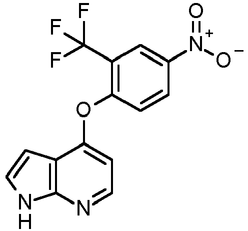

A solution of 1-fluoro-4-nitro-2-(trifluoromethyl)benzene (5.3 mL, 38 mmol) and 1*H*-pyrrolo[2,3-b]pyridin-4-ol (CAS No. [74420-02-3]; 4.67 g, 34.8 mmol) in DMSO (110 mL) was treated with potassium carbonate (19.2 g, 139 mmol) and stirred at room temperature for 1 hour. The reaction mixture was diluted with ethyl acetate (200 mL) and washed with two times with 20 mL water and brine (20 mL), dried with sodium sulfate and concentrated in vacuo. The resulting residue was purified via a Biotage chromatography system (100g snap KP-Sil column, hexane / 20 - 100% ethyl acetate) to obtain 4.62 g (96 % purity, 39 % yield) of the desired title compound.
¹H-NMR (400 MHz, DMSO-d6) δ [ppm]: 6.11 (d, 1H), 6.92 (d, 1H), 7.25 (d, 1H), 7.48 (d, 1H), 8.28 (d, 1H), 8.46 (dd, 1H), 8.58 (d, 1H), 12.05 (br s, 1H).

### Intermediate 13

### 4-[4-nitro-2-(trifluoromethyl)phenoxy]-1-{[2-(trimethylsilyl)ethoxy]methyl}-1H-pyrrolo[2,3-b]pyridine

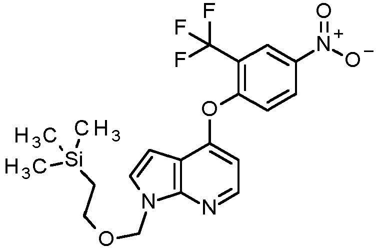

An ice-cooled solution of 4-[4-nitro-2-(trifluoromethyl)phenoxy]-1H-pyrrolo[2,3-b]pyridine (4.62 g, 14.3 mmol, intermediate 12) in acetonitrile (90 mL) was treated with *N*,*N*-diisopropyl ethylamine (6.2 mL, 36 mmol) and [2-(chloromethoxy)ethyl](trimethyl)silane (CAS No. [76513-69-4]; 3.5 mL, 20.0 mmol), warmed to rt and stirred overnight. The reaction mixture was diluted with ethyl acetate (200 mL)) and washed with water (30 mL) and brine (20 mL), dried with sodium sulfate and concentrated in vacuo.

The resulting residue was purified via a Biotage chromatography system (50g snap KP-Sil column, hexane / 0 - 80% ethyl acetate) to obtain 6.5 g (100 % purity, 99 % yield) of the desired title compound.
¹H-NMR (400 MHz, DMSO-d6) δ [ppm]: -0.12 - -0.06 (m, 9H), 0.78 - 0.87 (m, 2H), 3.51 - 3.57 (m, 2H), 5.65 (s, 2H), 6.23 (d, 1H), 6.99 (d, 1H), 7.30 (d, 1H), 7.67 (d, 1H), 8.35 (d, 1H), 8.48 (dd, 1H), 8.59 (d, 1H).

### Intermediate 14

### 3-chloro-4-[4-nitro-2-(trifluoromethyl)phenoxy]-1-{[2-(trimethylsilyl)ethoxy]methyl}-1H-pyrrolo[2,3-b]pyridine

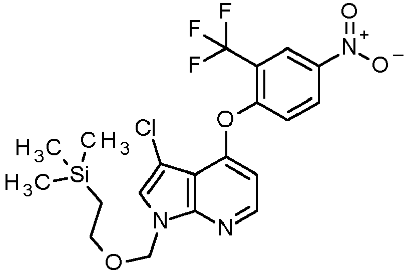

A solution of 4-[4-nitro-2-(trifluoromethyl)phenoxy]-1-{[2-(trimethylsilyl)ethoxy]methyl}-1H-pyrrolo[2,3-b]pyridine (6.52 g, 14.4 mmol, intermediate 13) in acetonitrile (270 mL) was treated with 1-chloropyrrolidine-2,5-dione (2.02 g, 15.1 mmol) and stirred at 60°C overnight. After cooling to rt the reaction mixture was concentrated in vacuo. The resulting residue was purified via a Biotage chromatography system (55g snap KP-NH column, hexane / 30 - 100% ethyl acetate) to obtain 7.26 g (91 % purity, 94 % yield) of the desired title compound.
¹H-NMR (400 MHz, DMSO-d6) δ [ppm]: -0.09 - -0.06 (m, 9H), 0.81 - 0.88 (m, 2H), 3.53 - 3.59 (m, 2H), 5.65 (s, 2H), 7.01 (d, 1H), 7.25 (d, 1H), 7.95 (s, 1H), 8.41 (d, 1H), 8.48 (dd, 1H), 8.59 (d, 1H).

### Intermediate 15

### 4-[(3-chloro-1-{[2-(trimethylsilyl)ethoxy]methyl}-1H-pyrrolo[2,3-b]pyridin-4-yl)oxy]-3-(trifluoromethyl)aniline

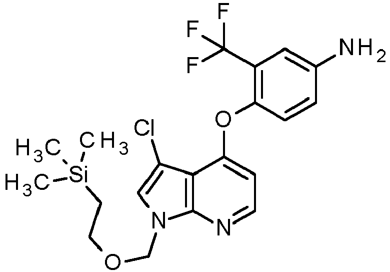

To a solution of 3-chloro-4-[4-nitro-2-(trifluoromethyl)phenoxy]-1-{[2-(trimethylsilyl)ethoxy]methyl}-1H-pyrrolo[2,3-b]pyridine (7.26 g, 14.9 mmol, intermediate 14) in a mixture of THF (74 mL) and methanol (74 mL) was added iron powder (4.15 g, 74.4 mmol), ammonium chloride (3.98 g, 74.4 mmol) and water (150 mL). This mixture was stirred for 3 hours at 80°C. After cooling to rt the reaction mixture was dilutet with ethyl acetate (200 mL) and washed with water (30 mL) and brine (20 mL), dried with sodium sulfate and concentrated in vacuo. The resulting residue was purified via a Biotage chromatography system (100g snap KP-NH column, hexane / 0 - 80% ethyl acetate) to obtain 5.85 g (94 % purity, 81 % yield) of the desired title compound.
¹H-NMR (400 MHz, DMSO-d6) δ [ppm]: -0.12 - -0.07 (m, 9H), 0.79 - 0.85 (m, 2H), 3.50 - 3.56 (m, 2H), 5.58 (s, 2H), 5.63 (s, 2H), 6.28 (d, 1H), 6.88 (dd, 1H), 6.97 (d, 1H), 7.12 (d, 1H), 7.76 (s, 1H), 8.13 (d, 1H).

### Intermediate 16

### phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate

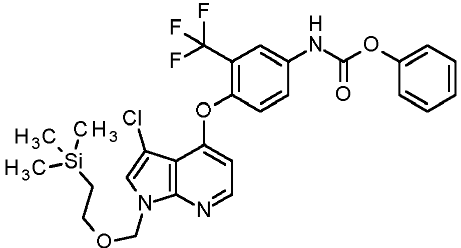

In analogy to intermediate 3), we obtained from 4-[(3-chloro-1-{[2-(trimethylsilyl)ethoxy]methyl}-1H-pyrrolo[2,3-b]pyridin-4-yl)oxy]-3-(trifluoromethyl)aniline (1.95 g, 4.26 mmol, intermediate 15) and phenyl carbonochloridate (590 µL, 4.7 mmol) in a mixture of pyridine (2.3 mL, 28 mmol) and THF (30 mL) after purification using a Biotage chromatography system 2.92 g of the crude title compound.
¹H-NMR (400 MHz, DMSO-d6) δ [ppm]: -0.11 - -0.06 (m, 9H), 0.79 - 0.86 (m, 2H), 3.50 - 3.57 (m, 2H), 5.60 (s, 2H), 6.45 (d, 1H), 7.24 - 7.31 (m, 3H), 7.40 - 7.48 (m, 3H), 7.79 - 7.84 (m, 2H), 8.07 (d, 1H), 8.20 (d, 1H), 10.68 (br s, 1H).

### Intermediate 17

### 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-[3-(morpholin-4-yl)propyl]urea

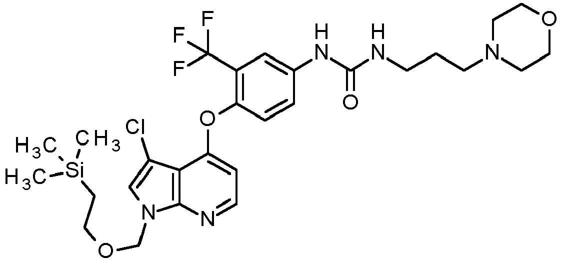

In analogy to intermediate 4), we obtained from phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate (100 mg, intermediate 16) and 3-(morpholin-4-yl)propan-1-amine (25 µL, 170 µmol) in DMF (900 µL) after purification using a Biotage chromatography system 90.5 mg (97 % purity, 81 % yield) of the desired title compound.
¹H-NMR (400 MHz, DMSO-d6) δ [ppm]: -0.11 - -0.07 (m, 9H), 0.79 - 0.86 (m, 2H), 1.60 (quin, 2H), 2.27 - 2.38 (m, 6H), 3.13 (q, 2H), 3.50 - 3.60 (m, 6H), 5.60 (s, 2H), 6.32 (t, 1H), 6.38 (d, 1H), 7.30 (d, 1H), 7.62 (dd, 1H), 7.80 (s, 1H), 8.07 (d, 1H), 8.17 (d, 1H), 8.92 (s, 1H).

### Intermediate 18

### 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-[2-(morpholin-4-yl)ethyl]urea

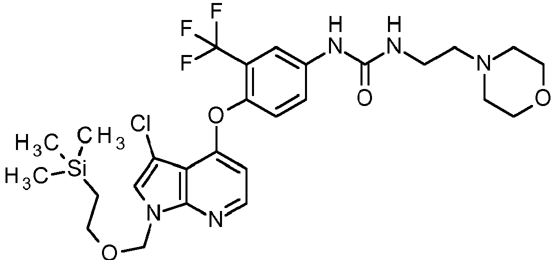

In analogy to intermediate 4), we obtained from phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate (100 mg, 173 µmol, intermediate 16) and 2-(morpholin-4-yl)ethanamine (23 µL, 170 µmol) in DMF (900 µL) after purification using a Biotage chromatography system 88.4 mg (97 % purity, 81 % yield) of the desired title compound. ¹H-NMR (400 MHz, DMSO-d6) δ [ppm]: -0.11 - -0.07 (m, 9H), 0.79 - 0.86 (m, 2H), 2.36 - 2.44 (m, 6H), 3.23 (q, 2H), 3.51 - 3.63 (m, 6H), 5.60 (s, 2H), 6.22 (t, 1H), 6.38 (d, 1H), 7.30 (d, 1H), 7.60 (dd, 1H), 7.80 (s, 1H), 8.08 (d, 1H), 8.17 (d, 1H), 9.11 (s, 1H).

### Intermediate 19

### 1-{4-[(3-chloro-1-{[2-(trimethylsilyl)ethoxy]methyl}-1H-pyrrolo[2,3-b]pyridin-4-yl)oxy]-3-(trifluoromethyl)phenyl}urea

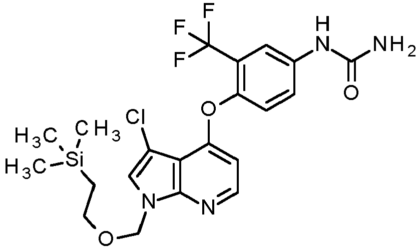

In analogy to intermediate 4), we obtained from phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate (100 mg, 173 µmol, intermediate 16) and ammonia (25 µL, 7.0 M, 170 µmol) in DMF (900 µL) after purification using a Biotage chromatography system 83.1 mg (97 % purity, 93 % yield) of the desired title compound.
¹H-NMR (400 MHz, DMSO-d6) δ [ppm]: -0.11 - -0.07 (m, 9H), 0.79 - 0.86 (m, 2H), 3.50 - 3.56 (m, 2H), 5.60 (s, 2H), 6.05 (s, 2H), 6.38 (d, 1H), 7.30 (d, 1H), 7.63 (dd, 1H), 7.80 (s, 1H), 8.07 (d, 1H), 8.17 (d, 1H), 9.00 (s, 1H).

### Intermediate 20

### 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-pyridazin-3-ylurea

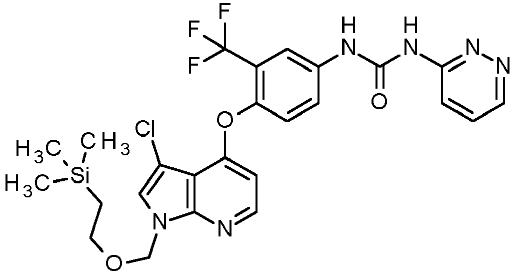

In analogy to intermediate 4), we obtained from phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate (100 mg, 173 µmol, intermediate 16) and pyridazin-3-amine (16.5 mg, 173 µmol) in DMF (900 µL) after purification using a Biotage chromatography system 57.5 mg (69 % purity, 40 % yield) of the desired title compound.
¹H-NMR (400 MHz, DMSO-d6) δ [ppm]: -0.12 - -0.05 (m, 9H), 0.79 - 0.87 (m, 2H), 3.48 - 3.59 (m, 2H), 5.61 (s, 2H), 6.47 (d, 1H), 7.36 - 7.42 (m, 1H), 7.65 - 7.76 (m, 2H), 7.81 - 7.85 (m, 1H), 8.05 (dd, 1H), 8.14 - 8.18 (m, 1H), 8.21 (d, 1H), 8.91 (dd, 1H), 9.90 (s, 1H), 10.11 (s, 1H).

### Intermediate 21

### 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-(2-methylpyrimidin-5-yl)urea

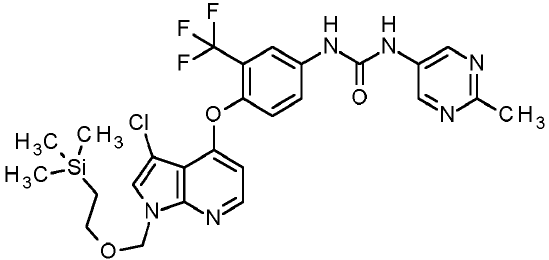

In analogy to intermediate 4), we obtained from phenyl {4-[(3-chloro-1-{[2-(trimethylsilyl)ethoxy]methyl}-1H-pyrrolo[2,3-b]pyridin-4-yl)oxy]-3-(trifluoromethyl)phenyl}carbamate (100 mg, 173 µmol, intermediate 16) and 2-methylpyrimidin-5-amine (18.9 mg, 173 µmol) in DMF (900 µL) after purification using a Biotage chromatography system 70.8 mg (92 % purity, 63 % yield) of the desired title compound. ¹H-NMR (400 MHz, DMSO-d6) δ [ppm]: -0.11 - -0.06 (m, 9H), 0.79 - 0.87 (m, 2H), 2.56 (s, 3H), 3.51 - 3.57 (m, 2H), 5.60 (s, 2H), 6.44 (d, 1H), 7.37 (d, 1H), 7.73 (dd, 1H), 7.82 (s, 1H), 8.10 (d, 1H), 8.20 (d, 1H), 8.81 (s, 2H), 9.07 (s, 1H), 9.42 (s, 1H).

### Intermediate 22

### 4-{[6-chloro-3-(trifluoromethyl)-1-{[2-(trimethylsilyl)ethoxy]methyl}-1 H-pyrrolo[2,3-b]pyridin-4-yl]oxy}-3-methoxyaniline

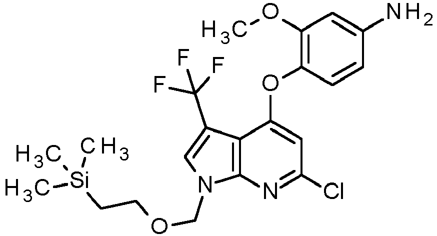

In analogy to intermediate 1), we obtained from 6-chloro-4-nitro-3-(trifluoromethyl)-1-{[2-(trimethylsilyl)ethoxy]methyl}-1H-pyrrolo[2,3-b]pyridine (416 mg, 1.05 mmol), 4-amino-2-methoxyphenol (161 mg, 1.16 mmol) and potassium carbonate (436 mg, 3.16 mmol) in DMSO (4.2 mL) after purification using a Biotage chromatography system 361 mg (purity 94%, 66% yield) of the desired title compound.
¹H-NMR (400 MHz, DMSO-d6) δ [ppm]: -0.11 - -0.03 (m, 9H), 0.82 - 0.88 (m, 2H), 3.53 - 3.60 (m, 2H), 3.64 (s, 3H), 5.28 (s, 2H), 5.60 (s, 2H), 6.20 (d, 2H), 6.42 (d, 1H), 6.85 (d, 1H), 8.30 (s, 1H).

### Intermediate 23

### 3-methoxy-4-{[3-(trifluoromethyl)-1-{[2-(trimethylsilyl)ethoxy]methyl}-1H-pyrrolo[2,3-b]pyridin-4-l]oxaniline

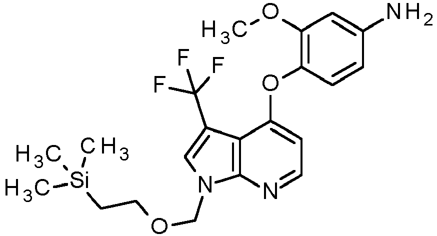

In analogy to intermediate 2), we obtained from 4-{[6-chloro-3-(trifluoromethyl)-1-{[2-(trimethylsilyl)ethoxy]methyl}-1H-pyrrolo[2,3-b]pyridin-4-yl]oxy}-3-methoxyaniline (315 mg, 646 µmol, intermediate 22) in an hydrogenation reaction using palladium on carbon (31.5 mg, 10 % purity, 29.6 µmol) and triethylamine (110 µL, 770 µmol) in ethanol (22 mL) after purification using a Biotage chromatography system 83 mg (84% purity, 24% yield) of the desired title compound.
¹H-NMR (400 MHz, DMSO-d6) δ [ppm]: -0.10 - -0.05 (m, 9H), 0.81 - 0.86 (m, 2H), 3.53 - 3.59 (m, 2H), 3.62 (s, 3H), 5.20 (br s, 2H), 5.65 (s, 2H), 6.19 (dd, 1H), 6.29 (d, 1H), 6.41 (d, 1H), 6.81 (d, 1H), 8.17 (d, 1H), 8.23 (s, 1H).

### Intermediate 24

### phenyl (3-methoxy-4-{[3-(trifluoromethyl)-1-{[2-(trimethylsilyl)ethoxy]methyl}-1H-pyrrolo[2,3-b]pyridin-4-yl]oxy}phenyl)carbamate

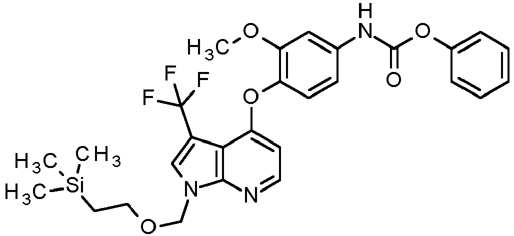

In analogy to intermediate 3), we obtained from 3-methoxy-4-{[3-(trifluoromethyl)-1-{[2-(trimethylsilyl)ethoxy]methyl}-1H-pyrrolo[2,3-b]pyridin-4-yl]oxy}aniline (79.0 mg, 174 µmol, intermediate 23) and phenyl carbonochloridate (24 µL, 190 µmol) in a mixture of pyridine (80 µL, 990 µmol) and THF (1.2 mL) after purification using a Biotage chromatography system 86 mg (92% purity, 79% yield) of the desired title compound.
¹H-NMR (400 MHz, DMSO-d6) δ [ppm]: -0.11 - -0.04 (m, 9H), 0.80 - 0.88 (m, 2H), 3.54 - 3.60 (m, 2H), 3.68 (s, 3H), 5.62 - 5.69 (m, 2H), 5.76 (s, 1H), 6.30 (d, 1H), 6.73 - 6.78 (m, 1H), 7.12 - 7.31 (m, 4H), 7.40 - 7.48 (m, 1H), 7.49 - 7.52 (m, 1H), 8.20 (d, 1H), 8.28 (s, 1H), 10.40 (br s, 1H).

### Intermediate 25

### 1-(3-methoxy-4-{[3-(trifluoromethyl)-1-{[2-(trimethylsilyl)ethoxy]methyl}-1H-pyrrolo[2,3-b]pyridin-4-yl]oxy}phenyl)-3-[3-(morpholin-4-yl)propyl]urea

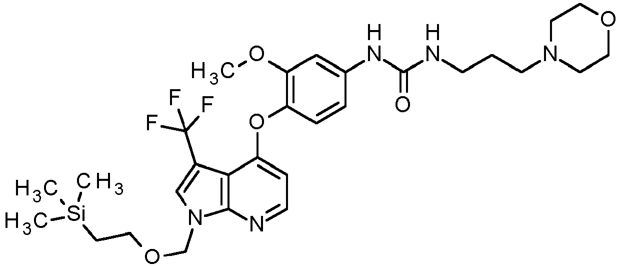

In analogy to intermediate 4), we obtained from phenyl (3-methoxy-4-{[3-(trifluoromethyl)-1-{[2-(trimethylsilyl)ethoxy]methyl}-1H-pyrrolo[2,3-b]pyridin-4-yl]oxy}phenyl)carbamate (81.0 mg, 141 µmol, intermediate 24) and 3-(morpholin-4-yl)propan-1-amine (21 µL, 140 µmol) in DMF (700 µL) after purification using a Biotage chromatography system 84mg (89% purity, 85% yield) of the desired title compound.
¹H-NMR (400 MHz, DMSO-d6) δ [ppm]: -0.10 - -0.04 (m, 9H), 0.81 - 0.87 (m, 2H), 2.20 - 2.42 (m, 6H), 3.09 - 3.21 (m, 2H), 3.51 - 3.64 (m, 6H), 3.67 (s, 3H), 5.66 (s, 2H), 6.28 (d, 1H), 6.96 (br d, 1H), 7.05 (d, 1H), 7.45 (d, 1H), 8.18 (d, 1H), 8.27 (s, 1H).

### Intermediate 26

### 4-chloro-3-(trifluoromethyl)-1 H-pyrrolo[2,3-b]pyridine 7-oxide

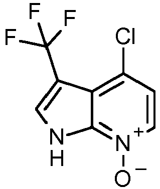

To a solution of 3-chlorobenzenecarboperoxoic acid (12.0 g, 69.6 mmol) in 98 mL dichlomethane was added portionwise at 0°C 4-chloro-3-(trifluoromethyl)-1H-pyrrolo[2,3-b]pyridine (11.8 g, 53.5 mmol). Stirring was continued at 0°C for 1h and at 25°C for 2 hours. The precipitate was collected by filtration. The solid was then suspended in 40 mL acetone and stirred for 3 hours. The solid was the collected by filtration to obtain 8.54 g (99 % purity, 67 % yield) of the desired title compound.
¹H-NMR (400 MHz, DMSO-d6) δ [ppm]: 7.38 (d, 1H), 8.24 (s, 1H), 8.30 (d, 1H).

### Intermediate 27

### 4,6-dichloro-3-(trifluoromethyl)-1H-pyrrolo[2,3-b]pyridine

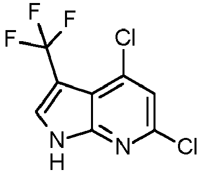

To a suspension of 4-chloro-3-(trifluoromethyl)-1H-pyrrolo[2,3-b]pyridine 7-oxide (8.54 g, 36.1 mmol, intermediate 26) 200 mL THF was added at 0°C 1,1,1-trimethyl-N-(trimethylsilyl)silanamine (7.5 mL, 36 mmol) followed by dropwise addition of trichloroacetyl chloride (14 mL, 130 mmol). The mixture was stirred for 15 minutes at 0°C and then for 2 hours at 25°C. The reaction was poured into 150 mL ice water and then was sodium bicarbonate added until pH=8. This mixture was extracted three times with 100 mL ethyl acetate. The combined organic layers were washed with brine, dried over sodium sulfate and after filtration evaporated to dryness.The residue was solved in a minimum of methanol and precipitated by the addition of water. The mixture was cooled at 0°C for 1 hour. The precipitate was collected by filtration, solved again in ethyl acetate, dried over sodium sulfate and after filtration evaporated to dryness to obtain 7.65 g (83 % yield) of the desired title compound.
¹H-NMR (400 MHz, DMSO-d6) δ [ppm]: 7.58 (s, 1H), 8.33 (s, 1H), 13.10 (br s, 1H).

### Intermediate 28

### 4,6-dichloro-3-(trifluoromethyl)-1-{[2-(trimethylsilyl)ethoxy]methyl}-1H-pyrrolo[2,3-b]pyridine

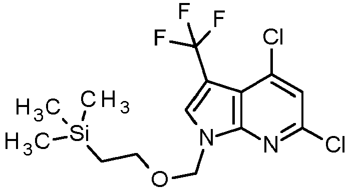

To a mixture of 4,6-dichloro-3-(trifluoromethyl)-1H-pyrrolo[2,3-b]pyridine (7.65 g, 30.0 mmol, intermediate 27) and [2-(chloromethoxy)ethyl](trimethyl)silane (5.8 mL, 33 mmol) in 77 mL DMF was added portionwise sodium hydride (3.60 g, 60 % purity, 90.0 mmol) at 0°C. After stirring for 3.5 hours this mixture was poured carefully into 100 mL ice water. This was then extracted three times with 50 mL ethyl acetate. The combined organic layers were washed with brine, dried over sodium sulfate and after filtration evaporated to dryness. The residue was purified using a Biotage chromatography system to obtain 175 mg (30 % purity, 88 % yield) of the desired title compound.
¹H-NMR (400 MHz, DMSO-d6) δ [ppm]: -0.12 - -0.08 (m, 9H), 0.79 - 0.87 (m, 2H), 3.51 - 3.60 (m, 2H), 5.64 (s, 2H), 7.70 (s, 1H), 8.58 (s, 1H).

### Intermediate 29

### 4-{[6-chloro-3-(trifluoromethyl)-1-{[2-(trimethylsilyl)ethoxy]methyl}-1H-pyrrolo[2,3-b]pyridin-4-yl]oxy}-3-(trifluoromethoxy)aniline

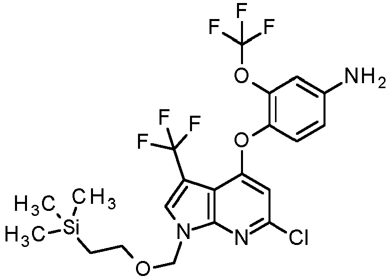

In analogy to intermediate 1), we obtained from 4,6-dichloro-3-(trifluoromethyl)-1-{[2-(trimethylsilyl)ethoxy]methyl}-1H-pyrrolo[2,3-b]pyridine (500 mg, 1.30 mmol, intermediate 28), 4-amino-2-(trifluoromethoxy)phenol (276 mg, 1.43 mmol) and potassium carbonate (538 mg, 3.89 mmol) in DMSO (5.0 mL, 70 mmol) after purification using a Biotage chromatography system 370 mg (93 % purity, 49 % yield) of the desired title compound.
¹H-NMR (400 MHz, DMSO-d6) δ [ppm]: -0.14 - -0.09 (m, 9H), 0.79 - 0.87 (m, 2H), 3.54 - 3.60 (m, 2H), 5.61 (s, 2H), 5.68 (s, 2H), 6.31 (s, 1H), 6.65 (dd, 1H), 6.70 - 6.73 (m, 1H), 7.17 (d, 1H), 8.35 (s, 1H).

### Intermediate 30

### 3-(trifluoromethoxy)-4-{[3-(trifluoromethyl)-1-{[2-(trimethylsilyl)ethoxy]methyl}-1H-pyrrolo[2,3-b]pyridin-4-yl]oxy}aniline

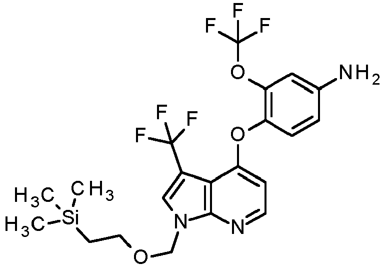

In analogy to intermediate 2), we obtained from 4-{[6-chloro-3-(trifluoromethyl)-1-{[2-(trimethylsilyl)ethoxy]methyl}-1H-pyrrolo[2,3-b]pyridin-4-yl]oxy}-3-(trifluoromethoxy)aniline (365 mg, 673 µmol), intermediate 29) in an hydrogenation reaction using palladium on carbon (36.5 mg, 10 % purity, 34.3 µmol) and triethylamine (110 µL, 810 µmol) in ethanol (23 mL, 390 mmol) after purification using a Biotage chromatography system 259 mg (92 % purity, 70 % yield) of the desired title compound.
¹H-NMR (400 MHz, DMSO-d6) δ [ppm]: -0.15 - -0.08 (m, 9H), 0.78 - 0.84 (m, 2H), 3.53 - 3.60 (m, 2H), 5.60 (s, 2H), 5.67 (s, 2H), 6.38 (d, 1H), 6.64 (dd, 1H), 6.68 - 6.71 (m, 1H), 7.11 (d, 1H), 8.23 (d, 1H), 8.28 (s, 1H).

### Intermediate 31

### phenyl [3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]carbamate

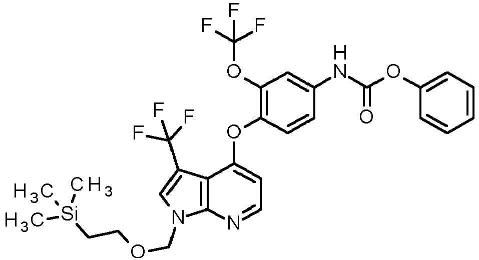

In analogy to intermediate 3), we obtained from 3-(trifluoromethoxy)-4-{[3-(trifluoromethyl)-1-{[2-(trimethylsilyl)ethoxy]methyl}-1H-pyrrolo[2,3-b]pyridin-4-yl]oxy}aniline (253 mg, 499 µmol), intermediate 30) and phenyl carbonochloridate (69 µL, 550 µmol) in a mixture of pyridine (240 µL, 2.9 mmol) and THF (3.5 mL) after purification using a Biotage chromatography system 282 mg (89 % purity, 81 % yield) of the desired title compound.
¹H-NMR (400 MHz, DMSO-d6) δ [ppm]: -0.15 - -0.07 (m, 9H), 0.78 - 0.87 (m, 2H), 3.54 - 3.60 (m, 2H), 5.68 (s, 2H), 6.47 (d, 1H), 6.72 - 6.77 (m, 1H), 7.24 - 7.31 (m, 2H), 7.42 - 7.48 (m, 2H), 7.51 (d, 1H), 7.57 (dd, 1H), 7.86 (s, 1H), 8.27 (d, 1H), 8.34 (s, 1H), 10.69 (br s, 1H).

### Intermediate 32

### 1-[3-(morpholin-4-yl)propyl]-3-[3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]urea

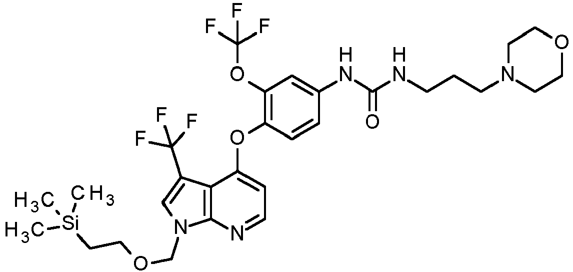

In analogy to intermediate 4), we obtained from phenyl [3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]carbamate (135 mg, 215 µmol), intermediate 31) and 3-(morpholin-4-yl)propan-1-amine (32 µL, 220 µmol) in DMF (1.1 mL) after purification using a Biotage chromatography system 117 mg (98 % purity, 78 % yield) of the desired title compound.
¹H-NMR (400 MHz, DMSO-d6) δ [ppm]: -0.14 - -0.07 (m, 9H), 0.78 - 0.86 (m, 2H), 1.60 (quin, 2H), 2.27 - 2.38 (m, 6H), 3.13 (q, 2H), 3.53 - 3.62 (m, 6H), 5.68 (s, 2H), 6.32 (t, 1H), 6.42 (d, 1H), 7.30 - 7.37 (m, 2H), 7.90 (s, 1H), 8.25 (d, 1H), 8.32 (s, 1H), 8.93 (s, 1H).

### Intermediate 33

### 1-[2-(morpholin-4-yl)ethyl]-3-[3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]urea

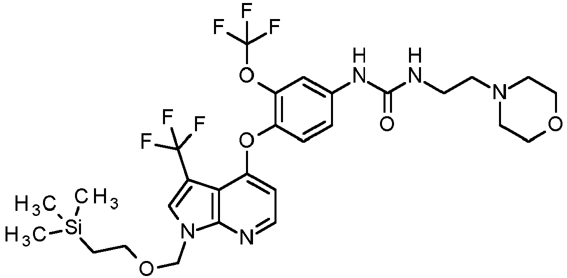

In analogy to intermediate 4), we obtained from phenyl [3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]carbamate (135 mg, 215 µmol), intermediate 31) and 2-(morpholin-4-yl)ethanamine (28 µL, 220 µmol) in DMF (1.1 mL) after purification using a Biotage chromatography system 111 mg (99 % purity, 77 % yield) of the desired title compound. ¹H-NMR (400 MHz, DMSO-d6) δ [ppm]: -0.16 - -0.08 (m, 9H), 0.78 - 0.85 (m, 2H), 2.36 - 2.44 (m, 6H), 3.23 (q, 2H), 3.54 - 3.63 (m, 6H), 5.68 (s, 2H), 6.21 (t, 1H), 6.43 (d, 1H), 7.31 (dd, 1H), 7.35 (d, 1H), 7.89 - 7.93 (m, 1H), 8.25 (d, 1H), 8.32 (s, 1H), 9.10 (s, 1H).

### Example 1

### 1-[2-(morpholin-4-yl)ethyl]-3-[3-(trifluoromethyl)-4-{[3-(trifluoromethyl)-1H-pyrrolo[2,3-b]pyridin-4-yl]oxy}phenyl]urea

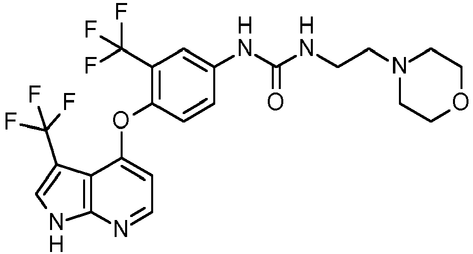

To a solution of 1-[2-(morpholin-4-yl)ethyl]-3-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea (139 mg, 215 µmol, intermediate 4) in dichloromethane (2.9 mL) was added trifluoroacidic acid (1.5 mL, 19 mmol) and this mixture was stirred for 3 hours at room temperature. The reaction mixture was carefully poured into an aqueous solution of sodium hydrogencarbonate. This aqueous phase was extracted two times with ethyl actetate. Then the combined organic phases were washed with brine, dried over sodium sulfate and then after filtration evaporated to dryness in vacuum. The obtained crude product was purified via a Biotage chromatography system (11 g snap KP-NH column, ethyl acetate / 0 - 50% methanol) to obtain 104.4 mg (95 % purity, 89 % yield) of the desired title compound.
¹H-NMR (500 MHz, DMSO-d6) δ [ppm]: 2.38 - 2.44 (m, 6H), 3.23 (q, 2H), 3.60 (t, 4H), 6.23 (t, 1H), 6.36 (d, 1H), 7.29 (d, 1H), 7.61 (dd, 1H), 8.06 - 8.09 (m, 2H), 8.19 (d, 1H), 9.12 (s, 1H), 12.57 (br s, 1H).

### Example 2

### 1-[3-(morpholin-4-yl)propyl]-3-[3-(trifluoromethyl)-4-{[3-(trifluoromethyl)-1H-pyrrolo[2,3-b]pyridin-4-yl]oxy}phenyl]urea

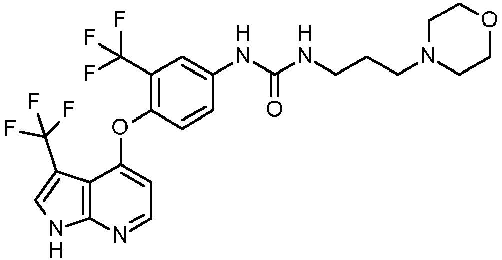

In analogy to example 1), 1-[3-(morpholin-4-yl)propyl]-3-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea (132 mg, 199 µmol, intermediate 5) was stirred with trifluoroacidic acid (1.4 mL, 18 mmol) in dichloromethane (2.7 mL) to obtain 100.7 mg (95 % purity, 90 % yield) of the desired title compound.
¹H-NMR (500 MHz, DMSO-d6) δ [ppm]: 1.61 (quin, 2H), 2.28 - 2.37 (m, 6H), 3.13 (q, 2H), 3.58 (t, 4H), 6.32 (t, 1H), 6.36 (d, 1H), 7.28 (d, 1H), 7.62 (dd, 1H), 8.05 - 8.09 (m, 2H), 8.19 (d, 1H), 8.92 (s, 1H), 12.57 (s, 1H).

### Example 3

### 1-[3-(trifluoromethyl)-4-{[3-(trifluoromethyl)-1H-pyrrolo[2,3-b]pyridin-4-yl]oxy}phenyl]urea

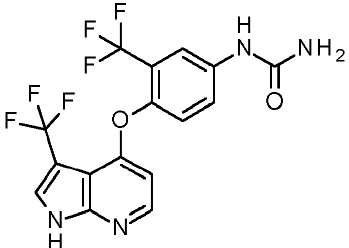

In analogy to example 1), 1-[3-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]urea (9.00 mg, 16.8 µmol, intermediate 6) was stirred with trifluoroacidic acid (120 µL, 1.6 mmol) in dichloromethane (240 µL) to obtain 6.6 mg (97 % purity, 94 % yield) of the desired title compound.
¹H-NMR (400 MHz, METHANOL-d4) δ [ppm]: 6.39 (d, 1H), 7.20 (d, 1H), 7.65 (dd, 1H), 7.77 - 7.82 (m, 1H), 7.97 (d, 1H), 8.15 (d, 1H).

### Example 4

### 1-[3-fluoro-5-(trifluoromethyl)-4-{[3-(trifluoromethyl)-1H-pyrrolo[2,3-b]pyridin-4-yl]oxy}phenyl]-3-[3-(morpholin-4-yl)propyl]urea

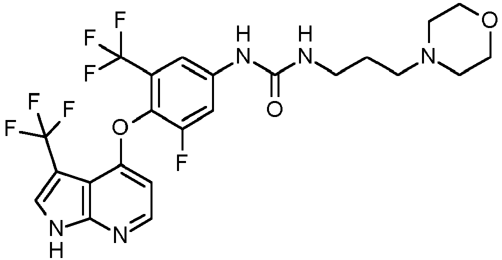

In analogy to example 1), 1-[3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]-3-[3-(morpholin-4-yl)propyl]urea (94.0 mg, 138 µmol, intermediate 10) was stirred with trifluoroacidic acid (950 µL, 12 mmol) in dichloromethane (1.9 mL) to obtain after an additional HPLC-purification (method 1) 43.1 mg (97 % purity, 55 % yield) of the desired title compound.
¹H-NMR (400 MHz, DMSO-d6) δ [ppm]: 1.61 (quin, 2H), 2.26 - 2.39 (m, 6H), 3.14 (q, 2H), 3.54 - 3.62 (m, 4H), 6.40 (dd, 1H), 6.46 (t, 1H), 7.75 (s, 1H), 7.84 (dd, 1H), 8.10 (d, 1H), 8.19 (d, 1H), 9.14 (s, 1H), 12.62 (br d, 1H).

### Example 5

### 1-[3-fluoro-5-(trifluoromethyl)-4-{[3-(trifluoromethyl)-1H-pyrrolo[2,3-b]pyridin-4-yl]oxy}phenyl]-3-[2-(morpholin-4-yl)ethyl]urea

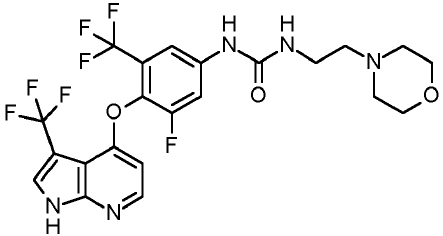

In analogy to example 1), 1-[3-fluoro-5-(trifluoromethyl)-4-{[3-(trifluoromethyl)-1-{[2-(trimethylsilyl)ethoxy]methyl}-1H-pyrrolo[2,3-b]pyridin-4-yl]oxy}phenyl]-3-[2-(morpholin-4-yl)ethyl]urea (86.0 mg, 129 µmol, intermediate 11) was stirred with trifluoroacidic acid (900 µL, 12 mmol) in dichloromethane (1.8 mL) to obtain after an additional HPLC-purification (method 1) 34.7 mg (97 % purity, 49 % yield) of the desired title compound.
¹H-NMR (400 MHz, DMSO-d6) δ [ppm]: 2.37 - 2.46 (m, 6H), 3.24 (q, 2H), 3.55 - 3.63 (m, 4H), 6.35 (t, 1H), 6.40 (dd, 1H), 7.74 (s, 1H), 7.82 (dd, 1H), 8.10 (s, 1H), 8.20 (d, 1H), 9.34 (s, 1H), 12.62 (s, 1H).

### Example 6

### 1-{4-[(3-chloro-1H-pyrrolo[2,3-b]pyridin-4-yl)oxy]-3-(trifluoromethyl)phenyl}-3-[3-(morpholin-4-yl)propyl]urea

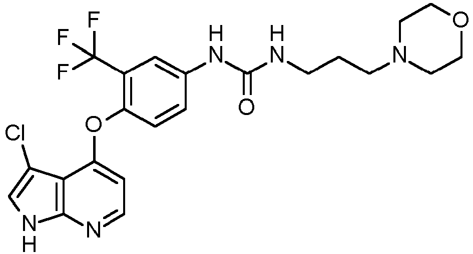

In analogy to example 1), 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-[3-(morpholin-4-yl)propyl]urea (86.0 mg, 137 µmol, intermediate 17) was stirred with trifluoroacidic acid (950 µL, 12 mmol) in dichloromethane (1.9 mL) to obtain after an additional HPLC-purification (method 1) 35.1 mg (97 % purity, 50 % yield) of the desired title compound.
¹H-NMR (400 MHz, DMSO-d6) δ [ppm]: 1.60 (quin, 2H), 2.27 - 2.37 (m, 6H), 3.13 (q, 2H), 3.54 - 3.61 (m, 4H), 6.28 - 6.33 (m, 2H), 7.26 (d, 1H), 7.57 - 7.62 (m, 2H), 8.06 (s, 1H), 8.10 (d, 1H), 8.90 (s, 1H), 12.08 (br s, 1H).

### Example 7

### 1-{4-[(3-chloro-1H-pyrrolo[2,3-b]pyridin-4-yl)oxy]-3-(trifluoromethyl)phenyl}-3-[2-(morpholin-4-yl)ethyl]urea

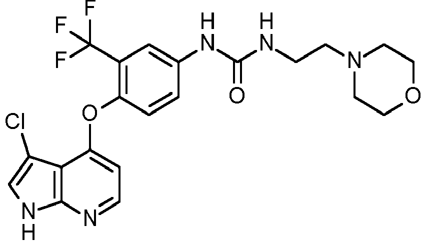

In analogy to example 1), 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-[2-(morpholin-4-yl)ethyl]urea (85.0 mg, 138 µmol, intermediate 18) was stirred with trifluoroacidic acid (950 µL, 12 mmol) in dichloromethane (1.9 mL) to obtain after an additional HPLC-purification (method 1) 11.2 mg (95 % purity, 16 % yield) of the desired title compound.
¹H-NMR (400 MHz, DMSO-d6) δ [ppm]: 2.37 - 2.44 (m, 6H), 3.17 - 3.28 (m, 2H), 3.53 - 3.66 (m, 4H), 6.21 (br s, 1H), 6.30 (br d, 1H), 7.27 (br d, 1H), 7.58 (br s, 2H), 8.03 - 8.15 (m, 2H), 9.09 (br s, 1H), 12.08 (br s, 1H).

### Example 8

### 1-{4-[(3-chloro-1H-pyrrolo[2,3-b]pyridin-4-yl)oxy]-3-(trifluoromethyl)phenyl}urea

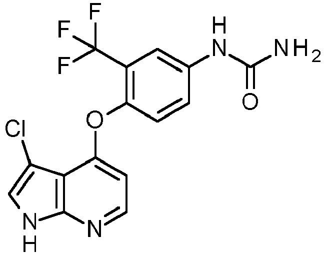

In analogy to example 1), 1-{4-[(3-chloro-1-{[2-(trimethylsilyl)ethoxy]methyl}-1H-pyrrolo[2,3-b]pyridin-4-yl)oxy]-3-(trifluoromethyl)phenyl}urea (79.0 mg, 158 µmol, intermediate 19) was stirred with trifluoroacidic acid (1.1 mL, 14 mmol) in dichloromethane (2.2 mL) to obtain 38.4 mg (93 % purity, 61 % yield) of the desired title compound.
¹H-NMR (400 MHz, DMSO-d6) δ [ppm]: 6.04 (br s, 2H), 6.30 (d, 1H), 7.27 (d, 1H), 7.58 (d, 1H), 7.62 (dd, 1H), 8.06 (d, 1H), 8.10 (d, 1H), 8.98 (s, 1H), 12.08 (br s, 1H).

### Example 9

### 1-{4-[(3-chloro-1H-pyrrolo[2,3-b]pyridin-4-yl)oxy]-3-(trifluoromethyl)phenyl}-3-pyridazin-3-ylurea

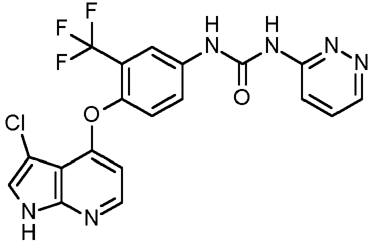

In analogy to example 1), 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-pyridazin-3-ylurea (53.0 mg, 91.5 µmol, intermediate 20) was stirred with trifluoroacidic acid (650 µL, 8.4 mmol) in dichloromethane (1.3 mL) to obtain 14.6 mg (90 % purity, 32 % yield) of the desired title compound.
¹H-NMR (400 MHz, DMSO-d6) δ [ppm]: 6.39 (d, 1H), 7.34 (br d, 1H), 7.60 (br s, 1H), 7.64 - 7.73 (m, 2H), 8.05 (d, 1H), 8.11 - 8.18 (m, 2H), 8.91 (d, 1H), 9.90 (br s, 1H), 10.11 (br s, 1H), 12.12 (br s, 1H).

### Example 10

### 1-{4-[(3-chloro-1H-pyrrolo[2,3-b]pyridin-4-yl)oxy]-3-(trifluoromethyl)phenyl}-3-(2-methylpyrimidin-5-yl)urea

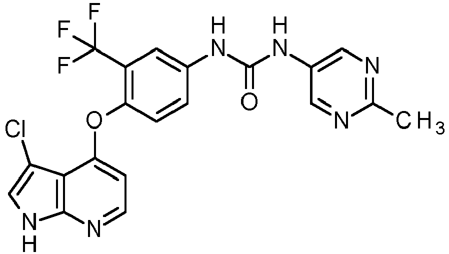

In analogy to example 1), 1-{4-[(3-chloro-1-{[2-(trimethylsilyl)ethoxy]methyl}-1H-pyrrolo[2,3-b]pyridin-4-yl)oxy]-3-(trifluoromethyl)phenyl}-3-(2-methylpyrimidin-5-yl)urea (67.0 mg, 113 µmol, intermediate 21) was stirred with trifluoroacidic acid (800 µL, 10 mmol) in dichloromethane (1.6 mL) to obtain 33.8 mg (97 % purity, 63 % yield) of the desired title compound.
¹H-NMR (400 MHz, DMSO-d6) δ [ppm]: 2.56 (s, 3H), 6.37 (d, 1H), 7.33 (d, 1H), 7.60 (d, 1H), 7.71 (dd, 1H), 8.09 (s, 1H), 8.13 (d, 1H), 8.81 (s, 2H), 9.07 (s, 1H), 9.41 (s, 1H), 12.11 (br s, 1H).

### Example 11

### 1-(3-methoxy-4-{[3-(trifluoromethyl)-1H-pyrrolo[2,3-b]pyridin-4-yl]oxy}phenyl)-3-[3-(morpholin-4-yl)propyl]urea

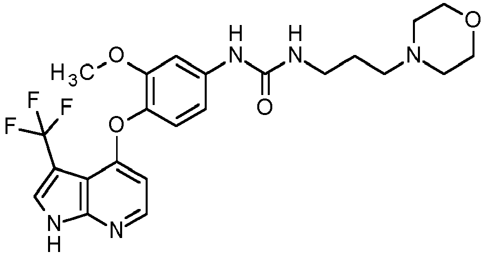

In analogy to example 1), 1-(3-methoxy-4-{[3-(trifluoromethyl)-1-{[2-(trimethylsilyl)ethoxy]methyl}-1H-pyrrolo[2,3-b]pyridin-4-yl]oxy}phenyl)-3-[3-(morpholin-4-yl)propyl]urea (79.0 mg, 127 µmol, intermediate 25) was stirred with trifluoroacidic acid (900 µL, 12 mmol) in dichloromethane (1.8 mL) to obtain 53.8 mg (92 % purity, 79 % yield) of the desired title compound.
¹H-NMR (400 MHz, DMSO-d6) δ [ppm]: 1.60 (quin, 2H), 2.27 - 2.39 (m, 6H), 3.12 (q, 2H), 3.55 - 3.61 (m, 4H), 3.67 (s, 3H), 6.16 - 6.22 (m, 2H), 6.94 (dd, 1H), 7.02 (d, 1H), 7.44 (d, 1H), 8.01 (s, 1H), 8.11 (d, 1H), 8.61 (s, 1H), 12.44 (s, 1H).

### Example 12

### 1-[3-(morpholin-4-yl)propyl]-3-[3-(trifluoromethoxy)-4-{[3-(trifluoromethyl)-1H-pyrrolo[2,3-b]pyridin-4-yl]oxy}phenyl]urea

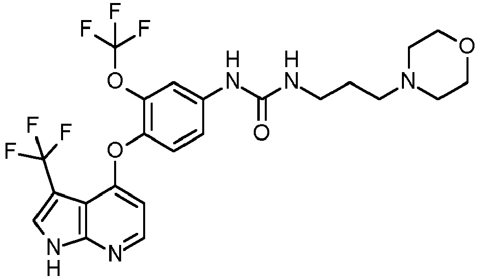

In analogy to example 1), 1-[3-(morpholin-4-yl)propyl]-3-[3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]urea (113 mg, 167 µmol, intermediate 32) was stirred with trifluoroacidic acid (1.2 mL, 15 mmol) in dichloromethane (2.3 mL) to obtain 78.8 mg (97 % purity, 84 % yield) of the desired title compound.
¹H-NMR (400 MHz, DMSO-d6) δ [ppm]: 1.60 (quin, 2H), 2.27 - 2.39 (m, 6H), 3.13 (q, 2H), 3.54 - 3.62 (m, 4H), 6.30 (t, 1H), 6.33 (d, 1H), 7.32 (s, 2H), 7.89 (d, 1H), 8.07 (s, 1H), 8.18 (d, 1H), 8.91 (s, 1H), 12.56 (br s, 1H).

### Example 13

### 1-[2-(morpholin-4-yl)ethyl]-3-[3-(trifluoromethoxy)-4-{[3-(trifluoromethyl)-1H-pyrrolo[2,3-b]pyridin-4-yl]oxy}phenyl]urea

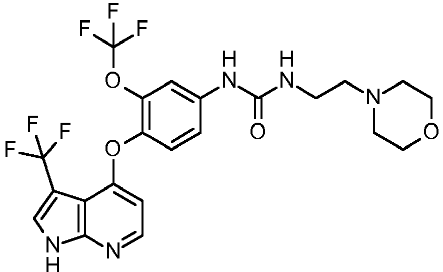

In analogy to example 1), 1-[2-(morpholin-4-yl)ethyl]-3-[3-(trifluoromethoxy)-4-{[3-(trifluoromethyl)-1-{[2-(trimethylsilyl)ethoxy]methyl}-1H-pyrrolo[2,3-b]pyridin-4-yl]oxy}phenyl]urea (107 mg, 161 µmol, intermediate 33) was stirred with trifluoroacidic acid (1.1 mL, 14 mmol) in dichloromethane (2.2 mL) to obtain 45.8 mg (97 % purity, 52 % yield) of the desired title compound.
¹H-NMR (400 MHz, DMSO-d6) δ [ppm]: 2.36 - 2.43 (m, 6H), 3.23 (q, 2H), 3.55 - 3.64 (m, 4H), 6.21 (t, 1H), 6.33 (d, 1H), 7.27 - 7.35 (m, 2H), 7.91 (s, 1H), 8.07 (s, 1H), 8.18 (d, 1H), 9.10 (s, 1H), 12.56 (br s, 1H).

### EXPERIMENTAL SECTION - BIOLOGICAL ASSAYS

### Biological in vitro assays

The *in vitro* activity of the compounds of the present invention can be demonstrated in the following assays:
The example testing experiments described herein serve to illustrate the present invention and the invention is not limited to the examples given.

### Biological Evaluation

In order that this invention may be better understood, the following examples are set forth. These examples are for the purpose of illustration only, and are not to be construed as limiting the scope of the invention in any manner. All publications mentioned herein are incorporated by reference in their entirety.
Demonstration of the activity of the compounds of the present invention may be accomplished through in vitro and in vivo assays that are well known in the art. For example, to demonstrate the efficacy of a pharmaceutical agent to inhibit and be selective against e.g. TBK1 the following assays may be used.

### Binding competition assay

The ability of the compounds of the present invention to inhibit the binding of an Alexa647-labelled ATP-competitive kinase inhibitor to a Glutathione-S-transferase- (GST-) fusion protein was quantified employing the TR-FRET-based binding competition assay as described in the following paragraphs.

A recombinant fusion protein of N-terminal GST and full-length human , expressed by baculovirus infected SF9 insect cells and purified by Glutathione Sepharose affinity chromatography, was used as GST- fusion protein. Tracer 222 from Invitrogen (catalogue no. PR9198A) was used as Alexa647-labelled ATP-competitive kinase inhibitor.
For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 3 µl solution of Tracer 222 (25 nM => final concentration in 5 µl assay volume is 15 nM) in aqueous assay buffer [25 mM Tris/HCI pH 7.5, 10 mM MgCl₂, 5 mM β-glycerolphosphate, 2.5 mM dithiothreitol, 0.5 mM ethylene glycol-bis(2-aminoethylether)-*N*,*N*,*N*',*N'*-tetraacetic acid [EGTA], 0.5 mM sodium orthovanadate, 0.01 % (w/v) bovine serum albumin [BSA], 0.005% (w/v) Pluronic F-127 (Sigma)] were added. Then the binding competition was started by the addition of 2 µl of a solution of the GST- fusion protein (2.5 nM => final conc. in the 5 µl assay volume is 1 nM) and of Anti-GST-Tb (1.25 nM => final conc. in the 5 µl assay volume is 0.5 nM), a Lumi4®-Tb Cryptate-conjugated anti-GST-antibody from Cisbio Bioassays (France), in assay buffer.

The resulting mixture was incubated 30 min at 22°C to allow the formation of a complex between the Tracer 222, the fusion protein and Anti-GST-Tb. Subsequently the amount of this complex was evaluated by measurement of the resonance energy transfer from the Tb-cryptate to the Tracer 222. Therefore, the fluorescence emissions at 620 nm and 665 nm after excitation at 350 nm were measured in a TR-FRET reader, e.g. a Pherastar (BMG Labtechnologies, Offenburg, Germany) or a Viewlux (Perkin-Elmer). The ratio of the emissions at 665 nm and at 622 nm was taken as the measure for the amount of the complex. The data were normalised (assay reaction without inhibitor = 0 % inhibition, all other assay components but GST- fusion protein = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener™ software.

**Table 1: Measured IC₅₀ values of compounds regarding inhibition**

| **Example** | **IC₅₀ [nM]** |
|---|---|
| 1 | 69,4 |
| 2 | 56,2 |
| 3 | 56,2 |
| 4 | 65 |
| 5 | 64,6 |
| 6 | 158 |
| 7 | 124 |
| 8 | 188 |
| 9 | 1260 |
| 10 | 171 |
| 11 | 315 |
| 12 | 469 |
| 13 | 439 |

### TBK1 high ATP kinase assay

TBK1 -inhibitory activity of compounds of the present invention at a high ATP concentration after preincubation of enzyme and test compounds was quantified employing the TR-FRET-based TBK1 assay as described in the following paragraphs.

Recombinant full-length N-terminally His-tagged human TBK1, expressed in insect cells and purified by Ni-NTA affinity chromatography, was purchased from Life Technologies (Cat. No PR5618B) and used as enzyme. As substrate for the kinase reaction biotinylated peptide biotin-Ahx-GDEDFSSFAEPG (C-terminus in amide form) was used which can be purchased e.g. form the company Biosyntan (Berlin-Buch, Germany).
For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 2 µl of a solution of TBK1 in aqueous assay buffer [50 mM HEPES pH 7.0, 10 mM MgCl₂, 1.0 mM dithiothreitol, 0.05 % (w/v) bovine serum albumine, 0.01% (v/v) Nonidet-P40 (Sigma), protease inhibitor mixture ("Complete w/o EDTA" from Roche, 1 tablet per 5 mL)] were added and the mixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 1.67 mM => final conc. in the 5 µl assay volume is 1 mM) and substrate (1.67 µM => final conc. in the 5 µl assay volume is 1 µM) in assay buffer and the resulting mixture was incubated for a reaction time of 30 min at 22°C. The concentration of TBK1 was adjusted depending of the activity of the enzyme lot and was chosen appropriate to have the assay in the linear range, typical concentrations were in the range of 0.002-0.004 µg/mL. The reaction was stopped by the addition of 3 µl of a solution of TR-FRET detection reagents (0.33 µM streptavidine-XL665 [Cisbio Bioassays, Codolet, France], 2.5 nM anti-phosho-Serine antibody [Merck Millipore, "STK antibody", cat. # 35-002] and 1.25 nM LANCE EU-W1024 labeled anti-mouse IgG antibody [Perkin-Elmer, product no. AD0077]) in an aqueous EDTA-solution (167 mM EDTA, 0.13 % (w/v) bovine serum albumin in 100 mM HEPES/NaOH pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM,
0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener™ software.

**Table 2: Measured IC₅₀ values of compounds regarding TBK1 inhibition as selectivity assay**

| **Example** | **IC₅₀ [nM]** |
|---|---|
| 1 | >20 |
| 2 | >20 |
| 3 | >20 |
| 4 | >20 |
| 5 | >20 |
| 6 | >20 |
| 7 | >20 |
| 8 | >20 |
| 9 | >20 |
| 10 | >20 |
| 11 | >20 |
| 12 | >20 |
| 13 | >20 |

### Phosphorylation assay in human cell line

Phosphorylation assays were carried out in Jurkat E6.1 cells from American Type Culture Collection (ATCC) stably overexpressing human FLAG-tagged SLP-76 (proprietary). Cultured cells were kept in RPMI 1640 medium supplemented with 1% FCS at a cell density of 2x 10e6/mL 24h prior compound testing. Starved cells were simultaneously treated with 350 ng/mL a-CD3 antibody (clone OKT3. ebioscience #16-0037-85. plate-bound) and test compound for 30 min at 37 °C. Applied compounds were tested at either fixed concentration of 10 µmol/L and 20 µmol/L or in a 8 point dose response titration of increase compound concentration with 10 nmol/L. 50 nmol/L. 100 nmol/L. 500 nmol/L. 1 µmol/L. 5 µmol/L. 10 µmol/L and 20 µmol/L in triplicates. The cells were washed once in phosphate-buffered saline (pH 7.4). Cells were lysed using a lysis buffer containing 50 mmol/L Tris-CI (pH 7.5). 150 mM NaCl. 2 mM EDTA. 1% Triton-X 100. 0.5 % Na-DOC. 0.1% SDS. 1/10 complete mini protease inhibitor cocktail (Roche #11836170001) and 1/10 PhosSTOP phosphatase inhibitor cocktail (Roche #04906837001). A total of 1.25 µg cell lysate was analyzed by capillary electrophoresis using the Peggy Sue™ System (proteinsimple® San Jose. CA USA) with a 12-230kDa sizebased master kit with split buffer / a-rabbit-HRP #PS-MK18 / a-mouse-HRP #PS-MK19 according to manufacture's protocol. Probe antibodies used were a rabbit monoclonal antibody supernatant raised against human phospho-Ser376-SLP-76 peptide (proprietary) and for mormalization an a-alpha-Tubulin. mouse monoclonal antibody (Sigma #T9026). As control for maximal effect (max control. which represent the maximally possible inhibition of pSer376-SLP-76 by a test compound) cells with no a-CD3 (clone OKT3. ebioscience #16-0037-85. plate-bound) and no test compound treatment were used. Cells with a-CD3 treatment only were used as negative control (min control. which represent the minimally possible inhibition of pSer376-SLP-76 by a test compound)

AUC values of each respective test sample were normalized using the AUC of housekeeping gene alpha-Tubulin and AUC of pSer376-SLP-76 of the min control. The percentage of the amount of pSer-SLP-76 in the treatment samples was calculated using the max control and min control values of the respective Peggy Sue™ run.

**Table 3: Measured % amount of pSer376-SLP-76 of compound**

| **Example** | **% amount of pSer376-SLP-76 @ 20µM** | **% amount of pSer376-SLP-76 @ 10µM** |
|---|---|---|
| 1 | 5.5 | 25.2 |
| 2 | 6.5 | 25.1 |
| 3 | 2.6 | 21.0 |
| 4 | <1 | 17.0 |
| 5 | <1 | 27.1 |
| 6 | 29.5 | 69.9 |
| 7 | <1 | 5.1 |
| 8 | 73.6 | 92.7 |
| 10 | <1 | 15.9 |
| 11 | 53.8 | 68.1 |
| 12 | 23.9 | 61.8 |

## Claims

1. Compounds of formula (I) 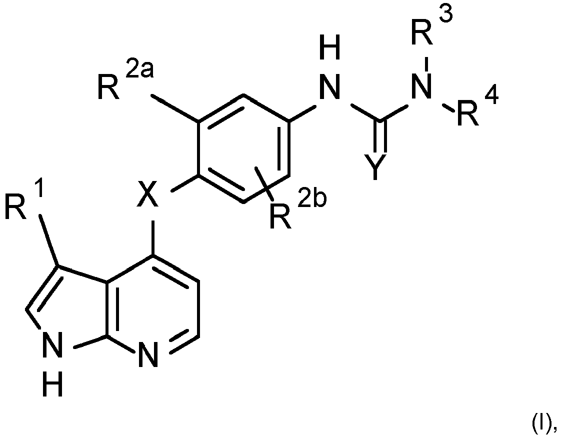 in which
X represents a nitrogen, a sulphur or an oxygene atom,
Y represents a sulphur or an oxygene atom,
R¹ represents hydrogen, halogen, cyano, C₁-C₆-alkyl or halo-C₁-C₆-alkyl,
R^{2a} represents C₁-C₆-alkoxy, halo-C₁-C₆-alkyl or halo-C₁-C₆-alkoxy
R^{2b} represents hydrogen, halogen, cyano or C₁-C₃-alkyl,
R³ represents hydrogen or C₁-C₆-alkyl,
R⁴ represents hydrogen or represents C₁-C₆-alkyl-,
which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy-, -S(=O)₂NH₂, -C(=O)-R⁹, -C(=O)-NH-R^{x}, -NH-C(=O)-R⁹, -NH-S(=O)₂-R⁹, -S(=O)₂-R⁹, -S(=O)(=NR^{y})-R^{x}, -S(=O)-R^{x}, C₃-C₁₀-cycloalkyl- which itself may optionally be mono- or polysubstituted by dentical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸,-C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms, monocyclic heteroaryl- having 5 or 6 ring atoms which itself may optionally be mono- or polysubstituted by identical or different substituents from the group consisting of halogen, amino, hydroxy, cyano, nitro, carboxy, C₁-C₆-alkyl-, C₁-C₆-alkoxy-, C₁-C₆- alkoxy-C₁-C₆-alkyl-, hydroxy-C₁-C₆-alkyl-, C₁-C₆-alkylamino-, amino-C₁-C₆-alkyl-, C₁-C₆-alkylamino-C₁-C₆-alkyl, halo-C₁-C₆-alkyl-, halo-C₁-C₆-alkoxy-, C₃-C₁₀-cycloalkyl and a monocyclic heterocyclyl radical having 3 to 8 ring atoms,
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
phenyl- which itself may optionally be mono- or polysubstituted by identical or different substituents from the group consisting of halogen, amino, hydroxy, cyano, nitro, carboxy, C₁-C₆-alkyl-, C₁-C₆-alkoxy-, C₁-C₆-alkoxy-C₁-C₆-alkyl-, C₁-C₆-alkyl-amino-, amino-C₁-C₆-alkyl-, C₁-C₆-alkylaminocarbonyl-, C₁-C₆-alkyl-aminosulphonyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
spirocycloalkyl-, heterospirocycloalkyl-, bicycloalkyl-, heterobicycloalkyl-, bridged cycloalkyl or a bridged heterocycloalkyl, naphthyl or bicyclic heteroaryl, or partially saturated bicyclic aryl- or heteroaryl,
each of which mentioned *supra* may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
R⁴ represents C₃-C₁₀-cycloalkyl- which may optionally be mono- or polysubstituted by dentical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents monocyclic heteroaryl- having 5 or 6 ring atoms which may optionally be mono- or polysubstituted by identical or different substituents from the group consisting of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents monocyclic heterocyclyl- having 3 to 8 ring atoms which may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents phenyl- which may optionally be mono- or polysubstituted by identical or different substituents from the group consisting of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
represents a spirocycloalkyl radical, a heterospirocycloalkyl radical, a bicycloalkyl, a heterobicycloalkyl radical, a bridged cycloalkyl radical or a bridged heterocycloalkyl radical, a naphthyl radical or a bicyclic heteroaryl radical, or a partially saturated bicyclic aryl- or heteroaryl radical, where the radicals mentioned may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
or
R³ and R⁴ together with the nitrogen atom form a 4 to 10 membered heterocycloalkyl ring, which may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-,-C(=O)-NR⁵R⁶, -NH-C(=O)-R⁸, -C(=O)-R⁷, -S(=O)₂-NR⁵R⁶, -S(=O)-R⁸,-S(=O)(=NR⁵)-R⁶, -S(=O)₂-R⁸, -NH-S(=O)₂-R⁸, or a monocyclic heterocyclyl radical having 3 to 8 ring atoms,
R⁵ and R⁶ independently of one another represent hydrogen, C₁-C₃-alkyl-, cyclopropyl- or di-C₁-C₃-alkylaminO-C₁-C₃-alkyl-,
R⁷ represents hydroxy, C₁-C₆-alkyl-, C₁-C₆-alkoxy-, halo-C₁-C₃-alkyl-, hydroxy-C₁-C₃-alkyl-, C₁-C₃-alkoxy-C₁-C₃-alkyl-, C₃-C₈-cycloalkyl-, phenyl-, monocyclic heterocyclyl- having 3 to 8 ring atoms or monocyclic heteroaryl- having 5 or 6 ring atoms where phenyl-, heteroaryl- and heterocyclyl- may optionally be mono- or disubstituted by halogen, C₁-C₃-alkoxy- or C₁-C₃-alkyl-,
R⁸ represents C₁-C₆-alkyl-,
R⁹ represents C₁-C₆-alkyl-, -NH₂, -NH-C₁-C₆-alkyl, -N(C₁-C₆-alkyl)₂, or C₁-C₆-alkoxy-C₁-C₆-alkyl-,
or represents monocyclic heterocyclyl- having 3 to 8 ring atoms which itself may optionally be mono- or polysubstituted by identical or different substituents from the group consisting of halogen, cyano, nitro, hydroxy, amino, oxo, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-, phenyl-, halophenyl-, phenyl-C₁-C₆-alkyl-, phenoxy-, pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, or a monocyclic heterocyclyl radical having 3 to 8 ring atoms,
R^{x} represents C₁-C₆-alkyl- or C₁-C₆-alkoxy-C₁-C₆-alkyl-,
R^{y} represents hydrogen, halo-C₁-C₆-alkyl, C₁-C₆-alkyl, C₁-C₆-alkyl substituted with C₃-C₆-cycloalkyl,
and their polymorphs, enantiomers, diastereomers, racemates, tautomers, solvates, physiologically acceptable salts and solvates of these salts.

2. The compound of general formula (**I**) according to any claims 1, wherein
X represents a nitrogen, a sulphur or an oxygene atom,
Y represents a sulphur or an oxygene atom,
R¹ represents hydrogen, halogen, cyano, C₁-C₃-alkyl or halo-C₁-C₃-alkyl,
R^{2a} represents C₁-C₃-alkoxy, halo-C₁-C₃-alkyl or halo-C₁-C₃-alkoxy
R^{2b} represents hydrogen, halogen, cyano or C₁-C₃-alkyl,
R³ represents hydrogen,
R⁴ represents hydrogen or represents C₁-C₆-alkyl-,
which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy-,
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
or
R⁴ represents a monocyclic heteroaryl- having 5 or 6 ring atoms which may optionally be mono- or polysubstituted by identical or different substituents from the group consisting of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-,
and their polymorphs, enantiomers, diastereomers, racemates, tautomers, solvates, physiologically acceptable salts and solvates of these salts.

3. The compound of general formula (**I**) according to claim 1 or 2, wherein
X represents a nitrogen, a sulphur or an oxygene atom,
Y represents a sulphur or an oxygene atom,
R¹ represents halogen or perfluoro-C₁-C₃-alkyl,
R^{2a} represents C₁-C₃-alkoxy, perfluoro-C₁-C₃-alkyl or perfluoro-C₁-C₃-alkoxy
R^{2b} represents hydrogen or halogen,
R³ represents hydrogen or C₁-C₆-alkyl,
R⁴ represents hydrogen or represents C₁-C₆-alkyl-,
which may optionally be mono- or polysubstituted by identical or different substituents from the group consisting of halogen, cyano, hydroxy, C₁-C₃-alkoxy-, -NH₂, -NH-(C₁-C₆-alkyl), -N(C₁-C₆-alkyl)₂, -NH-(C₁-C₆-alkyl)-C₁-C₆-alkoxy, C₁-C₆-alkyl-, C₁-C₆-alkoxy
monocyclic heterocyclyl- having 3 to 8 ring atoms which itself may optionally be mono- or polysubstituted by identical or different substituents from the group consisting of halogen, amino, hydroxy, cyano, oxo, carboxy, C₁-C₆-alkyl-, -carboxy-C₁-C₆-alkyl-, C₁-C₆-alkoxy-, C₁-C₆-alkoxy-C₁-C₆-alkyl-, C₁-C₆-alkylamino-, amino-C₁-C₆-alkyl-, C₁-C₆-alkylamino-C₁-C₆-alkyl-, hydroxy-C₁-C₆-alkyl-, halo-C₁-C₆-alkyl-, halo-C₁-C₆-alkoxy-, C₃-C₁₀-cycloalkyl- and a monocyclic heterocyclyl radical having 3 to 8 ring atoms,
or
R⁴ represents a monocyclic heteroaryl- having 6 ring atoms which may optionally be mono- or polysubstituted by identical or different substituents from the group consisting of halogen, cyano, nitro, hydroxy, amino, carboxy, C₁-C₆-alkyl-, C₁-C₆-alkoxy-, C₁-C₆-alkoxy-C₁-C₆-alkyl-, hydroxy-C₁-C₆-alkyl-, C₁-C₆-alkylamino-, C₁-C₆-alkylcarbonylamino-, amino-C₁-C₆-alkyl-, C₁-C₆-alkylamino-C₁-C₆-alkyl-, halo-C₁-C₆-alkyl-, halo-C₁-C₆-alkoxy-, C₃-C₁₀-cycloalkyl-,
and their polymorphs, enantiomers, diastereomers, racemates, tautomers, solvates, physiologically acceptable salts and solvates of these salts.

4. The compound of general formula (**I**) according to any one of claims 1 to 3, wherein
X represents an oxygene atom,
Y represents an oxygene atom,
R¹ represents chlorine or trifluoromethyl,
R^{2a} represents methoxy, trifluoromethyl or trifluoromethoxy,
R^{2b} represents hydrogen or fluorine,
R³ represents hydrogen,
R⁴ represents hydrogen or represents C₂- or C₃-alkyl-, which is substituted by 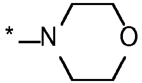 or
R⁴ represents a pyrimidinyl or pyridazinyl which may optionally be mono- or polysubstituted by C₁-C₆-alkyl-,
and their polymorphs, enantiomers, diastereomers, racemates, tautomers, solvates, physiologically acceptable salts and solvates of these salts.

5. The compound of general formula (**I**) according to any one of claims 1 to 3, wherein
X represents an oxygene atom,
and their polymorphs, enantiomers, diastereomers, racemates, tautomers, solvates,
physiologically acceptable salts and solvates of these salts.

6. The compound of general formula (**I**) according to any one of claims 1 to 3, wherein
Y represents an oxygene atom,
and their polymorphs, enantiomers, diastereomers, racemates, tautomers, solvates, physiologically acceptable salts and solvates of these salts.

7. The compound of general formula (**I**) according to any one of claims 1 to 3, wherein
R¹ represents chlorine or trifluoromethyl,
and their polymorphs, enantiomers, diastereomers, racemates, tautomers, solvates, physiologically acceptable salts and solvates of these salts.

8. The compound of general formula (**I**) according to any one of claims 1 to 3, wherein
R^{2a} represents methoxy, trifluoromethyl or trifluoromethoxy,
and their polymorphs, enantiomers, diastereomers, racemates, tautomers, solvates, physiologically acceptable salts and solvates of these salts.

9. The compound of general formula (**I**) according to any one of claims 1 to 3, wherein
R^{2b} represents hydrogen or fluorine,
and their polymorphs, enantiomers, diastereomers, racemates, tautomers, solvates, physiologically acceptable salts and solvates of these salts.

10. The compound of general formula (**I**) according to any one of claims 1 to 3, wherein
R³ represents hydrogen,
and their polymorphs, enantiomers, diastereomers, racemates, tautomers, solvates, physiologically acceptable salts and solvates of these salts.

11. The compound of general formula (**I**) according to any one of claims 1 to 3, wherein
R⁴ represents hydrogen or represents C₂- or C₃-alkyl-, which is substituted by 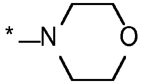 or
R⁴ represents a pyrimidinyl or pyridazinyl which may optionally be mono- or polysubstituted by C₁-C₆-alkyl-,
and their polymorphs, enantiomers, diastereomers, racemates, tautomers, solvates, physiologically acceptable salts and solvates of these salts.

12. The compound according to claim 1, which is selected from
• 1-[2-(morpholin-4-yl)ethyl]-3-[3-(trifluoromethyl)-4-{[3-(trifluoromethyl)-1H-pyrrolo[2,3-b]pyridin-4-yl]oxy}phenyl]urea
• 1-[3-(morpholin-4-yl)propyl]-3-[3-(trifluoromethyl)-4-{[3-(trifluoromethyl)-1H-pyrrolo[2,3-b]pyridin-4-yl]oxy}phenyl]urea
• 1-[3-(trifluoromethyl)-4-{[3-(trifluoromethyl)-1H-pyrrolo[2,3-b]pyridin-4-yl]oxy}phenyl]urea
• 1-[3-fluoro-5-(trifluoromethyl)-4-{[3-(trifluoromethyl)-1H-pyrrolo[2,3-b]pyridin-4-yl]oxy}phenyl]-3-[3-(morpholin-4-yl)propyl]urea
• 1-[3-fluoro-5-(trifluoromethyl)-4-{[3-(trifluoromethyl)-1H-pyrrolo[2,3-b]pyridin-4-yl]oxy}phenyl]-3-[2-(morpholin-4-yl)ethyl]urea
• 1-{4-[(3-chloro-1H-pyrrolo[2,3-b]pyridin-4-yl)oxy]-3-(trifluoromethyl)phenyl}-3-[3-(morpholin-4-yl)propyl]urea
• 1-{4-[(3-chloro-1H-pyrrolo[2,3-b]pyridin-4-yl)oxy]-3-(trifluoromethyl)phenyl}-3-[2-(morpholin-4-yl)ethyl]urea
• 1-{4-[(3-chloro-1H-pyrrolo[2,3-b]pyridin-4-yl)oxy]-3-(trifluoromethyl)phenyl}urea
• 1-{4-[(3-chloro-1H-pyrrolo[2,3-b]pyridin-4-yl)oxy]-3-(trifluoromethyl)phenyl}-3-pyridazin-3-ylurea
• 1-{4-[(3-chloro-1H-pyrrolo[2,3-b]pyridin-4-yl)oxy]-3-(trifluoromethyl)phenyl}-3-(2-methylpyrimidin-5-yl)urea
• 1-(3-methoxy-4-{[3-(trifluoromethyl)-1H-pyrrolo[2,3-b]pyridin-4-yl]oxy}phenyl)-3-[3-(morpholin-4-yl)propyl]urea
• 1-[3-(morpholin-4-yl)propyl]-3-[3-(trifluoromethoxy)-4-{[3-(trifluoromethyl)-1H-pyrrolo[2,3-b]pyridin-4-yl]oxy}phenyl]urea
• 1-[2-(morpholin-4-yl)ethyl]-3-[3-(trifluoromethoxy)-4-{[3-(trifluoromethyl)-1H-pyrrolo[2,3-b]pyridin-4-yl]oxy}phenyl]urea
and their polymorphs, enantiomers, diastereomers, racemates, tautomers, solvates, physiologically acceptable salts and solvates of these salts.

13. A compound of general formula (**I**) according to any one of claims 1 to 12 for the use as a medicament.

14. A compound of general formula (I) according to any one of claims 1 to 12 for use in the treatment or prophylaxis of a disease.

15. A pharmaceutical composition comprising a compound of general formula (I) according to any one of claims 1 to 12 and one or more pharmaceutically acceptable excipients.

16. A pharmaceutical combination comprising:
• one or more first active ingredients, in particular compounds of general formula (I) according to any one of claims 1 to 12, and
• one or more pharmaceutical active anti cancer compounds or
• one or more pharmaceutical active immune checkpoint inhibitors.

17. A pharmaceutical combination according to claim 16, **characterized in that** the pharmaceutical active immune checkpoint inhibitor is an antibody.

18. Use of a compound of general formula (I) according to any one of claims 1 to 12 for manufacturing a medicament for the treatment or prophylaxis of a disease.

19. Use of a compound of general formula (I) according to any one of claims 1 to 12 for the preparation of a medicament for the treatment or prophylaxis of a disease.

20. Use according to claim 18 or 19, wherein the disease is cancer.

21. Use according to claim 18 or 19, wherein the diseases, respectively the disorders are benign hyperplasias, atherosclerotic disorders, sepsis, autoimmune disorders, vascular disorders, viral infections, neurodegenerative disorders, in inflammatory disorders, and male fertility control.

## Patentansprüche

1. Verbindungen der Formel (I) 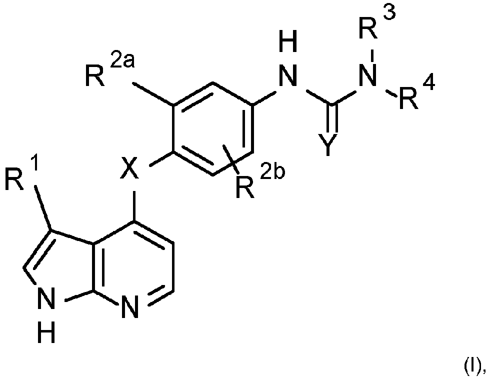 in der
X für ein Stickstoff-, ein Schwefel- oder ein Sauerstoffatom steht,
Y für ein Schwefel- oder ein Sauerstoffatom steht,
R¹ für Wasserstoff, Halogen, Cyano, C₁-C₆-Alkyl oder Halogen-C₁-C₆-alkyl steht,
R^{2a} für C₁-C₆-Alkoxy, Halogen-C₁-C₆-alkyl oder Halogen-C₁-C₆-alkoxy steht,
R^{2b} für Wasserstoff, Halogen, Cyano oder C₁-C₃-Alkyl steht,
R³ für Wasserstoff oder C₁-C₆-Alkyl steht,
R⁴ für Wasserstoff steht oder für C₁-C₆-Alkyl- steht,
das gegebenenfalls ein- oder mehrfach durch gleiche oder verschiedene Substituenten aus der Gruppe bestehend aus Halogen, Cyano, Hydroxy, C₁-C₃-Alkoxy-, -NH₂, -NH-(C₁-C₆-Alkyl), -N(C₁-C₆-Alkyl)₂, -NH-(C₁-C₆-Alkyl)-C₁-C₆-alkoxy, C₁-C₆-Alkyl-, C₁-C₆-Alkoxy-, -S(=O)₂NH₂, -C(=O)-R⁹, -C(=O)-NH-R^{x}, -NH-C(=O)-R⁹, -NH-S(=O)₂-R⁹, -S(=O)₂-R⁹, -S(=O)(=NR^{y})-R^{x}, -S(=O)-R^{x},
C₃-C₁₀-Cycloalkyl-, das selbst gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C (=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann, monocyclischem Heteroaryl- mit 5 oder 6 Ringatomen, das selbst gegebenenfalls ein- oder mehrfach durch gleiche oder verschiedene Substituenten aus der Gruppe bestehend aus Halogen, Amino, Hydroxy, Cyano, Nitro, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl- und einem monocyclischen Heterocyclylrest mit 3 bis 8 Ringatomen substituiert sein kann,
monocyclischem Heterocyclyl- mit 3 bis 8 Ringatomen, das selbst gegebenenfalls ein- oder mehrfach durch gleiche oder verschiedene Substituenten aus der Gruppe bestehend aus Halogen, Amino, Hydroxy, Cyano, Oxo, Carboxy, C₁-C₆-Alkyl-, -Carboxy-C₁-C₆-alkyl-, C₁-C₆-Alkoxy-, C₁-C₆-Alkoxy-C₁-C₆-alkyl-, C₁-C₆-Alkylamino-, Amino-C₁-C₆-alkyl-, C₁-C₆-Alkylamino-C₁-C₆-alkyl-, Hydroxy-C₁-C₆-alkyl-, Halogen-C₁-C₆-alkyl-, Halogen-C₁-C₆-alkoxy-, C₃-C₁₀-Cycloalkyl- und einem monocyclischen Heterocyclylrest mit 3 bis 8 Ringatomen substituiert sein kann, Phenyl-, das selbst gegebenenfalls ein- oder mehrfach durch gleiche oder verschiedene Substituenten aus der Gruppe bestehend aus Halogen, Amino, Hydroxy, Cyano, Nitro, Carboxy, C₁-C₆-Alkyl-, C₁-C₆-Alkoxy-, C₁-C₆-Alkoxy-C₁-C₆-alkyl-, C₁-C₆-Alkylamino-, Amino-C₁-C₆-alkyl-, C₁-C₆-Alkylaminocarbonyl, C₁-C₆-Alkylaminosulfonyl, C₁-C₆-Alkylamino-C₁-C₆-alkyl-, Hydroxy-C₁-C₆-alkyl-, Halogen-C₁-C₆-alkyl-, Halogen-C₁-C₆-alkoxy-, C₃-C₁₀-Cycloalkyl- und einem monocyclischen Heterocyclylrest mit 3 bis 8 Ringatomen substituiert sein kann,
Spirocycloalkyl-, Heterospirocycloalkyl-, Bicycloalkyl-, Heterobicycloalkyl-, verbrücktem Cycloalkyl oder verbrücktem Heterocycloalkyl, Naphthyl oder bicyclischem Heteroaryl oder teilgesättigtem bicyclischem Aryl- oder Heteroaryl, wobei jeder der oben genannten Reste gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C (=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann,
substituiert sein kann,
oder
R⁴ für C₃-C₁₀-Cycloalkyl- steht, das gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C (=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann,
oder
für monocyclisches Heteroaryl- mit 5 oder 6 Ringatomen steht, das gegebenenfalls ein- oder mehrfach durch gleiche oder verschiedene Substituenten aus der Gruppe bestehend aus Halogen, Cyano, Nitro, Hydroxy, Amino, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann, oder
für monocyclisches Heterocyclyl- mit 3 bis 8 Ringatomen steht, das gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C (=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann, oder
für Phenyl- steht, das gegebenenfalls ein- oder mehrfach durch gleiche oder verschiedene Substituenten aus der Gruppe bestehend aus Halogen, Cyano, Nitro, Hydroxy, Amino, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein kann, oder
für einen Spirocycloalkylrest, einen Heterospirocycloalkylrest, einen Bicycloalkylrest, einen Heterobicycloalkylrest, einen verbrückten Cycloalkylrest oder einen verbrückten Heterocycloalkylrest, einen Naphthylrest oder einen bicyclischen Heteroarylrest oder einen teilgesättigtem bicyclischen Aryl- oder Heteroarylrest steht, wobei die genannten Reste gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸ oder einem monocyclischen Heterocyclylrest mit 3 bis 8 Ringatomen substituiert sein können,
oder
R³ und R⁴ zusammen mit dem Stickstoffatom einen 4-bis 10-gliedrigen Heterocycloalkylring bilden, der gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸ oder einem monocyclischen Heterocyclylrest mit 3 bis 8 Ringatomen substituiert sein kann,
R⁵ und R⁶ unabhängig voneinander für Wasserstoff, C₁-C₃-Alkyl-, Cyclopropyl- oder Di-C₁-C₃-alkylamino-C₁-C₃-Alkyl- stehen,
R⁷ für Hydroxy, C₁-C₆-Alkyl-, C₁-C₆-Alkoxy-, Halogen-C₁-C₃-alkyl-, Hydroxy-C₁-C₃-alkyl-, C₁-C₃-Alkoxy-C₁-C₃-alkyl-, C₃-C₈-Cycloalkyl-, Phenyl-, monocyclisches Heterocyclyl- mit 3 bis 8 Ringatomen oder monocyclisches Heteroaryl- mit 5 oder 6 Ringatomen steht, wobei Phenyl-, Heteroaryl- und Heterocyclyl gegebenenfalls ein- oder mehrfach durch Halogen, C₁-C₃-Alkoxy- oder C₁-C₃-Alkyl-substituiert sein können,
R⁸ für C₁-C₆-Alkyl-steht,
R⁹ für C₁-C₆-Alkyl-, -NH₂, -NH-(C₁-C₆-Alkyl), -N(C₁-C₆-Alkyl)₂ oder C₁-C₆-Alkoxy-C₁-C₆-alkyl- steht
oder
für monocyclisches Heterocyclyl- mit 3 bis 8 Ringatomen steht, das selbst gegebenenfalls ein- oder mehrfach durch gleiche oder verschiedene Substituenten aus der Gruppe bestehend aus Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl-, Phenyl-, Halogenphenyl-, Phenyl-C₁-C₆-alkyl-, Phenoxy-, Pyridinyl-, -C (=O)-NR⁵R⁶, -NH-C (=O)-R⁸, -C (=O)-R⁷, -S (=O)₂-NR⁵R⁶, -S (=O)-R⁸, -S (=O)(=NR⁵)-R⁶, -S (=O)₂-R⁸, -NH-S (=O)₂-R⁸ oder einem monocyclischen Heterocyclyl-rest mit 3 bis 8 Ringatomen substituiert sein kann,
R^{x} für C₁-C₆-Alkyl- oder C₁-C₆-Alkoxy-C₁-C₆-alkyl- steht,
R^{y} für Wasserstoff, Halogen-C₁-C₆-alkyl-, C₁-C₆-Alkyl oder durch C₃-C₆-Cycloalkyl substituiertes C₁-C₆-Alkyl steht,
sowie deren Polymorphe, Enantiomere, Diastereomere, Racemate, Tautomere, Solvate, physiologisch verträglichen Salze und Solvate dieser Salze.

2. Verbindung der allgemeinen Formel (I) nach Anspruch 1, in der
X für ein Stickstoff-, ein Schwefel- oder ein Sauerstoffatom steht,
Y für ein Schwefel- oder ein Sauerstoffatom steht,
R¹ für Wasserstoff, Halogen, Cyano, C₁-C₃-Alkyl oder Halogen-C₁-C₃-alkyl steht,
R^{2a} für C₁-C₃-Alkoxy, Halogen-C₁-C₃-alkyl oder Halogen-C₁-C₃-alkoxy steht,
R^{2b} für Wasserstoff, Halogen, Cyano oder C₁-C₃-Alkyl steht,
R³ für Wasserstoff steht,
R⁴ für Wasserstoff steht oder für C₁-C₆-Alkyl- steht,
das gegebenenfalls ein- oder mehrfach durch gleiche oder verschiedene Substituenten aus der Gruppe bestehend aus Halogen, Cyano, Hydroxy, C₁-C₃-Alkoxy-, -NH₂, -NH-(C₁-C₆-Alkyl), -N(C₁-C₆-Alkyl)₂, -NH- (C₁-C₆-Alkyl) -C₁-C₆-alkoxy, C₁-C₆-Alkyl-, C₁-C₆-Alkoxy-,
monocyclischem Heterocyclyl- mit 3 bis 8 Ringatomen, das selbst gegebenenfalls ein- oder mehrfach durch gleiche oder verschiedene Substituenten aus der Gruppe bestehend aus Halogen, Amino, Hydroxy, Cyano, Oxo, Carboxy, C₁-C₆-Alkyl-, -Carboxy-C₁-C₆-alkyl-, C₁-C₆-Alkoxy-, C₁-C₆-Alkoxy-C₁-C₆-alkyl-, C₁-C₆-Alkylamino-, Amino-C₁-C₆-alkyl-, C₁-C₆-Alkylamino-C₁-C₆-alkyl-, Hydroxy-C₁-C₆-alkyl-, Halogen-C₁-C₆-alkyl-, Halogen-C₁-C₆-alkoxy-, C₃-C₁₀-Cycloalkyl- und einem monocyclischen Heterocyclylrest mit 3 bis 8 Ringatomen substituiert sein kann, substituiert sein kann, oder
R⁴ für ein monocyclisches Heteroaryl- mit 5 oder 6 Ringatomen steht, das gegebenenfalls ein- oder mehrfach durch gleiche oder verschiedene Substituenten aus der Gruppe bestehend aus Halogen, Cyano, Nitro, Hydroxy, Amino, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl- substituiert sein kann,
sowie deren Polymorphe, Enantiomere, Diastereomere, Racemate, Tautomere, Solvate, physiologisch verträglichen Salze und Solvate dieser Salze.

3. Verbindung der allgemeinen Formel (I) nach Anspruch 1 oder 2, in der
X für ein Stickstoff-, ein Schwefel- oder ein Sauerstoffatom steht,
Y für ein Schwefel- oder ein Sauerstoffatom steht,
R¹ für Halogen oder Perfluor-C₁-C₃-alkyl steht,
R^{2a} für C₁-C₃-Alkoxy, Perfluor-C₁-C₃-alkyl oder Perfluor-C₁-C₃-alkoxy steht,
R^{2b} für Wasserstoff oder Halogen steht,
R³ für Wasserstoff oder C₁-C₆-Alkyl steht,
R⁴ für Wasserstoff steht oder für C₁-C₆-Alkyl- steht,
das gegebenenfalls ein- oder mehrfach durch gleiche oder verschiedene Substituenten aus der Gruppe bestehend aus Halogen, Cyano, Hydroxy, C₁-C₃-Alkoxy-, -NH₂, -NH-(C₁-C₆-Alkyl), -N(C₁-C₆-Alkyl)₂, -NH- (C₁-C₆-Alkyl) -C₁-C₆-alkoxy, C₁-C₆-Alkyl-, C₁-C₆-Alkoxy-,
monocyclischem Heterocyclyl- mit 3 bis 8 Ringatomen, das selbst gegebenenfalls ein- oder mehrfach durch gleiche oder verschiedene Substituenten aus der Gruppe bestehend aus Halogen, Amino, Hydroxy, Cyano, Oxo, Carboxy, C₁-C₆-Alkyl-, -Carboxy-C₁-C₆-alkyl-, C₁-C₆-Alkoxy-, C₁-C₆-Alkoxy-C₁-C₆-alkyl-, C₁-C₆-Alkylamino-, Amino-C₁-C₆-alkyl-, C₁-C₆-Alkylamino-C₁-C₆-alkyl-, Hydroxy-C₁-C₆-alkyl-, Halogen-C₁-C₆-alkyl-, Halogen-C₁-C₆-alkoxy-, C₃-C₁₀-Cycloalkyl- und einem monocyclischen Heterocyclylrest mit 3 bis 8 Ringatomen substituiert sein kann, substituiert sein kann,
oder
R⁴ für ein monocyclisches Heteroaryl- mit 6 Ringatomen steht, das gegebenenfalls ein- oder mehrfach durch gleiche oder verschiedene Substituenten aus der Gruppe bestehend aus Halogen, Cyano, Nitro, Hydroxy, Amino, Carboxy, C₁-C₆-Alkyl-, C₁-C₆-Alkoxy-, C₁-C₆-Alkoxy-C₁-C₆-alkyl-, Hydroxy-C₁-C₆-alkyl-, C₁-C₆-Alkylamino-, C₁-C₆-Alkylcarbonylamino-, Amino-C₁-C₆-alkyl-, C₁-C₆-Alkylamino-C₁-C₆-alkyl-, Halogen-C₁-C₆-alkyl-, Halogen-C₁-C₆-alkoxy-, C₃-C₁₀-Cycloalkyl- substituiert sein kann,
sowie deren Polymorphe, Enantiomere, Diastereomere, Racemate, Tautomere, Solvate, physiologisch verträglichen Salze und Solvate dieser Salze.

4. Verbindung der allgemeinen Formel (I) nach einem der Ansprüche 1 bis 3, in der
X für ein Sauerstoffatom steht,
Y für ein Sauerstoffatom steht,
R¹ für Chlor oder Trifluormethyl steht,
R^{2a} für Methoxy, Trifluormethyl oder Trifluormethoxy steht,
R^{2b} für Wasserstoff oder Fluor steht,
R³ für Wasserstoff steht,
R⁴ für Wasserstoff steht oder für C₂- oder C₃-Alkyl- steht,
das durch 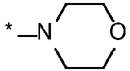 substituiert ist,
oder
R⁴ für ein Pyrimidinyl oder Pyridazinyl steht, das gegebenenfalls ein- oder mehrfach durch C₁-C₆-Alkyl- substituiert sein kann,
sowie deren Polymorphe, Enantiomere, Diastereomere, Racemate, Tautomere, Solvate, physiologisch verträglichen Salze und Solvate dieser Salze.

5. Verbindung der allgemeinen Formel (I) nach einem der Ansprüche 1 bis 3, in der
X für ein Sauerstoffatom steht,
sowie deren Polymorphe, Enantiomere, Diastereomere, Racemate, Tautomere, Solvate, physiologisch verträglichen Salze und Solvate dieser Salze.

6. Verbindung der allgemeinen Formel (I) nach einem der Ansprüche 1 bis 3, in der
Y für ein Sauerstoffatom steht,
sowie deren Polymorphe, Enantiomere, Diastereomere, Racemate, Tautomere, Solvate, physiologisch verträglichen Salze und Solvate dieser Salze.

7. Verbindung der allgemeinen Formel (I) nach einem der Ansprüche 1 bis 3, in der
R¹ für Chlor oder Trifluormethyl steht,
sowie deren Polymorphe, Enantiomere, Diastereomere, Racemate, Tautomere, Solvate, physiologisch verträglichen Salze und Solvate dieser Salze.

8. Verbindung der allgemeinen Formel (I) nach einem der Ansprüche 1 bis 3, in der
R^{2a} für Methoxy, Trifluormethyl oder Trifluormethoxy steht,
sowie deren Polymorphe, Enantiomere, Diastereomere, Racemate, Tautomere, Solvate, physiologisch verträglichen Salze und Solvate dieser Salze.

9. Verbindung der allgemeinen Formel (I) nach einem der Ansprüche 1 bis 3, in der
R^{2b} für Wasserstoff oder Fluor steht,
sowie deren Polymorphe, Enantiomere, Diastereomere, Racemate, Tautomere, Solvate, physiologisch verträglichen Salze und Solvate dieser Salze.

10. Verbindung der allgemeinen Formel (I) nach einem der Ansprüche 1 bis 3, in der
R³ für Wasserstoff steht,
sowie deren Polymorphe, Enantiomere, Diastereomere, Racemate, Tautomere, Solvate, physiologisch verträglichen Salze und Solvate dieser Salze.

11. Verbindung der allgemeinen Formel (I) nach einem der Ansprüche 1 bis 3, in der
R⁴ für Wasserstoff steht oder für C₂- oder C₃-Alkyl- steht,
das durch 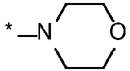 substituiert ist,
oder
R⁴ für ein Pyrimidinyl oder Pyridazinyl steht, das gegebenenfalls ein- oder mehrfach durch C₁-C₆-Alkyl- substituiert sein kann,
sowie deren Polymorphe, Enantiomere, Diastereomere, Racemate, Tautomere, Solvate, physiologisch verträglichen Salze und Solvate dieser Salze.

12. Verbindung nach Anspruch 1, die ausgewählt ist aus
• 1-[2-(Morpholin-4-yl)ethyl]-3-[3-(trifluormethyl)-4-{[3-(trifluormethyl)-1H-pyrrolo[2,3-b]pyridin-4-yl]oxy}phenyl]harnstoff
• 1-[3-(Morpholin-4-yl)propyl]-3-[3-(trifluormethyl)-4-{[3-(trifluormethyl)-1H-pyrrolo[2,3-b]pyridin-4-yl]oxy}phenyl]harnstoff
• 1-[3-(Trifluormethyl)-4-{[3-(trifluormethyl)-1H-pyrrolo[2,3-b]pyridin-4-yl]oxy}phenyl]harnstoff
• 1-[3-Fluor-5-(trifluormethyl)-4-{[3-(trifluormethyl)-1H-pyrrolo[2,3-b]pyridin-4-yl]oxy}phenyl]-3-[3-(morpholin-4-yl)propyl]harnstoff
• 1-[3-Fluor-5-(trifluormethyl)-4-{[3-(trifluormethyl)-1H-pyrrolo[2,3-b]pyridin-4-yl]oxy}phenyl]-3-[2-(morpholin-4-yl)ethyl]harnstoff
• 1-{4-[(3-Chlor-1H-pyrrolo[2,3-b]pyridin-4-yl)oxy]-3-(trifluormethyl)phenyl}-3-[3-(morpholin-4-yl)propyl]harnstoff
• 1-{4-[(3-Chlor-1H-pyrrolo[2,3-b]pyridin-4-yl)oxy]-3-(trifluormethyl)phenyl}-3-[2-(morpholin-4-yl)ethyl]harnstoff
• 1-14-[(3-Chlor-1H-pyrrolo[2,3-b]pyridin-4-yl)oxy]-3-(trifluormethyl)phenyl}harnstoff
• 1-{4-[(3-Chlor-1H-pyrrolo[2,3-b]pyridin-4-yl)oxy]-3-(trifluormethyl)phenyl}-3-pyridazin-3-ylharnstoff
• 1-{4-[(3-Chlor-1H-pyrrolo[2,3-b]pyridin-4-yl)oxy]-3-(trifluormethyl)phenyl}-3-(2-methylpyrimidin-5-yl)harnstoff
• 1-(3-Methoxy-4-{[3-(trifluormethyl)-1H-pyrrolo[2,3-b]pyridin-4-yl]oxy}phenyl)-3-[3-(morpholin-4-yl)propyl]harnstoff
• 1-[3-(Morpholin-4-yl)propyl]-3-[3-(trifluormethoxy)-4-{[3-(trifluormethyl)-1H-pyrrolo[2,3-b]pyridin-4-yl]oxy}phenyl]harnstoff
• 1-[2-(Morpholin-4-yl)ethyl]-3-[3-(trifluormethoxy)-4-{[3-(trifluormethyl)-1H-pyrrolo[2,3b]pyridin-4-yl]oxy}phenyl]harnstoff
sowie deren Polymorphe, Enantiomere, Diastereomere, Racemate, Tautomere, Solvate, physiologisch verträglichen Salze und Solvate dieser Salze.

13. Verbindung der allgemeinen Formel (I) nach einem der Ansprüche 1 bis 12 zur Verwendung als Medikament.

14. Verbindung der allgemeinen Formel (I) nach einem der Ansprüche 1 bis 12 zur Verwendung bei der Behandlung oder Prophylaxe einer Krankheit.

15. Pharmazeutische Zusammensetzung, umfassend eine Verbindung der allgemeinen Formel (I) nach einem der Ansprüche 1 bis 12 und einen oder mehrere pharmazeutisch unbedenkliche Hilfsstoffe.

16. Pharmazeutische Kombination, umfassend:
• einen oder mehrere erste Wirkstoffe, insbesondere Verbindungen der allgemeinen Formel (I) nach einem der Ansprüche 1 bis 12, und
• eine oder mehrere pharmazeutisch wirksame Antikrebsverbindungen oder
• eine oder mehrere pharmazeutisch wirksame Immun-Checkpoint-Inhibitoren.

17. Pharmazeutische Kombination nach Anspruch 16, **dadurch gekennzeichnet, dass** es sich bei dem pharmazeutisch wirksamen Immun-Checkpoint-Inhibitor um einen Antikörper handelt.

18. Verwendung einer Verbindung der allgemeinen Formel (I) nach einem der Ansprüche 1 bis 12 zur Anfertigung eines Medikaments zur Behandlung oder Prophylaxe einer Krankheit.

19. Verwendung einer Verbindung der allgemeinen Formel (I) nach einem der Ansprüche 1 bis 12 zur Herstellung eines Medikaments zur Behandlung oder Prophylaxe einer Krankheit.

20. Verwendung nach Anspruch 18 oder 19, wobei es sich bei der Krankheit um Krebs handelt.

21. Verwendung nach Anspruch 18 oder 19, wobei es sich bei den Krankheiten bzw. den Erkrankungen um benigne Hyperplasien, atherosklerotische Erkrankungen, Sepsis, Autoimmunerkrankungen, Gefäßerkrankungen, Virusinfektionen, neurodegenerative Erkrankungen, inflammatorische Erkrankungen und männliche Fertilitätskontrolle handelt.

## Revendications

1. Composés de formule (I) 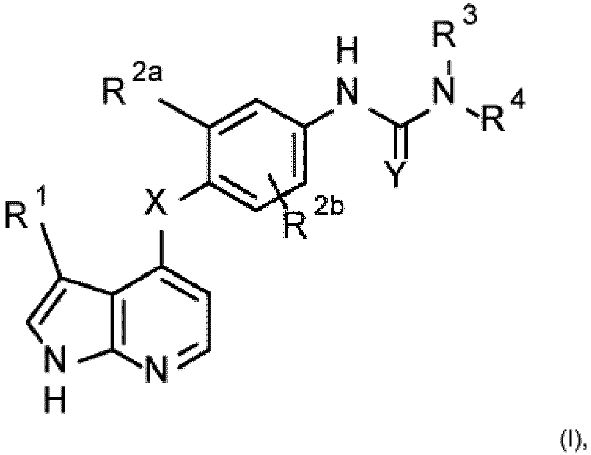 dans laquelle
X représente un atome d'azote, un atome de soufre ou un atome d'oxygène,
Y représente un atome de soufre ou un atome d'oxygène,
R¹ représente hydrogène, halogène, cyano, C₁-C₆-alkyle ou halogéno-C₁-C₆-alkyle,
R^{2a} représente C₁-C₆-alcoxy, halogéno-C₁-C₆-alkyle ou halogéno-C₁-C₆-alcoxy,
R^{2b} représente hydrogène, halogène, cyano ou C₁-C₃-alkyle,
R³ représente hydrogène ou C₁-C₆-alkyle,
R⁴ représente hydrogène ou représente C₁-C₆-alkyle-, qui peut éventuellement être monosubstitué ou polysubstitué par des substituants identiques ou différents du groupe constitué par halogène, cyano, hydroxy, C₁-C₃-alcoxy-, -NH₂, -NH-(C₁-C₆-alkyl), -N(C₁-C₆-alkyl)₂, -NH-(C₁-C₆-alkyl)-C₁-C₆-alcoxy, C₁-C₆-alkyle-, C₁-C₆-alcoxy-, -S(=O)₂NH₂, -C(=O)-R⁹, -C(=O)-NH-R^{x}, -NH-C(=O)-R⁹, -NH-S(=O)₂-R⁹, -S(=O)₂-R⁹, -S(=O)(=NR^{y})-R^{x}, -S(=O)-R^{x}, C₃-C₁₀-cycloalkyle- qui lui-même peut éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-,-C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸,-NH-S(=O)₂-R⁸, ou un radical hétérocyclyle monocyclique possédant 3 à 8 atomes de cycle, hétéroaryle monocyclique possédant 5 ou 6 atomes de cycle qui lui-même peut éventuellement être monosubstitué ou polysubstitué par des substituants identiques ou différents du groupe constitué par halogène, amino, hydroxy, cyano, nitro, carboxy, C₁-C₆-alkyle-, C₁-C₆-alcoxy-, C1-C₆-alcoxy-C₁-C₆-alkyle-, hydroxy-C₁-C₆-alkyle-, C₁-C₆-alkylamino-, amino-C₁-C₆-alkyle-, C₁-C₆-alkylamino-C₁-C₆-alkyle, halogéno-C₁-C₆-alkyle-, halogéno-C₁-C₆-alcoxy-, C₃-C₁₀-cycloalkyle et un radical hétérocyclyle monocyclique possédant 3 à 8 atomes de cycle,
hétérocyclyle monocyclique possédant 3 à 8 atomes de cycle qui lui-même peut éventuellement être monosubstitué ou polysubstitué par des substituants identiques ou différents du groupe constitué par halogène, amino, hydroxy, cyano, oxo, carboxy, C₁-C₆-alkyle-, -carboxy-C₁-C₆-alkyle-, C₁-C₆-alcoxy-, C₁-C₆-alcoxy-C₁-C₆-alkyle-, C₁-C₆-alkylamino-, amino-C₁-C₆-alkyle-, C₁-C₆-alkylamino-C₁-C₆-alkyle-, hydroxy-C₁-C₆-alkyle-, halogéno-C₁-C₆-alkyle-, halogéno-C₁-C₆-alcoxy-, C₃-C₁₀-cycloalkyle- et un radical hétérocyclyle monocyclique possédant 3 à 8 atomes de cycle, phényle- qui lui-même peut éventuellement être monosubstitué ou polysubstitué par des substituants identiques ou différents du groupe constitué par halogène, amino, hydroxy, cyano, nitro, carboxy, C₁-C₆-alkyle-, C₁-C₆-alcoxy-, C₁-C₆-alcoxy-C₁-C₆-alkyle-, C₁-C₆-alkyl-amino-, amino-C₁-C₆-alkyle-, C₁-C₆-alkylaminocarbonyle-, C₁-C₆-alkyl-aminosulphonyle-, C₁-C₆-alkylamino-C₁-C₆-alkyle-, hydroxy-C₁-C₆-alkyle-, halogéno-C₁-C₆-alkyle-, halogéno-C₁-C₆-alcoxy-, C₃-C₁₀-cycloalkyle- et un radical hétérocyclyle monocyclique possédant 3 à 8 atomes de cycle, spirocycloalkyle-, hétérospirocycloalkyle-, bicycloalkyle-, hétérobicycloalkyle-, cycloalkyle ponté ou un hétérocycloalkyle ponté, naphtyle ou hétéroaryle bicyclique, ou aryle- ou hétéroaryle bicyclique partiellement insaturé,
chacun de ceux mentionnés ci-dessus pouvant éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-,-C(=O)-NR⁵R⁶, -NH-C (=O)-R⁸, -C(=O)-R⁷, -S(=O)₂-NR⁵R⁶, -S(=O)-R⁸, -S(=O)(=NR⁵)-R⁶, -S(=O)₂-R⁸,-NH-S(=O)₂-R⁸, ou un radical hétérocyclyle monocyclique possédant 3 à 8 atomes de cycle,
ou
R⁴ représente C₃-C₁₀-cycloalkyle- qui peut éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-,-C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸,-NH-S(=O)₂-R⁸, ou un radical hétérocyclyle monocyclique possédant 3 à 8 atomes de cycle, ou
représente hétéroaryle monocyclique possédant 5 ou 6 atomes de cycle qui peut éventuellement être monosubstitué ou polysubstitué par des substituants identiques ou différents du groupe constitué par halogène, cyano, nitro, hydroxy, amino, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-, -C(=O)-NR⁵R⁶, -NH-C (=O)-R⁸, -C(=O)-R⁷, -S(=O)₂-NR⁵R⁶, -S(=O)-R⁸,-S(=O)(=NR⁵)-R⁶, -S(=O)₂-R⁸, -NH-S (=O)₂-R⁸, ou un radical hétérocyclyle monocyclique possédant 3 à 8 atomes de cycle,
ou
représente hétérocyclyle monocyclique possédant 3 à 8 atomes de cycle qui peut éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-, -C(=O)-NR⁵R⁶,-NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸, ou un radical hétérocyclyle monocyclique possédant 3 à 8 atomes de cycle,
ou
représente phényl- qui peut éventuellement être monosubstitué ou polysubstitué par des substituants identiques ou différents du groupe constitué par halogène, cyano, nitro, hydroxy, amino, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-, -C(=O)-NR⁵R⁶, -NH-C (=O)-R⁸, -C(=O)-R⁷, -S(=O)₂-NR⁵R⁶, -S(=O)-R⁸,-S(=O)(=NR⁵)-R⁶, -S(=O)₂-R⁸, -NH-S (=O)₂-R⁸, ou un radical hétérocyclyle monocyclique possédant 3 à 8 atomes de cycle,
ou
représente un radical spirocycloalkyle, un radical hétérospirocycloalkyle, un bicycloalkyle, un radical hétérobicycloalkyle, un radical cycloalkyle ponté ou un radical hétérocycloalkyle ponté, un radical naphtyle ou un radical hétéroaryle bicyclique, ou un radical aryle- ou hétéroaryle bicyclique partiellement insaturé, où les radicaux mentionnés peuvent éventuellement être monosubstitués ou polysubstitués par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-, -C(=O)-NR⁵R⁶,-NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S (=O)₂-R⁸, ou un radical hétérocyclyle monocyclique possédant 3 à 8 atomes de cycle,
ou
R³ et R⁴ conjointement avec l'atome d'azote forment un cycle hétérocycloalkyle à 4 à 10 chaînons, qui peut éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-, -C(=O)-NR⁵R⁶,-NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸, -NH-S(=O)₂-R⁸, ou un radical hétérocyclyle monocyclique possédant 3 à 8 atomes de cycle,
R⁵ et R⁶ représentent indépendamment l'un de l'autre hydrogène, C₁-C₃-alkyle-, cyclopropyle- ou di-C₁-C₃-alkylamino-C₁-C₃-alkyle-,
R⁷ représente hydroxy, C₁-C₆-alkyle-, C₁-C₆-alcoxy-, halogéno-C₁-C₃-alkyle-, hydroxy-C₁-C₃-alkyle-, C₁-C₃-alcoxy-C₁-C₃-alkyle-, C₃-C₈-cycloalkyle-, phényle-, hétérocyclyle monocyclique possédant 3 à 8 atomes de cycle ou hétéroaryle monocyclique possédant 5 ou 6 atomes de cycle où phényle-, hétéroaryle- et hétérocyclyle-peuvent éventuellement être monosubstitués ou disubstitués par halogène, C₁-C₃-alcoxy- ou C₁-C₃-alkyle- ,
R⁸ représente C₁-C₆-alkyle-,
R⁹ représente C₁-C₆-alkyle-, -NH₂, -NH-C₁-C₆-alkyle, -N(C₁-C₆-alkyl)₂, ou C₁-C₆-alcoxy-C₁-C₆-alkyle-,
ou
représente hétérocyclyle monocyclique possédant 3 à 8 atomes de cycle qui lui-même peut éventuellement être monosubstitué ou polysubstitué par des substituants identiques ou différents du groupe constitué par halogène, cyano, nitro, hydroxy, amino, oxo, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, phényle-, halogénophényle-, phényl-C₁-C₆-alkyle-, phénoxy-, pyridinyle-,-C(=O)-NR⁵R⁶, -NH-C(=O)-R⁸, -C(=O)-R⁷, -S(=O)₂-NR⁵R⁶, -S(=O)-R⁸, -S(=O)(=NR⁵)-R⁶, -S(=O)₂-R⁸,-NH-S(=O)₂-R⁸, ou un radical hétérocyclyle monocyclique possédant 3 à 8 atomes de cycle,
R^{x} représente C₁-C₆-alkyle- ou C₁-C₆-alcoxy-C₁-C₆-alkyle-,
R^{y} représente hydrogène, halogéno-C₁-C₆-alkyle, C₁-C₆-alkyle, C₁-C₆-alkyle substitué par C₃-C₆-cycloalkyle,
et leurs sels physiologiquement acceptables, et solvates de ces sels, et polymorphes, énantiomères, diastéréoisomères, racémiques, formes tautomères et solvates.

2. Composé de formule générale (I) selon la revendication 1,
X représentant un atome d'azote, un atome de soufre ou un atome d'oxygène,
Y représentant un atome de soufre ou un atome d'oxygène,
R¹ représentant hydrogène, halogène, cyano, C₁-C₃-alkyle ou halogéno-C₁-C₃-alkyle,
R^{2a} représentant C₁-C₃-alcoxy, halogéno-C₁-C₃-alkyle ou halogéno-C₁-C₃-alcoxy,
R^{2b} représentant hydrogène, halogène, cyano ou C₁-C₃-alkyle,
R³ représentant hydrogène,
R⁴ représentant hydrogène ou représentant C₁-C₆-alkyle-, qui peut éventuellement être monosubstitué ou polysubstitué par des substituants identiques ou différents du groupe constitué par halogène, cyano, hydroxy, C₁-C₃-alcoxy-, -NH₂, -NH-(C₁-C₆-alkyl), -N(C₁-C₆-alkyl)₂, -NH-(C₁-C₆-alkyl)-C₁-C₆-alcoxy, C₁-C₆-alkyle-, C₁-C₆-alcoxy-,
hétérocyclyle monocyclique possédant 3 à 8 atomes de cycle qui lui-même peut éventuellement être monosubstitué ou polysubstitué par des substituants identiques ou différents du groupe constitué par halogène, amino, hydroxy, cyano, oxo, carboxy, C₁-C₆-alkyle-, -carboxy-C₁-C₆-alkyle-, C₁-C₆-alcoxy-, C₁-C₆-alcoxy-C₁-C₆-alkyle-, C₁-C₆-alkylamino-, amino-C₁-C₆-alkyle-, C₁-C₆-alkylamino-C₁-C₆-alkyle-, hydroxy-C₁-C₆-alkyle-, halogéno-C₁-C₆-alkyle-, halogéno-C₁-C₆-alcoxy-, C₃-C₁₀-cycloalkyle- et un radical hétérocyclyle monocyclique possédant 3 à 8 atomes de cycle, ou
R⁴ représentant un hétéroaryle monocyclique possédant 5 ou 6 atomes de cycle qui peut éventuellement être monosubstitué ou polysubstitué par des substituants identiques ou différents du groupe constitué par halogène, cyano, nitro, hydroxy, amino, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-, et leurs sels physiologiquement acceptables, et solvates de ces sels, et polymorphes, énantiomères, diastéréoisomères, racémiques, formes tautomères et solvates.

3. Composé de formule générale (I) selon la revendication 1 ou 2,
X représentant un atome d'azote, un atome de soufre ou un atome d'oxygène,
Y représentant un atome de soufre ou un atome d'oxygène,
R¹ représentant halogène ou perfluoro-C₁-C₃-alkyle,
R^{2a} représentant C₁-C₃-alcoxy, perfluoro-C₁-C₃-alkyle ou perfluoro-C₁-C₃-alcoxy,
R^{2b} représentant hydrogène ou halogène,
R³ représentant hydrogène ou C₁-C₆-alkyle,
R⁴ représentant hydrogène ou représentant C₁-C₆-alkyle-,
qui peut éventuellement être monosubstitué ou polysubstitué par des substituants identiques ou différents du groupe constitué par halogène, cyano, hydroxy, C₁-C₃-alcoxy-, -NH₂, -NH-(C₁-C₆-alkyl), -N(C₁-C₆-alkyl)₂, -NH-(C₁-C₆-alkyl)-C₁-C₆-alcoxy, C₁-C₆-alkyle-, C₁-C₆-alcoxy hétérocyclyle monocyclique possédant 3 à 8 atomes de cycle qui lui-même peut éventuellement être monosubstitué ou polysubstitué par des substituants identiques ou différents du groupe constitué par halogène, amino, hydroxy, cyano, oxo, carboxy, C₁-C₆-alkyle-, -carboxy-C₁-C₆-alkyle-, C₁-C₆-alcoxy-, C₁-C₆-alcoxy-C₁-C₆-alkyle-, C₁-C₆-alkylamino-, amino-C₁-C₆-alkyle-, C₁-C₆-alkylamino-C₁-C₆-alkyle-, hydroxy-C₁-C₆-alkyle-, halogéno-C₁-C₆-alkyle-, halogéno-C₁-C₆-alcoxy-, C₃-C₁₀-cycloalkyle- et un radical hétérocyclyle monocyclique possédant 3 à 8 atomes de cycle, ou
R⁴ représentant un hétéroaryle monocyclique possédant 6 atomes de cycle qui peut éventuellement être monosubstitué ou polysubstitué par des substituants identiques ou différents du groupe constitué par halogène, cyano, nitro, hydroxy, amino, carboxy, C₁-C₆-alkyle-, C₁-C₆-alcoxy-, C₁-C₆-alcoxy-C₁-C₆-alkyle-, hydroxy-C₁-C₆-alkyle-, C₁-C₆-alkylamino-, C₁-C₆-alkylcarbonylamino-, amino-C₁-C₆-alkyle-, C₁-C₆-alkylamino-C₁-C₆-alkyle-, halogéno-C₁-C₆-alkyle-, halogéno-C₁-C₆-alcoxy-, C₃-C₁₀-cycloalkyle-,
et leurs sels physiologiquement acceptables, et solvates de ces sels, et polymorphes, énantiomères, diastéréoisomères, racémiques, formes tautomères et solvates.

4. Composé de formule générale (I) selon l'une quelconque des revendications 1 à 3,
X représentant un atome d'oxygène,
Y représentant un atome d'oxygène,
R¹ représentant chlore ou trifluorométhyle,
R^{2a} représentant méthoxy, trifluorométhyle ou trifluorométhoxy,
R^{2b} représentant hydrogène ou fluorine,
R³ représentant hydrogène,
R⁴ représentant hydrogène ou représentant C₂- ou C₃-alkyle-,
qui est substitué par 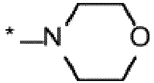 ou
R⁴ représentant un pyrimidinyle ou pyridazinyle qui peut éventuellement être monosubstitué ou polysubstitué par C₁-C₆-alkyle-,
et leurs sels physiologiquement acceptables, et solvates de ces sels, et polymorphes, énantiomères, diastéréoisomères, racémiques, formes tautomères et solvates.

5. Composé de formule générale (I) selon l'une quelconque des revendications 1 à 3,
X représentant un atome d'oxygène,
et leurs sels physiologiquement acceptables, et
solvates de ces sels, et polymorphes, énantiomères, diastéréoisomères, racémiques, formes tautomères et solvates.

6. Composé de formule générale (I) selon l'une quelconque des revendications 1 à 3,
Y représentant un atome d'oxygène,
et leurs sels physiologiquement acceptables, et solvates de ces sels, et polymorphes, énantiomères, diastéréoisomères, racémiques, formes tautomères et solvates.

7. Composé de formule générale (I) selon l'une quelconque des revendications 1 à 3,
R¹ représentant chlore ou trifluorométhyle,
et leurs sels physiologiquement acceptables, et solvates de ces sels, et polymorphes, énantiomères, diastéréoisomères, racémiques, formes tautomères et solvates.

8. Composé de formule générale (I) selon l'une quelconque des revendications 1 à 3,
R^{2a} représentant méthoxy, trifluorométhyle ou trifluorométhoxy,
et leurs sels physiologiquement acceptables, et solvates de ces sels, et polymorphes, énantiomères, diastéréoisomères, racémiques, formes tautomères et solvates.

9. Composé de formule générale (I) selon l'une quelconque des revendications 1 à 3,
R^{2b} représentant hydrogène ou fluor,
et leurs sels physiologiquement acceptables, et solvates de ces sels, et polymorphes, énantiomères, diastéréoisomères, racémiques, formes tautomères et solvates.

10. Composé de formule générale (I) selon l'une quelconque des revendications 1 à 3,
R³ représentant hydrogène,
et leurs sels physiologiquement acceptables, et solvates de ces sels, et polymorphes, énantiomères, diastéréoisomères, racémiques, formes tautomères et solvates.

11. Composé de formule générale (I) selon l'une quelconque des revendications 1 à 3,
R⁴ représentant hydrogène ou représentant C₂-alkyle- ou C₃₋alkyle-, qui est substitué par 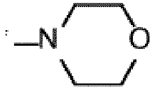 ou
R⁴ représentant un pyrimidinyle ou pyridazinyle qui peut éventuellement être monosubstitué ou polysubstitué par C₁₋₆₋alkyle-,
et leurs sels physiologiquement acceptables, et solvates de ces sels, et polymorphes, énantiomères, diastéréoisomères, racémiques, formes tautomères et solvates.

12. Composé selon la revendication 1, qui est choisi parmi
• 1-[2-(morpholin-4-yl)éthyl]-3-[3-(trifluorométhyl)-4-{[3-(trifluorométhyl)-1H-pyrrolo[2,3-b]pyridin-4-yl]oxy}phényl]urée
• 1-[3-(morpholin-4-yl)propyl]-3-[3-(trifluorométhyl)-4-{[3-(trifluorométhyl)-1H-pyrrolo[2,3-b]pyridin-4-yl]oxy}phényl]urée
• 1-[3-(trifluorométhyl)-4-{[3-(trifluorométhyl)-1H-pyrrolo[2,3-b]pyridin-4-yl]oxy}phényl]urée
• 1-[3-fluoro-5-(trifluorométhyl)-4-{[3-(trifluorométhyl)-1H-pyrrolo[2,3-b]pyridin-4-yl]oxy}phényl]-3-[3-(morpholin-4-yl)propyl]urée
• 1-[3-fluoro-5-(trifluorométhyl)-4-{[3-(trifluorométhyl)-1H-pyrrolo[2,3-b]pyridin-4-yl]oxy}phényl]-3-[2-(morpholin-4-yl)éthyl]urée
• 1-{4-[(3-chloro-1H-pyrrolo[2,3-b]pyridin-4-yl)oxy]-3-(trifluorométhyl)phényl}-3-[3-(morpholin-4-yl)propyl]urée
• 1-{4-[(3-chloro-1H-pyrrolo[2,3-b]pyridin-4-yl)oxy]-3-(trifluorométhyl)phényl}-3-[2-(morpholin-4-yl)éthyl]urée
• 1-{4-[(3-chloro-1H-pyrrolo[2,3-b]pyridin-4-yl)oxy]-3-(trifluorométhyl)phényl}urée
• 1-{4-[(3-chloro-1H-pyrrolo[2,3-b]pyridin-4-yl)oxy]-3-(trifluorométhyl)phényl}-3-pyridazin-3-yl)urée
• 1-{4-[(3-chloro-1H-pyrrolo[2,3-b]pyridin-4-yl)oxy]-3-(trifluorométhyl)phényl}-3-(2-méthylpyrimidin-5-yl)urée
• 1-(3-méthoxy-4-{[3-(trifluorométhyl)-1H-pyrrolo[2,3-b]pyridin-4-yl]oxy}phényl)-3-[3-(morpholin-4-yl)propyl]urée
• 1-[3-(morpholin-4-yl)propyl]-3-[3-(trifluorométhoxy)-4-{[3-(trifluorométhyl)-1H-pyrrolo[2,3-b]pyridin-4-yl]oxy}phényl]urée
• 1-[2-(morpholin-4-yl)éthyl]-3-[3-(trifluorométhoxy)-4-{[3-(trifluorométhyl)-1H-pyrrolo[2,3b]pyridin-4-yl]oxy}phényl]urée
et leurs sels physiologiquement acceptables, et solvates de ces sels, et polymorphes, énantiomères, diastéréoisomères, racémiques, formes tautomères et solvates.

13. Composé de formule générale (I) selon l'une quelconque des revendications 1 à 12 pour l'utilisation en tant que médicament.

14. Composé de formule générale (I) selon l'une quelconque des revendications 1 à 12 pour une utilisation dans le traitement ou la prophylaxie d'une maladie.

15. Composition pharmaceutique comprenant un composé de formule générale (I) selon l'une quelconque des revendications 1 à 12 et un ou plusieurs excipients pharmaceutiquement acceptables.

16. Combinaison pharmaceutique comprenant :
• un ou plusieurs premiers ingrédients actifs, en particulier des composés de formule générale (I) selon l'une quelconque des revendications 1 à 12, et
• un ou plusieurs composés anticancéreux actifs pharmaceutiques ou
• un ou plusieurs inhibiteurs de point de contrôle immunitaire actifs pharmaceutiques.

17. Combinaison pharmaceutique selon la revendication 16, **caractérisée en ce que** l'inhibiteur de point de contrôle immunitaire actif pharmaceutique est un anticorps.

18. Utilisation d'un composé de formule générale (I) selon l'une quelconque des revendications 1 à 12 pour la fabrication d'un médicament pour le traitement ou la prophylaxie d'une maladie.

19. Utilisation d'un composé de formule générale (I) selon l'une quelconque des revendications 1 à 12 pour la préparation d'un médicament pour le traitement ou la prophylaxie d'une maladie.

20. Utilisation selon la revendication 18 ou 19, la maladie étant un cancer.

21. Utilisation selon la revendication 18 ou 19, les maladies, respectivement les troubles étant des hyperplasies bénignes, des troubles athérosclérotiques, une septicémie, des troubles auto-immuns, des troubles vasculaires, des infections virales, des troubles neurodégénératifs, dans des troubles inflammatoires, et la régulation de la fertilité masculine.
